(19) Europäisches Patentamt
European Patent Office
Office européen des brevets

(11) Numéro de publication: **0 290 583 B1**

(12) # FASCICULE DE BREVET EUROPEEN

(45) Date de publication de fascicule du brevet: **07.04.93**

(51) Int. Cl.$^5$: **C07H 21/04**

(21) Numéro de dépôt: **88900022.0**

(22) Date de dépôt: **02.12.87**

(86) Numéro de dépôt internationale :
**PCT/FR87/00481**

(87) Numéro de publication internationale :
**WO 88/04301 (16.06.88 88/13)**

(54) **OLIGONUCLEOTIDES -g(a).**

(30) Priorité: **02.12.86 FR 8616797**
**27.03.87 FR 8704339**

(43) Date de publication de la demande:
**17.11.88 Bulletin 88/46**

(45) Mention de la délivrance du brevet:
**07.04.93 Bulletin 93/14**

(84) Etats contractants désignés:
**AT BE CH DE FR GB IT LI LU NL SE**

(56) Documents cités:
**EP-A- 0 169 787**
**FR-A- 2 540 122**

**NUCLEIC ACIDS RESEARCH, vol. 14, no. 12, 1986, IRL Press Limited, Oxford (GB); F. MORVAN et al., pp. 5019-5035&NUM;**

(73) Titulaire: **CENTRE NATIONAL DE LA RECHERCHE SCIENTIFIOUE (CNRS)**
**15, Ouai Anatole France**
**F-75700 Paris Cedex 07(FR)**

(72) Inventeur: **HELENE, Claude**
**252, rue Haute**
**F-45590 Saint-Cyr-en-Val(FR)**
Inventeur: **IMBACH, Jean-Louis**
**Chemin Clos-des-Oliviers 1108, rue de Las-Sorbes**
**F-34000 Montpellier(FR)**
Inventeur: **NGUYEN, Thanh, Thuong**
**31, route de Tigy Vienne-en-Val**
**F-45510 Tigy(FR)**
Inventeur: **PAOLETTI, Claude**
**84, rue Michel-Ange**
**F-75016 Paris(FR)**
Inventeur: **RAYNER, Bernard Chênes-Colombière, G 68**
**180, avenue d'Occitanie**
**F-34100 Montpellier(FR)**

(74) Mandataire: **Ahner, Francis et al**
**CABINET REGIMBEAU, 26, avenue Kléber**
**F-75116 Paris (FR)**

**EP 0 290 583 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des composés chimiques nouveaux, ainsi que leur application, notamment à titre de sondes permettant la détection d'une séquence définie d'ADN ou d'ARN.

Les composés chimiques selon la présente invention sont constitués par un oligonucléotide ou un oligodésoxynucléotide comportant un enchaînement de nucléotides possédant la configuration anomérique non naturelle α par opposition à la configuration anomérique naturelle β.

Ainsi sont représentés par les formules (a) et (b) respectivement des nucléosides α et β

(a) 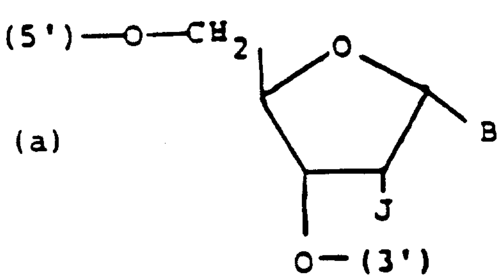

(b) 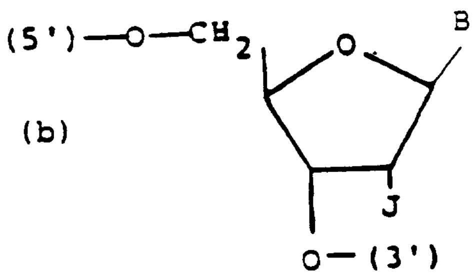

(a) et (b) représentent des nucléosides, il faut rajouter un phosphate en 5' pour obtenir des nucléotides.

L'emploi d'oligonucléotides naturels β comme sonde, c'est-à-dire dans toutes applications faisant intervenir leur appariement avec une séquence d'oligonucléotides complémentaire à des fins pharmacologiques est connu. Cependant, l'emploi d'oligonucléotides naturels β se heurte à certaines difficultés.

Une première difficulté réside dans le fait qu'il est nécessaire de prévoir des séquences suffisamment longues pour que ces séquences s'hybrident avec leur séquence cible complémentaire, de manière suffisamment stable.

Pour réduire cette longueur, il est necessaire d'adjoindre à l'oligonucléotide, comme l'ont proposé pour la première fois C. HELENE et ses collaborateurs, une chaîne portant un agent intercalant, tel l'acridine. Ces molécules se fixent plus fortement sur leurs séquences complémentaires, grâce à l'excès d'énergie produit par l'intercalation de la tête covalente dans l'hybride formé.

Une deuxième difficulté majeure de l'utilisation d'oligonucléotides naturels β consiste en la fragilité métabolique de nombreuses séquences oligonucléotidiques vis-à-vis de certaines enzymes. Or, on sait que tous les organismes vivants contiennent de nombreuses nucléases souvent très actives.

L'article de Nucleic Acids Research vol.14, no12, 1986 décrit la synthèse et caracterisation de l'hexaribonucleotide α d(CpCpTpTpCpCp)

Comme il est montré par la suite, les composés, selon la présente invention, comportent des séquences d'oligonucléotides α qui permettent de pallier ces inconvénients.

En effet, ils offrent la propriété intéressante de s'apparier de manière parallèle avec une séquence d'oligonucléotides β naturels complémentaire, alors que deux séquences α complémentaires ou deux séquences β complémentaires s'apparient anti-parallèlement, et cet appariement parallèle est beaucoup plus stable.

En outre, ces oligonucléotides α manifestent une résistance très élevée aux enzymes, notamment aux nucléases.

Dans ces conditions, de multiples utilisations à finalités biologiques et même pharmacologiques déjà connues pour les oligomères β peuvent être envisagées et ceci avec plus d'efficacité.

Plus précisément, la présente invention a pour objet des composés chimiques constitués par un oligonucléotide ou un oligodéoxynucléotide, caractérisés en ce qu'ils comportent un enchaînement de nucléotides ou déoxynucléotides à configuration anomérique non naturelle α, enchaînement lié à un agent effecteur, notamment un radical correspondant à un agent d'intercalation ou un radical chimique ou photoactivable, tel que un radical porteur d'une fonction réagissant directement ou indirectement avec les chaînes de nucléotides ou un radical dont la présence permet une détection facile et sensible.

La présente invention a aussi pour objet des composés selon la revendication 2 non liés à un agent effecteur.

En particulier, l'invention a pour objet de nouveaux dérivés d'α-oligonucléotides, leur préparation et leur emploi notamment comme sondes permettant la détection d'une séquence définie d'acides nucléiques, comme nucléases artificielles spécifiques de séquences d'ADN ou d'ARN ou bien comme agents de blocage sélectif de l'expression d'un gène qu'il soit endogène (par exemple, gène oncogène) ou exogène

(par exemple ADN ou ARN de virus, de parasites ou de bactéries).

Dans les demandes de brevet français FR 8301223 (2 540 122) et FR 8411795 (2 568 254) ont été décrits des composés chimiques constitués par un oligonucléotide ou un oligodésoxynucléotide comportant un enchaînement de nucléotides naturels ou modifiés, c'est-à-dire des $\beta$-nucléotides, sur lequel se trouve fixé par une liaison covalente au moins un groupe intercalant, qui possèdent la propriété de bloquer sélectivement l'expression d'un gène et qui, de ce fait, sont particulièrement utiles en thérapautique comme substances antivirales, antibiotiques, antiparasitaires ou antitumorales.

Dans la demande internationale PCT WO 83/01451 a été décrite une méthode pour bloquer la traduction de l'ARN messager (mRNA) en protéine par hybridation du mRNA avec un oligonucléotide ayant la séquence complémentaire du mRNA, l'oligonucléotide étant stabilisé sous forme phosphotriester.

Il a été trouvé, et c'est ce qui fait un objet de la présente invention, que des dérivés d'$\alpha$-nucléotides forment des complexes d'hybridation avec les séquences complémentaires d'ARN beaucoup plus stables que ceux formés avec l'ADN et que, vis-à-vis des ARNs, les dérivés d'$\alpha$-nucléotides forment des complexes d'hybridation plus stables que les dérivés de $\beta$-nucléotides naturels.

Parmi les composés, selon la présente invention, on peut citer plus particulièrement les composés oligomères $\alpha$ de formule

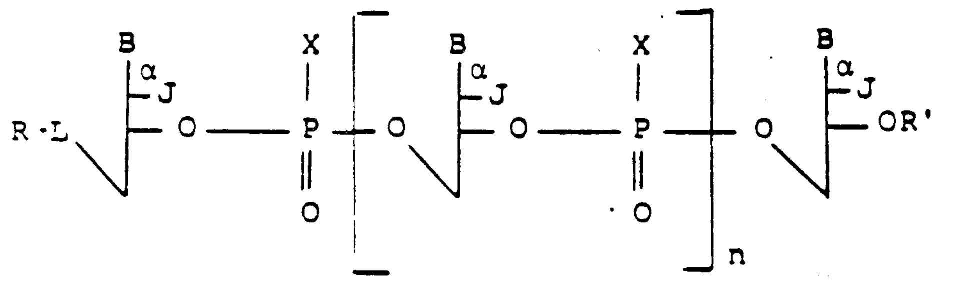

(I

dans laquelle :

les radicaux B peuvent être identiques ou différents et représentent chacun une base d'un acide nucléique éventuellement modifiée, activable et/ou comportant un groupement intercalant et rattachée au cycle glycosidique selon une configuration anomérique $\alpha$ non naturelle ;

les radicaux X peuvent être identiques ou différents et représentent chacun un oxoanion $O^{\ominus}$ , un thioanion $S^{\ominus}$ , un groupe alkyle, un groupe alcoxy, aryloxy, un groupe aminoalkyle, un groupe aminoalcoxy, un groupe thioalkyle, un radical alkyle ou alcoxy substitué par un hétérocycle azoté ou un groupement -Y-Z ;

R et R', qui peuvent être identiques ou différents, représentent chacun un atome d'hydrogène ou un groupement -Y-Z ou Y'-Z' ;

n est un nombre entier y compris O L représente un atome d'oxygène, un atome de soufre ou un groupe -NH-

Y et Y' identiques ou différents représentent chacun un radical alcoylène droit ou ramifié -alk- ou un radical choisi parmi

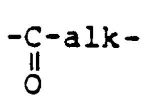 , 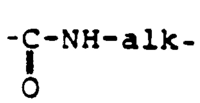 , 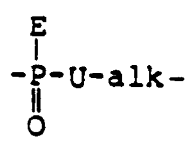 , 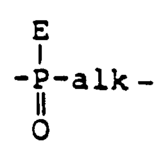 , alk-O-alk

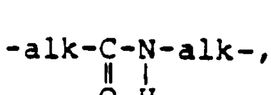 , 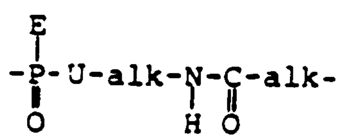 et 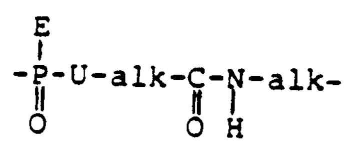 ,

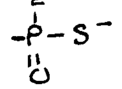 , 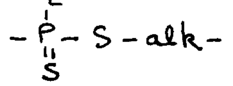 ,

avec U = O, S ou N

E peut avoir les mêmes significations que X excepté Y-Z,

J représente un atome d'hydrogène ou un groupe hydroxy ;

Z et Z', identiques ou différents, représentent chacun un radical correspondant à un agent effecteur, notamment un radical correspondant à un agent d'intercalation ou un radical porteur d'une fonction qui réagit directement ou indirectement avec les chaînes de nucléotides ou un radical dont la présence permet une détection facile et sensible.

Dans cette formule I , on utilise la représentation condensée des nucléosides suivante :

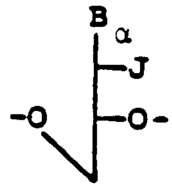

qui correspond à la formule développée :

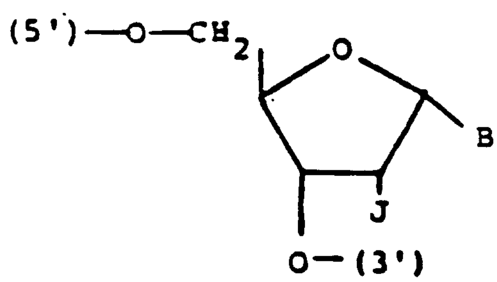

sur laquelle ont été mentionnées les extrémités (3') et (5').

Il convient de remarquer que la formule I représente un enchaînement de nucléotides qui peuvent être identiques ou différents, n indiquant simplement le nombre de nucleotides compris dans la molécule ; n est de préférence un nombre compris entre 1 et 50, et de préférence encore entre 1 et 25.

Les nouveaux dérivés selon l'invention peuvent être constitués par un analogue d'oligonucléotide ou d'oligodésoxynucléotide comportant un enchaînement d'anomères $\alpha$ de nucléotides éventuellement lié par une liaison covalente à un radical intercalant et/ou à un radical chimique porteur d'une fonction réagissant directement ou indirectement avec les chaînes de nucléotides ("radical chimique réactif") et/ou d'un marqueur dont la présence permet une détection facile, la radical chimique pouvant également constituer le radical intercalant.

En particulier la présente invention a pour objet, aussi, les composés des revendications 4 à 12.

Parmi ceux-ci plus particulièrement de nouveaux dérivés $\alpha$-oligonucléotides selon la présente invention peuvent répondre à la formule générale I dans laquelle

- lorsque L représente un atome de soufre ou un groupement -NH-, les radicaux R et R', identiques ou différents, représentent chacun un atome d'hydrogène ou un groupement -Y-Z ou Y'-Z' dans lequel :
  . Y et Y', identiques ou différents, représentent chacun un radical alcoylène droit ou ramifié (-alk-) ou un radical

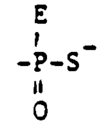

ou un radical

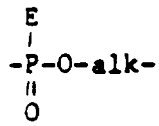, 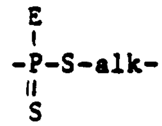

ou

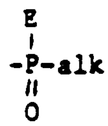

ou un radical -alk-O-alk- ou un radical -alk-CONH-alk- ou un radical

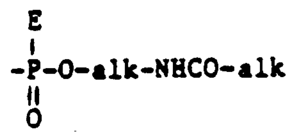

ou un radical

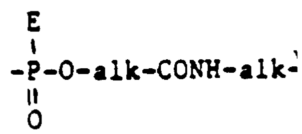

dans lesquels E a les mémes spécifications que X, excepte Y-Z, et

. Z et Z', identiques ou différents, représentent chacun un radical correspondant à un agent d'intercalation ou un radical porteur d'une fonction qui réagit directement ou indirectement avec les chaînes de nucléotides ou un radical dont la présence permet une détection facile et sensible,

- lorsque L représente un atome d'oxygène, les radicaux R et R', identiques ou différents, représentent chacun un atome d'hydrogène ou un groupement -Y-Z- ou Y'-Z' dans lequel Y, Y', Z et Z' sont définis comme précédemment, étant entendu que l'un au moins des radicaux R et R' contient un radical intercalant ou un radical chimique réactif,
- lorsque L représente un atome d'oxygène, R et R' représentent chacun un atome d'hydrogène et B représente une base nucléique modifiée activable et/ou comportant un groupement intercalant.

Les agents d'intercalation sont des produits connus dans les techniques relatives aux acides nucléiques ; ce sont des composés capables de "s'intercaler" dans la structure des ADN ou des ARN, c'est-à-dire capables de s'insérer entre les plateaux de base des acides nucléiques.

Les agents d'intercalation peuvent être choisis parmi les composés polycycliques ayant une configuration plane tels que l'acridine et ses dérivés, la furocoumarine et ses dérivés, 1a daunomycine et les autres dérivés de l'anthracycline, la 1,10-phénathroline, la phénanthridine et ses dérivés, la proflavine, les porphyrines, les dérivés du dipyrido [1,2-a : 3',2'-d] imidazole, l'ellipticine ou l'ellipticinium et leurs dérivés et le diazapyrène et ses dérivés.

Les radicaux chimiques réactifs peuvent être des radicaux pouvant réagir directement ou indirectement avec une chaîne de nucléotides pour former une liaison covalente ou pour la modifier chimiquement ou pour la couper. De préférence, ces radicaux chimiques réactifs sont activables, par exemple, par voie chimique, biochimique ou photochimique.

Les radicaux réactifs activables peuvent être choisis parmi l'acide éthylène-diamine-tétraacétique, l'acide diéthylène-triamine-pentaacétique, les porphyrines, la 1,10-phénanthroline, 1'azido-4 acétophénone, l'éthylène-imine, la $\beta$-chloroéthylamine, le psoralène et leurs dérivés, et les composés aromatiques absorbant les radiations du proche ultra-violet ou du visible ou pouvant réagir chimiquement avec les constituants nucléiques.

Plus particulièrement, les radicaux chimiquement activables en présence d'ions métalliques, d'oxygène et d un agent réducteur (acide éthylène-diamine-tétraacétique, acide diéthylène-triamine-pentaacétique, porphyrine, phénanthroline) induisent la coupure dans des séquences d'acides nucléiques situées dans leur voisinage.

Par irradiation dans le domaine du visible ou du proche ultra-violet, il est possible d'activer les dérivés qui absorbent ces radiations et de réaliser des réactions de pontage ou des réactions photo-induites (coupure et modification des bases nucléiques) des acides nucléiques sur lesquels est fixé l' α-oligonucléotide porteur du groupement activable.

Parmi les radicaux Z et Z' peuvent être plus particulièrement cités :

- les radicaux dérivés de l'acide éthylène-diamine-tétraacétique de formule :

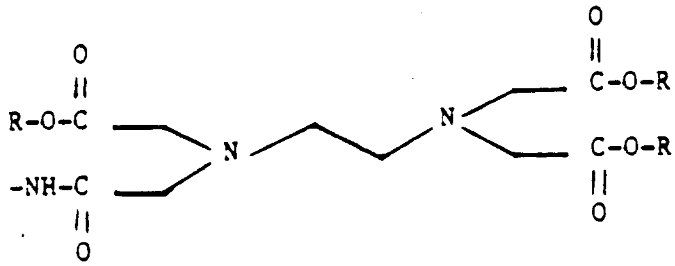

- les radicaux dérivés de l'acide diéthylène-triamine-pentaacétique,
- les radicaux dérivés de la méthylpyrroporphyrine de formule :

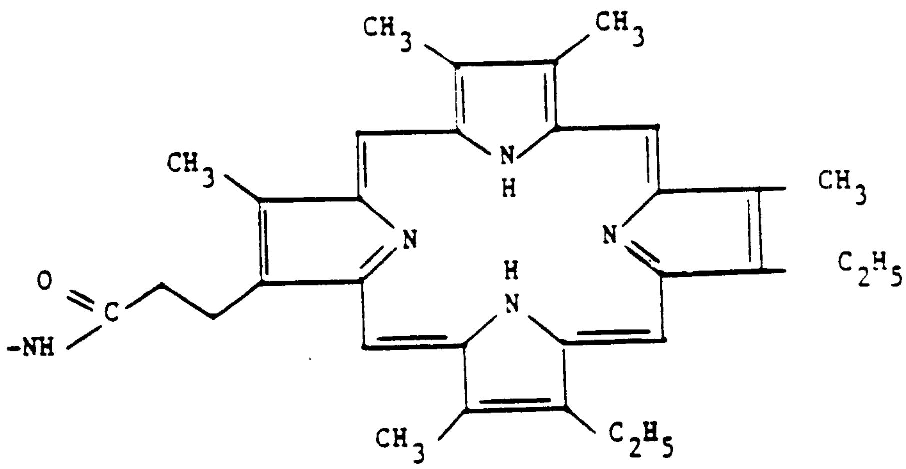

- les radicaux dérivés de la phénanthroline de formule :

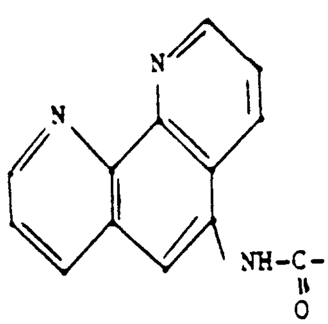

- les radicaux dérivés de l'acridine

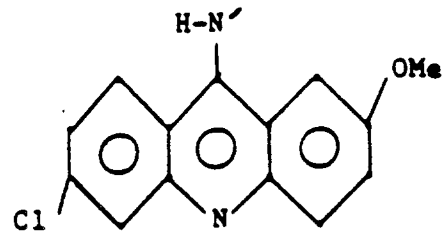

- les radicaux dérivés de la proflavine

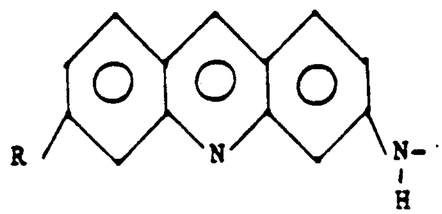

R représentant un groupement amino ($NH_2$) ou azido ($N_3$)

- les radicaux dérivés de la biotine

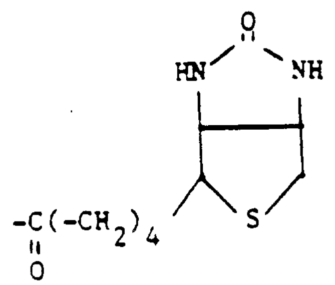

- les radicaux dérivés de l'azido-4 acétophénone de formule :

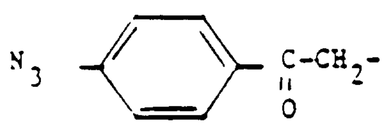

Le radical B peut être, de préférence, choisi parmi les bases nucléiques naturelles (thymine, adénine, cytosine, guanine, uracile) mais il est possible d'utiliser des bases nucléiques modifiées. Peuvent être cités l'amino-2 adénine et ses dérivés substitués, par exemple, sur l'atome d'azote $N^6$ par un radical aminoalkylène ou par un radical azidophénylalkylène, la guanine substituée sur l'atome d'oxygène $O^6$, par exemple, par un groupement ($\omega$-alkylène)-9-acridine, la ($\omega$-aminoalkyl)-amino-8-adénine et ses dérivés substitués sur le radical amino en $\omega$ par un groupe acridine, ou les dérivés halogénés ou azidés tels que le bromo-5 uracile, l'azido-8 adénine, la 7-déazaadénine, la 7-déazaguanine. Il est possible également d'utiliser des dérivés des bases nucléiques-comportant un groupement intercalant ou un groupement chimiquement ou photochimiquement activable.

Ainsi, la fonctionnalisation de C ou T par un groupement aziridine en position 4 conduit à la formation de pontages covalents $CH_2$-$CH_2$ entre les deux brins complémentaires sur respectivement G et A.

Donc, plus particulièrement, le radical 3 est choisi parmi la 4-azinidino-cytosine ou la 4-azinidino-thymine.

De préférence, le radical X représente un oxoanion. Cependant, le radical X peut représenter un radical alkyle contenant 1 à 7 atomes de carbone (méthyle, éthyle, propyle), un radical alcoxy dont la partie alkyle contient 1 à 7 atomes de carbone (méthoxy, éthoxy, diméthyl-2,2 propyloxy), un radical aminoalkyle ou aminoalcoxy de formule générale $R_1R_2N$-alk-A- dans laquelle A représente une liaison ou un atome d'oxygène, -alk- représente un radical alkylène contenant 1 à 10 atomes de carbone et $R_1$ et $R_2$, identiques ou différents, représentent un atome d'hydrogène ou un radical alkyle contenant 1 à 7 atomes de carbone ou forment ensemble avec l'atome d'azote auquel ils sont liés un hétérocycle azoté saturé à 5 ou 6 chaînons, étant entendu que le groupement $R_1R_2N$- peut être quaternarisé, ou un radical alcoylthio dont la partie alcoyle contient 1 à 7 atomes de carbone.

Dans la formule (I ), le radical -alk- est de préférence un radical alcoylène droit ou ramifié ayant de 1 à 10 atomes de carbone.

En particulier, à partir des fonctions alcool terminales 3' ou 5' de l'oligomère $\alpha$, l'effecteur Z peut donc être introduit via une chaîne $(CH_2)_n$ liée à une fonctionnalisation $Z'_1$ de natures diverses à la partie osidique de l'oligomère.

A partir de la formule suivante :

$$\text{3' ou 5'}\ -O-Z'_1-(CH_2)_n-\text{effecteur} \quad (Z)$$

on obtiendra, selon ce que représente. $Z'_1$ , par exemple

. un phosphate ou méthyl phosphonate de formule

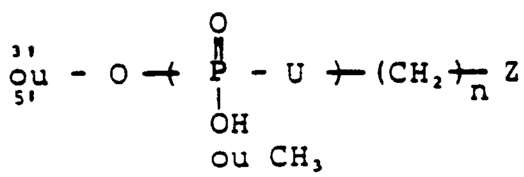

U = O, N ou S

. un éther de formule générale

$$\text{3' ou 5'}\ -O-CH_2-(CH_2)_{n-1}-Z$$

. un ester de formule générale

$$-O-CO-(CH_2)_n-Z$$

ou encore

. un carbamate de formule générale

$$-O-CO\ NH-(CH_2)_n-Z$$

La présente invention concerne également les composés précédents sous forme de sel avec des bases ou des acides, et les composés sous forme racémique, ou sous forme d'isomères R ou S optiques purifiés ou en mélange, de formule (I ).

La présente invention concerne de préférence les composés précédents de formule (I) dans la série D.

Parmi les produits de formule générale (I) plus particulièrement intéressants peuvent être cités : $\alpha$-[(Tp)-$_n$T]($Y_1$)$Z_1$ ; $Z_2$($Y_2$)$\alpha$-[(Tp)$_n$T] ; $Z_2$($Y_2$)$\alpha$-[(Tp)$_n$T]($Y_1$)$Z_1$ ; $Z_2$($Y_2$)$\alpha$-d(CpCpTpTpApTpApTpT) où A est un radical $\alpha$-D-désoxyadénosine, C est un radical $\alpha$-D-désoxycytidine, T est le radical $\alpha$-D-thymidine, n est un nombre entier compris entra 1 et 25 et $Y_1$ et $Y_2$ représentent un radical alcoylène contenant 1 à .10 atomes de carbone et $Z_1$ et $Z_2$ représentent chacun un dérivé de l'acridine ou un dérivé de la proflavine ou un dérivé de la 1,10-phénanthroline ou un dérivé de l'azido-4 acétophénone ou un dérivé de l'EDTA ou un dérivé de la biotine.

Les nouveaux produits de formule générale (I) peuvent être préparés par voie chimique par des procédés connus et, en particulier, ceux qui sont décrits dans Les demandes de brevets français FR 8301223 (2 540 122) et FR 8411795 (2 568 254).

Les composés de formule (I ) peuvent être préparés par voie chimique par des procédés dans lesquels on applique des synthèses au phosphodiester, au phosphotriester, au phosphoramidite ou à l'hydrogénophosphonate classiques pour des anomères $\beta$. Pour la méthode au phosphotriester, les dérivés $\alpha$-nucléotides de guanine sont éventuellement protégés en position O $^6$ par un groupe supplémentaire, de préférence de N,N-diphénylcarbamoyl.

Ainsi la présente invention a pour objet les procédés selon les revendications 13 à 26.

Dans ces procédés, on prépare tout d'abord la chaîne de nucléotides, les différents groupements non mis en oeuvre étant protégés, puis on élimine enfin les groupements protecteurs pour obtenir les produits recherchés. Ce procédé a permis d'obtenir, pour la première fois, des oligonucléotides $\alpha$ comprenant toutes les bases usuelles. Cette approche permet d'obtenir des oligonucléotides $\alpha$ de longueurs désirées.

Donc, selon la présente invention, on peut préparer les dérivés totalement protégés et éliminer les groupements protecteurs. Les produits de formule générale (I) peuvent aussi être obtenus en préparant, dans une première étape, les dérivés non protégés comportant, par exemple, un groupement thiophosphate à l'une des extrémités 3' ou 5' ou aux deux extrémités 3' et 5' de la chaîne d'$\alpha$-oligonucléotides puis en condensant le ou les groupe(s) thiophosphate(s) avec les dérivés Z et/ou Z' porteurs d'un groupement halogénoalkyle ou d'un groupement ester alkylé d'un acide sulfonique.

Un intérêt des composés selon la présente invention, constitués d'une chaîne d'oligonucléotides $\alpha$, est de pouvoir envisager leur utilisation indépendamment d'agents effecteurs, notamment d'agents intercalants, puisque ces nouvelles substances oligonucléotides assurent la reconnaissance de la séquence d'acide nucléique complémentaire avec une stabilité très accrue ; la fonction de l'agent intercalant étant d'augmenter la stabilité du complexe d'hybridation, sa présence n'est plus obligatoire.

Les composés selon l'invention, par leur grande affinité spécifique pour les séquences nucléiques complémentaires, sont donc supérieurs aux oligonucléotides actuels dont l'affinité pour les séquences complémentaires est beaucoup plus faible.

Les composés, selon la présente invention, sont donc utilisables de manière plus efficace, à titre de sondes d'hybridation, mais également comme composants de purification pour des séquences déterminées d'ADN ou d'ARN.

Ces composés peuvent également être utilisés pour détecter des mutations au niveau d'un ADN ou d'un ARN de manière plus efficace.

L'objet de la présente invention est également l'application de ces composés pour réaliser le blocage spécifique des gènes cellulaires ou d'agents pathogènes préalablement choisis (virus, bactéries, parasites).

Les composés de la présente invention, par leur propriété de se fixer fortement sur la séquence nucléique complémentaire, puis d'induire la coupure de la chaîne d'acides nucléiques en ce site, sont utilisables à titre de nucléases artificielles spécifiques de séquences. Ils peuvent être utilisés comme réactifs en biologie moléculaire ou en génie génétique.

Les composés selon l'invention sont donc aussi utilisables à titre de médicaments.

Les produits selon la présente invention peuvent être constitués d'une chaîne d'$\alpha$-oligonucléotides liée de façon covalente à un agent intercalant ou à un groupe chimique réactif ou à un agent intercalant et un groupe chimique réactif. Dans ces nouveaux produits, l'$\alpha$-oligonucléotide assure la reconnaissance de la séquence d'acide nucléique complémentaire, l'agent intercalant augmente fortement la stabilité du complexe d'hybridation et le groupe chimique réactif conduit, par activation, à une réaction de pontage ou à une modification chimique ou à une coupure de la chaîne d'acides nucléiques complémentaires. Ces nouveaux produits permettent donc d'induire des réactions de pontage, de modifier ou de couper avec une grande efficacité une chaîne d'acides nucléiques en un site déterminé préalablemant choisi.

Les nouveaux produits de formule générale (I) selon l'invention et les produits de formule générale (I) dans laquelle L représente un atome d'oxygène, R et R' représentent chacun un atome d'hydrogène et B représente un base nucléique naturelle forment des complexes d'hybridation avec les séquences complémentaires d'ARN beaucoup plus stables que ceux qui sont formés avec l'ADN. La stabilité peut être

mesurée par la température de demi-transition des complexes (T 1/2) lors d'une élévation de température. Les valeurs de T 1/2 sont déterminées à la concentration de $10^{-5}$ M en α -oligonucléotide dans un tampon à pH = 7 contenant du cacodylate de sodium ($10^{-2}$M) et du chlorure de sodium (0,1 M). Les résultats sont rassemblés dans les Tableaux I.

Cette différence de stabilité des complexes d'hybridation (Tableau I) permet une inhibition préférentielle des ARNs messagers et des ARNs viraux sans risque important de provoquer des effets indésirables au niveau de l'ADN du génome.

De plus, vis-à-vis des ARNs, les produits selon l'invention forment généralement des complexes plus stables que ceux qui sont obtenus à partir des dérivés des β-nucléosides naturels.

Dans les hybrides formés par les dérivés des α-oligonucléotides selon l'invention avec les séquences d'acides nucléiques complémentaires les deux brins possèdent généralement la même orientation. Par exemple, l'α-D-oligonucléotide Acr$(CH_2)_5$-α-$d^{5'}$ [pCpCpTpTpApTpApTpT]$^{3'}$ forme un complexe stable avec la séquence β-$d^{5'}$ (ApCpGpGpApApTpApTpApGpC]$^{3'}$ dont la température de demi-dissociation (T 1/2) est de 35°C dans un tampon de 10 mM cacodylate de sodium/0,1M NaCl à pH = 7 à une concentration de 10 µM. Dans les mêmes conditions, la température de demi-dissociation (T 1/2) est de 27°C pour le complexe formé par Acr$(CH_2)_5$ α-$d^{5'}$[TCTAAACTC]$^{3'}$ avec β-$d^{5'}$ [AGATTTGAG]$^{3'}$. Aucun complexe n'est mis en évidence au-dessus de 0°C avec le β-D-oligonucléotide dont la séquence est antiparallèle [β-$d^{5'}$-[GAGTTTAGA]$^{3'}$].

Les séquences des α-oligonucléotides, couplés ou non à un agent intercalent ou à un groupement activable chimiquement ou photochimiquement, comportant un enchaînement de nucléosides pyrimidiques sont capables de se fixer sur une double hélice d'ADN ou d'ARN comportant une séquence de bases puriques adjacentes associées par liaisons hydrogène à la séquence complémentaire des bases pyrimidiques . Cette fixation implique la formation locale d'une triple hélice dans laquelle les bases pyrimidiques de l'α-oligonucléotide forment des liaisons hydrogène (de type Hoogsteen ou Hoogsteen inversé) avec les bases puriques de la double hélice. Dans ce contexte, les α-oligonucléotides forment des complexes plus stables que les β-oligonucléotides correspondants et ils permettent d'induire des réactions irréversibles (pontage, modification ou coupure) sur une double hélice d'ARN ou d'ADN.

Les hybrides formés entre les dérivés α-oligonucléotides de l'invention et les séquences d'acide nucléique comportant une disposition de bases complémentaires sont beaucoup plus stables lorsque les deux brins possèdent la même orientation que lorsqu'ils sont antiparallèles. On entend donc par séquences d'acide nucléique complémentaires aux dérivés α -oligonucléotides de l'invention des portions d'acide nucléique comportant des séquences de bases complémentaires et orientées dans le même sens que les α -oligonucléotides.

Les nouveaux produits de formule générale (I) selon l'invention et les produits de formule générale (I) dans laquelle L représente un atome d'oxygène, R et R' représentent chacun un atome d'hydrogène et B représente un base nucléique naturelle, qui possèdent la propriété de se fixer fortement sur la séquence nucléique complémentaire, peuvent être utilisés comme sondes pour détecter la présence d'une chaîne de nucléotides complémentaires. Cette détection est facilitée par la présence d'un groupe fluorescent ou d'un groupe biotine dans ces molécules.

La structure non naturelle des α-oligonucléotides et la structure en chaînes parallèles des doubles hélices qu'ils forment avec une chaîne complémentaire confèrent des propriétés d'antigénicité rendant ainsi possible la préparation d'anticorps spécifiques dirigés contre des α-oligonucléotides éventuellement couplés à un agent intercalant ou contre leurs complexes avec un ADN ou un ARN naturels.

Dans un but diagnostique ou pronostique, les α-oligonucléotides, éventuellement couplés à un agent intercalant, peuvent être attachés de façon covalente à un support solide. Le couplage à un marqueur luminescent ou générateur d'une réaction colorée, luminescente ou fluorescente permet de les utiliser comme sondes de séquences d'ADN ou d'ARN dans un but diagnostique ou pronostique.

Un autre objet de la présente invention concerne l'application des nouveaux produits de formule générale (I) selon l'invention et des produits de formule générale (I) dans laquelle L représente un atome d'oxygène, R et R' représentent chacun un atome d'hydrogène et B représente un base nucléique naturelle au blocage spécifique de gènes cellulaires ou d'agents pathogènes préalablement choisis (virus, bactéries, parasites). De par leur configuration, les α-oligonucléotides selon l'invention sont beaucoup plus résistants vis-à-vis des nucléases que les dérivés de β-nucléosides naturels. Cette stabilité vis-à-vis de l'hydrolyse enzymatique permet l'utilisation des produits de formule générale (I) dans des expérimentations in vivo ou in vitro en présence de nucléases. De ce fait les α-oligonucléotides selon l'invention présentent des avantages incontestables sur les dérivés de β-nucléosides déjà connus.

Les nouveaux produits de formule générale (I) selon l'invention et les produits de formule générale (I) dans laquelle L représente un atome d'oxygène, R et R' représentent chacun un atome d'hydrogène et B

représente une base nucléique naturelle sont particulièrement utiles comme médicaments permettant de bloquer les gènes indésirables exogènes (virus, bactéries, parasites) ou endogènes (gènes cellulaires, oncogènes). L'expression de ces gènes peut être bloquée en agissant soit directement sur l'ADN ou l'ARN porteur de l'information génétique soit sur l'ARN messager, copie du gène, en bloquant alors toute traduction par hybridation ou par hybridation puis pontage ou modification ou coupure de l'ARN messager ou viral choisi comme cible.

Ce blocage peut être réalisé en utilisant un produit de formule générale (I) selon l'invention ou un produit de formule générale (I) dans laquelle L représente un atome d'oxygène, R et R' représentent chacun un atome d'hydrogène et B représente une base nucléique naturelle dont la séquence est complémentaire de celle d'une région non complexée d'un ARN ou d'un ADN et, en particulier, d'un ARN messager. L'hybridation, suivie ou non du pontage, de la modification ou de la coupure de l'ARN messager ou viral, empêche la synthèse de l'ARN ou de la protéine correspondante ou l'expression des fonctions virales ou parasitaires.

Si cet ARN ou cette protéine est vital pour le virus, la bactérie ou le parasite, les produits de formule générale (I) selon l'invention et les produits de formule générale (I) dans laquelle L représente un atome d'oxygène, R et R' représentent chacun un atome d'hydrogène et B représente une base nucléique naturelle constitueront des médicaments à activité antivirale, antibactérienne ou antiparasitaire.

Si cet ARN ou cette protéine n'est pas vital pour l'organisme, il est possible d'en supprimer sélectivement les effets. Dans ce cas, les produits de formule générale (I) selon l'invention et les produits de formule générale (I) dans laquelle L représente un atome d'oxygène, R et R' représentent chacun un atome d'hydrogène et B représente une base nucléique naturelle constitueront soit des médicaments à activité antitumorale lorsque le gène visé ou son ARN messager code pour une protéine impliquée dans la transformation cellulaire, soit des médicaments capables de supprimer Le caractère de résistance aux antiviraux, aux antibiotiques ou aux antiparasitaires lorsque la protéine codée est responsable de l'inactivation des antibiotiques, des antiviraux ou des antiparasitaires.

Des effets cytotoxiques spécifiques peuvent être obtenus par action d'un produit selon l'invention ou d'un produit de formule générale (I) dans laquelle L représente un atome d'oxygène, R et R' représentent chacun un atome d'hydrogène et B représente une base naturelle sur une fonction cellulaire indispensable à la cellule cible.

Les produits selon la présente invention et les produits de formule générale (I) dans laquelle L représente un atome d'oxygène, R et R' représentent chacun un atome d'hydrogène et B représente une base nucléique naturelle peuvent également être utilisés pour accroître la synthèse d'un ARN ou d'une protéine en agissant sur un gène de régulation du gène codant pour l'ARN ou pour la protéine.

Les produits selon la présente invention, qui possèdent la propriété de se fixer fortement sur la séquence nucléique complémentaire en simple brin ou sur une double hélice, puis d'induire éventuellement le pontage, la modification ou la coupure de la chaîne d'acides nucléiques en un site déterminé, sont utilisables comme réactifs sépcifiques de séquences et, plus particulièrement, comme nucléases artificielles. Ils peuvent être utilisés comme réactifs en biologie moléculaire ou en génie génétique. Ils peuvent être également utilisés pour obtenir des fragments d'acides nucléiques, en particulier des fragments d'ARNs pour lesquels il n'existe pas de nucléase spécifique de séquences.

**TABLEAU I**

| SEQUENCE COMPLEMENTAIRE \ (I) | (T 1/2)°C | | | | | |
|---|---|---|---|---|---|---|
| | α-$(Tp)_8(CH_2)_5$-Acr | Acr$(CH_2)_5$α-$(pT)_8$ | α-$(Tp)_7T$ | β-$(Tp)_8(CH_2)_5$Acr | Acr$(CH_2)_5$β-$(pT)_8$ | β-$(Tp)_7T$ |
| Polyr(A) | 38 | 37,5 | 2,5 | 36,4 | 28,5 | 16,1 |
| Polyd(A) | 25 | 25 | 14 | 34,4 | 26,4 | 18,1 |
| r$(Ap)_7A$ | 29,6 | 35,6 | 23 | 21,6 | 14,8 | 10 |
| d$(pA)_8$ | 18,5 | 27 | 13,5 | 21,9 | 20 | 11,2 |

Températures de demi-dissociation (T 1/2) des complexes formés entre les dérivés (I) et les séquences d'acides nucléiques complémentaires (anomères β), déterminées à la concentration de $10^{-5}$ M en α-D-oligonucléotide et avec le rapport [thymine/adénine] = 1 dans le tampon 10 mM cacodylate de sodium/0,1 M NaCl à pH = 7.

D'autres caractéristiques et avantages de la présente invention apparaîtront à la lumière des exemples qui vont suivre.

L'exemple V est illustré par les figures 1 et 2. Les résistances aux enzymes nucléase $S_1$ et phosphodiesterase respectivement, pour les anomères α et β sont représentées.

EXEMPLE I :Synthèse d'α-[d(CATGCG)] et α-[d(CGCATG)] par une méthode au phosphotriester en solution améliorée (voir schémas I et II ci-après)

Les deux hexadéoxyribonucléotides non naturels α-[d(CATGCG)] et α-[d(CGCATG)] comprenant seulement des unités de nucléotide à configuration anomérique α ont été obtenus par un procédé au phosphotriester en solution amélioré . Les produits de départ étaient les 4-désoxyribonucléosides 3a-d (voir schéma I ci-après). Les α-désoxynucléosides 3a et 3b (pyrimidine) ont été préparés par des réactions d'autoanomérisation suivi des protections sélectives des groupes hydroxyles, tandis que les deux déoxynucléotides 3c et 3d (purine) ont été préparés par des réactions de transglycosylation. Dans le cas des dérivés α-nucléoside de guanine un groupe supplémentaire protégeant la base a été introduit en position $O^6$ dans le but d'éviter des réactions secondaires pendant l'assemblage de l'oligonucléotide. Ce groupe était le N,N-diphénylcarbamoyle.

La N-benzoyl-4 di-O-acétyl-3',5' désoxy-2' cytidine a été choisie comme donneur de sucre et a été mise en réaction avec l'adénine protégée en N et la guanine protégée en N en présence d'un acide de Lewis : le triméthylsilyle trifluorométhanesulfonate, dans de l'acétonitrile à 70°C. Après avoir fait réagir le donneur de sucre avec la N-benzoyl-6 adénine l'anomère α 1c, en dépit du fait qu'il était le produit nucléosidique majoritaire, n'était pas séparé de l'anomère correspondant β.

En conséquence, ce mélange anomérique a été traité avec de l'ammoniac méthanolique et de l'α désoxy-2' adénosine (2c) a été obtenu dans un rendement de 27 % après purification par chromatographie sur colonne Dowex 1. Ensuite, le dérivé (2c) α désoxy-2' adénosine a été converti dans le dérivé (3c) 6-N-benzoylé dans un rendement de 70 %.

De la même manière, la réaction de transglycosylation a été tentée avec le N-acétyl-2 guanine. Toutefois, des rendements très faibles de dérivés nucléosidiques de la N-acétyl-2 guanine ont été obtenus, vraisemblablement à cause de la faible solubilité de ces dérivés dans des solvants organiques. Un meilleur rendement en 1d a été obtenu lorsque l'on utilise dans la réaction de transglycosylation le composé N-palmitoyl-2 guanine qui est plus lipophile.

Après 50 mn de réaction à 70°C, une analyse TLC du mélange de réaction indiquait la formation d'isomères N-7 comme produits majoritaires. En poursuivant le chauffage, une transformation douce des isomères N-7 en isomères N-9 a été observée après 6 heures de chauffage.

Le dérivé α-désoxy guanosine 1d recherché a été isolé dans un rendement de 26,6 % après chromatographie sur colonne de gel de silice et par cristallisation fractionnée.

La fonction lactame énolisable des restes guanine perturbe la synthèse d'oligodésoxynucléotide par la méthode phosphotriester à cause de réactions secondaires des fragments guanine qui entraînent une baisse de rendement dans l'élongation des chaînes contenant de la guanine. Il est donc nécessaire de protéger la fonction lactame de la guanine. Le N,N-diphénylcarbamoyle a été choisi comme groupe protecteur de la position $O^6$. Il peut être facilement introduit en une étape sur le dérivé de départ 1d et peut être éliminé à la fin de la synthèse au phosphotriester, sans étape de déprotection supplémentaire.

La réaction entre le chlorure de diphénylcarbamoyle et la diisopropyléthylamine et le produit 1d dans de la pyridine suivie par une addition d'hydroxide de sodium aqueux, fournie après purification par chromatographie sur colonne de gel de silice du N-palmitoyl-2 0-diphénylcarbamoyl-6 α-désoxy-2' guanosine (3d) sous forme de composé cristallin dans un rendement de 77 %.

Ensuite, la conversion des produits 3c et 3d, dont seule la base est protégée, dans les nucléotides-α 4c et 4d entièrement protégés correspondants (schéma II ci-après) a nécessité un protocole en deux étapes incluant une tritylation sélective de l'hydroxyle 5' par du chlorure diméthoxy-4,4' trityl (1,5 équivalent molaire) et une phosphorylation de l'hydroxyle 3' avec du (chloro-2-trityl-4 phényl) (cyano-2 éthyl) phosphate de triéthylammonium (1,1 équivalent molaire), du chlorure de mésitylènesulfonyle (2,2 équivalent molaires) et du méthyl-1 imidazole. Cette dernière étape permet d'introduire le chloro-2-trityl-4 phényle comme groupe protecteur de phosphate. Grâce à ses propriétés lipophiles, ce groupe protecteur a été choisi pour faciliter la purification et accroître les rendements des intermédiaires entièrement protégés pendant la synthèse d'oligonucléotides selon la méthode au phosphotriester en solution. Après purification par chromatographie sur colonne de gel de silice, les deux dérivés nucléotides α 4c et 4d ont été isolés sous forme de mélanges diastéréoisomériques respectivement dans des rendements de 92 et 53 %. Comme les deux hexa-mères cibles présentent une guanine terminale en 3' dans leur séquence de base, la seule unité terminale en 3' pour leurs synthèses est le dérivé O-benzoyl-3' α-déoxy guanosine 5d. La diméthoxytritylation sélective de 3d, suivie par une benzoylation et une détritylation fournit le dérivé 5'-OH 5d dans un rendement global de 81 %.

La synthèse des oligodésoxynucléotides α entièrement protégés α-[d(CATGCG)] et α-[d(CGCATG)] a été accomplie par la répétition d'un cycle de synthèse consistant essentiellement en trois étapes principales. L'étape (a) est une déprotection de l'hydroxyle 5' par traitement d'un nucléotide ou d'un oligonucléotide intermédiaire entièrement protégé avec de l'acide benzènesulfonique 2 % dans un mélange de chlorure de méthylène et de méthanol (7/3) à 0 ° C pendant 15 à 40 mn. L'étape (b) est la déprotection du phosphodiester 3' par élimination du groupe cyanoéthyle à partir d'un nucléotide ou d'un oligonucléotide entièrement protégé, avec de la ter-butylamine 10 % dans de la pyridine anhydre pendant 15 à 25 mn. L'étape (c) est la condensation entre les produits de l'étape (a) et de l'étape (b) (10 à 15 % dans un excès molaire) en présence d'un agent de couplage : le 1-(mésitylène-2-sulfonyl)-3-nitro-1,2,4-triazole suivi par la purification des produits par chromatographie sur colonne de gel de silice. Les conditions et résultats des réactions de couplage sont résumés dans le tableau II suivant.

TABLEAU II

A) α-d(CATGCG)

| Composante 3'-phosphodiester (mmol.) | composante 5'hydroxyl (mmol.) | MSNT (mmol.) | durée (mn) | Produit | rendement |
|---|---|---|---|---|---|
| Dmtr-A*$\dot{p}$ (0.152) | HO-T*$\dot{p}$-Cne (0.138) | 0.38 | 45 | Dmtr-A*$\dot{p}$T*$\dot{p}$-Cne | 90 |
| Dmtr-C*$\dot{p}$ (0.138) | HO-A*$\dot{p}$T*$\dot{p}$-Cne (0.124) | 0.35 | 40 | Dmtr-C*$\dot{p}$A*$\dot{p}$T*$\dot{p}$-Cne | 92 |
| Dmtr-C*$\dot{p}$ (0.137) | HO-G*-Bz (0.125) | 0.35 | 50 | Dmtr-C*$\dot{p}$G-Bz | 95 |
| Dmtr-G*$\dot{p}$ (0.131) | HO-C*$\dot{p}$G*-Bz (0.119) | 0.33 | 70 | Dmtr-G*$\dot{p}$C*$\dot{p}$G*-Bz | 89 |
| Dmtr-C*$\dot{p}$A*$\dot{p}$T*$\dot{p}$ (0.113) | HO-G*$\dot{p}$C*$\dot{p}$G*-Bz (0.096) | 0.57 | 60 | Dmtr-C*$\dot{p}$A*$\dot{p}$T*$\dot{p}$G*$\dot{p}$C*$\dot{p}$G*-Bz | 97 |

B) α-d(CGCATG)

| Composante 3'-phosphodiester (mmol.) | Composante 5'-hydroxyl (mmol.) | MSNT (mmol.) | durée (mn) | Produit | rendement |
|---|---|---|---|---|---|
| Dmtr-G*ṗ (0.154) | HO-C*ṗ-Cne (0.140) | 0.39 | 55 | Dmtr-G*ṗC*ṗ-Cne | 80 |
| Dmtr-C*ṗ (0.124) | HO-G*ṗC*ṗ-Cne (0.112) | 0.31 | 60 | Dmtr-C*ṗG*ṗC*ṗ-Cne | 96 |
| Dmtr-T*ṗ (0.137) | HO-G*-Bz (0.125) | 0.34 | 45 | Dmtr-T*ṗG-Bz | 95 |
| Dmtr-A*ṗ (0.112) | HO-T*ṗG*-Bz (0.102) | 0.28 | 60 | Dmtr-A*ṗT*ṗG*-Bz | 90 |
| Dmtr-C*ṗG*ṗC*ṗ (0.108) | HO-A*ṗT*ṗG*-Bz (0.082) | 0.54 | 45 | Dmtr-C*ṗG*ṗC*ṗA*ṗT*ṗG*-Bz | 100 |

[a] abréviations : Dmtr : diméthoxy-4,4' trityl, C* : N-benzoyl-4 α désoxy-2' cytidine, T* : α thymidine, A* : N-benzoyl-6 α désoxy-2' adénosine, G* : N-palmitoyl-2 0-diphényl-6 carbamoyl α désoxy-2' guanosine, ṗ : chloro-2 trityl-4 phénylphosphate, MSNT : 1-(mésitylène-2-sulfonyl)-3-nitro-1,2,4-triazole, Cne : 2-cyanoéthyl, Bz : benzoyle.

Au cours du processus d'élongation des oligonucléotides α entièrement protégés, aucune différence marquante (durée de réaction, formation de produits secondaires, etc.) n'a été détectée par rapport à celui des oligonucléotides β naturels.

En d'autres termes, aucun effet anomérique notable n'est apparu par la méthode phosphotriester.

La déprotection totale des hexa-mères a protégés a été effectuée comme suit : traitement des 6-mères α entièrement protégés avec du $N^1$,$N^1$,$N^3$,$N^3$-tétraméthylguanidinium, 2-pyridine-carboxaldoximate 1M dans un mélange dioxanne-eau (7/1, v/v) à 70 °C pendant 24 heures, puis avec de l'ammoniaque concentré aqueux. Il a résulté de ce traitement l'élimination simultanée des groupes diphénylcarbamoyle et des groupes chloro-2-trityl-4 phényle, et des groupes benzoyle et palmitoyle. Les oligonucléotides 5'-diméthoxytrityl résultants ont été ensuite traités avec de l'acide acétique pendant 15 mn à température ambiante. Ensuite, on a effectué une purification par chromatographie sur colonne à échange d'ion. Les deux oligonucléotides α déprotégés α-[d(CATGCG)] et α-[d(CGCATG)] ont été obtenus dans des rendements respectifs de 59 et 52 %.

Ainsi, la méthode au phosphotriester en solution a pu être appliquée avec succès à la synthèse d'oligonucléotides α contenant les quatre bases usuelles.

Le schéma I ci-après reproduit la synthèse des quatre déoxy-2' nucléosides α ayant leur base protégée (sauf T).

Les conditions réactionnelles étaient les suivantes pour les différentes étapes de (a) à (h).

Synthèse d'α-nucléosides protégés : conditions de réactions.

(a) trifluorométhanesulfonate de triméthylsilyle N,O-bis (triméthylsilyle) acétamide/acétonitrile ;

(b) méthoxyde de sodium/méthanol ou hydroxyde de sodium aqueux/eau/tétrahydrofuranne/méthanol ;

(c) trifluorométhanesulfonate de triméthylsilyle, N,O-bis(triméthylsilyle)acétamide, 6-N-benzoyladénine/acétonitrile ;

(d) ammoniaque méthanolique ;

(e) chlorure de triméthylsilyle/pyridine, chlorure de benzoyle, solution concentrée d'ammoniaque ;

(f) trifluorométhanesulfonate de triméthylsilyle, N,O-bis(triméthylsilyle)acétamide, N-palmitoyl-2 guanine/acetonitrile ;

(g) chlorure de diphénylcarbamoyle/pyridine ;

(h) hydroxyde de sodium aqueux/éthanol.

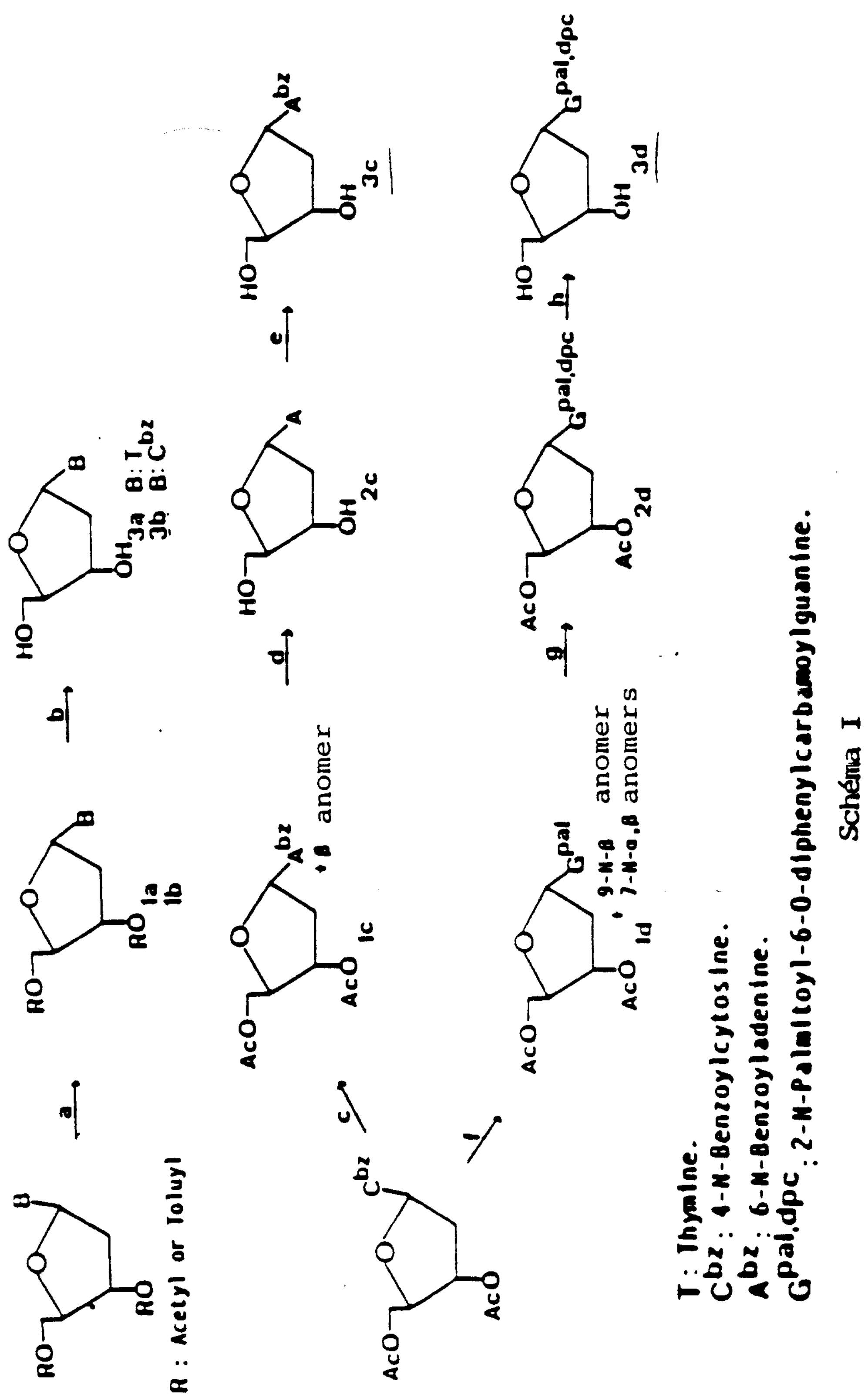

Schéma I

Le schéma II ci-après reproduit la synthèse des quatre désoxy-2' nucléosides $\alpha$, ayant leur base protégée (sauf T).

Les conditions réactionnelles étaient les suivantes dans les étapes (a) à (d) :

(a) chlorure de diméthoxy-4,4' trityle/pyridine ;

(b) (trityl-4 chloro-2 phényl) (cyano-2 éthyl) phosphate de triéthylammonium/chlorure de mésitylène-2-sulfonyle ;
(c) chlorure de benzoyle/pyridine ;
(d) acide benzènesulfonique 2 %/chlorure de méthylène-méthanol (7:3, v/v).

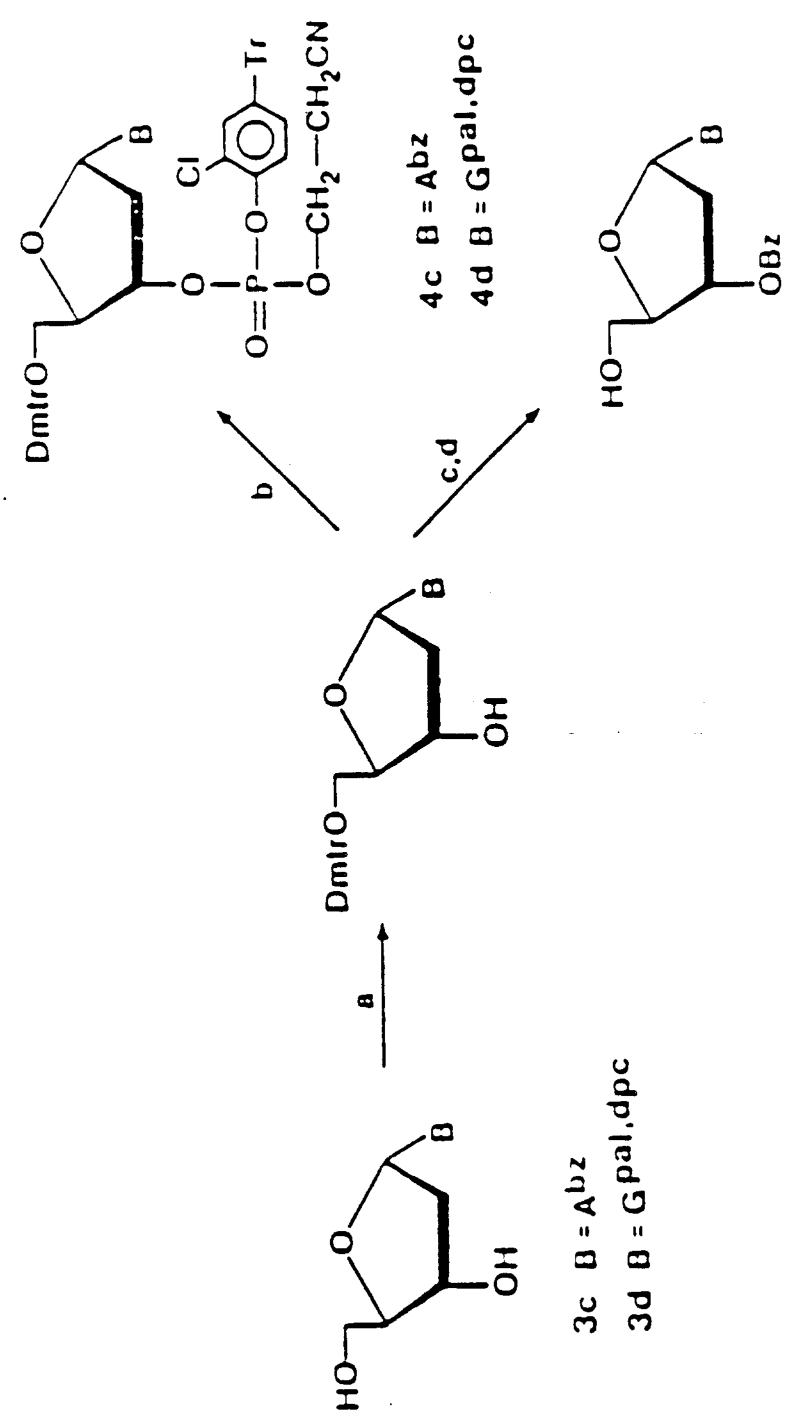


3c B = $A^{bz}$
3d B = $G^{pal,dpc}$

4c B = $A^{bz}$
4d B = $G^{pal,dpc}$

5d B = $G^{pal,dpc}$

$A^{bz}$ : 6-N-Benzoyladenine
$G^{pal,dpc}$ : 2-N-Palmitoyl-6-O-diphenylcarbamoyl-guanine
Tr : Triphenylmethyl

Schéma II

1) Préparation de l'α désoxy-2' adénosine (2c) (schéma I)

Ce composé a été préparé comme décrit par T. Yamaguchi et Cal. dans Chem. Pharm. Bull. 32, 1441 (1984). On a obtenu ce composé dans un rendement de 27,3 % pour un point de fusion de 212,5 - 213 ° C.

2) Préparation de la N-benzoyl-6 $\alpha$ désoxy-2' adénosine (3c) (schéma II)

En utilisant un procédé en trois étapes similaires à celui décrit pour les anomères $\beta$ correspondants par exemple dans Journal Amer. Chem. Soc., 104, 1316 (1982), ce produit a été préparé à partir de (2c) (2,01 g , 8 mmoles). A la fin de la troisième étape c'est-à-dire après traitement avec l'ammoniaque aqueux pendant 30 mn suivi de l'évaporation, le résidu a été dissous dans de l'eau (200 ml). La solution a été lavée avec de l'éther diéthylique (2 x 70 ml) et évaporée jusau'à siccité. Le résidu restant a été mélangé avec du méthanol (50 ml) puis filtré, et le filtrat a été évaporé jusqu'à siccité. Le résidu a été purifié par chromatographie sur colonne gel de silice (100g). Les fractions appropriées ont été combinées et évaporées. Le résidu a été cristallisé à partir d'éthanol-acétone. La filtration a donné 1,99 g (70 %) de 2. Le point de fusion est 163-164°C.

Les résultats analytiques étaient les suivants :

Anal. calc. pour $C_{17}H_{17}O_4N_5$ : C, 57.46; H, 4.82; N, 19.71;obtenu : C, 57.30; H, 4.86; N, 19.75. UV

$$\lambda_{max}^{H_2O}$$

(nm($\epsilon$)) : 280 (21,500);

$$\lambda_{max}^{pH\ 2.1}$$

(nm($\epsilon$)) : 253 (10,900), 284 (24,000). $[a]_D^{20}$ + 59° (c=1, DMF). $^1$H-NMR (DMSO-$d_6$) $\delta$ 2.44 (m,1H,$H_2$''); 2.8 (m,1H,$H_2$'); 3.49(m,2H,$H_5$' and $H_5$''); 4.21 (m,1H,$H_4$'); 4.37 (m,1H,$H_3$'); 4.86 (t,1H,$OH_5$'); 5.54 (d,1H,$OH_3$'); 6.50 (dd,1H,$H_1$',J=2.58 et 7.57 Hz); 7.52-8.06 (m,5H,Ar-H); 8.69 et 8.74 (2s,2H,$H_2$ et $H_8$); 11.14 (br s,1H,NH).

Les spectres RMN du proton ont été obtenus sur un appareil Bruker WBUM 360.

3) Préparation de la Di-O-acétyl-3',5' N-palmitoyl-2 $\alpha$ désoxy-2' guanosine (1d) (schéma I)

Du bis-triméthylsilylacétamide (7,4 ml, 300 mmoles) a été ajouté à une solution de di-O-acétyl3',5' N-benzoyl-4 désoxy-2' cytidine (15 g, 36 mmoles) et N-palmitoyl-2 guanine (37,4 g, 96 mmoles) dans de l'acétonitrile anhydre (220 ml). Le mélange a été chauffé à 70°C pendant une heure et du triflate de triméthylsilyle (8 ml, 47 mmoles) a été ajouté. Le mélange a été agité à 70°C pendant six heures. Le solvant a été éliminé à pression réduite et le résidu a été dissous avec du chlorure de méthylène (750 ml). De l'hydrogènocarbonate de sodium aqueux froid (70 ml) a été ajouté et après agitation vigoureuse, le mélange a été filtré par aspiration. La couche organique a été séparée et séchée avec du sulfate de sodium anhydre et le solvant a été éliminé sous pression réduite. Le résidu (Rf O.44 et O.57, silica gel TLC, $CH_2Cl_2$-$CH_3OH$ 9:1, v/v) a été chromatographié sur colonne silica gel (250 g) en utilisant des proportions croissantes (0-5 %) de méthanol dans le chloroforme comme éluant. Des fractions contenant de la matière avec un Rf de 0,44 (isomères N-9 $\alpha$ et $\beta$ ) ont été réunies et évaporées jusqu'à siccité. Le résidu a été dissous dans de l'éthanol chaud et refroidi, l'anomère $\alpha$1d a été cristallisé pour donner 5,655 g (26,6 %) de cristaux incolores ; point de fusion 129-130°C.

Anal. Calc. pour$C_{30}H_{47}H_5O_7$: C, 61.10: H, 8.03; N, 11.88;obtenu c, 60.95; H, 8.19; N, 12.06. UV

$$\lambda_{max}^{EtOH}$$

(nm($\epsilon$)): 258 (18,300); 279 (13,900). $[\alpha]_D^{20}$ + 19° (c = 1, DMF). $^1$H-NMR ($CDCl_3$) $\delta$ 0.86 (t, 3H, $(CH_2)_{14}$-$CH_3$); 1.20-1.40 (m,24H,-$(CH_2)_{12}$-$CH_3$); 1.71 (m, 2H,- C(=O)-$CH_2$- $CH_2$- ; 2.03 et 2.1 (2s,6H,2$CH_3$-C(=O)-); 2.48 (t,2H,- C(=O)-$CH_2$-$(CH_2)_{13}$-); 2.56 (m,1H,$H_2$''); 2.84 (m,1H,$H_2$'); 4.18-4.28 (m,2H,$H_5$' et $H_5$''): 4.52 (m,1H,$H_4$'); 5.28 (m,1H,$H_3$'); 6.23 (dd,1H,$H_1$',J=1.89 et 7.47 Hz); 7.92 (s,1H,$H_8$); 8.77 et 12 (2br s,2H,2 NH).

4) Préparation de la N-palmitoyl-2 O-diphénylcarbamoyl-6 $\alpha$ désoxy-2' guanosine (3d) (schéma I)

Du chlorure de diphénylcarbamoyle (2,192 g, 9,46 mmoles) et de la diisopropyléthylamine (1,23 ml, 7,1 mmoles) ont été ajoutés à une solution de 1d (2,536 g, 4,3 mmoles) dans de la pyridine anhydre (11 ml). Le

mélange résultant a été agité pendant 2,75 heures à température ambiante et (41 ml) de pyridine et (21,5 ml) d'éthanol ont été ajoutés. Le mélange de réaction a été agité pendant 10 mn à 0 ° C. Une solution d'hydroxyde de sodium aqueuse 2 N (21,5 ml) a été ajoutée et après une agitation supplémentaire de 10 mn à 0 ° C le mélange de réaction a été neutralisé avec de l'acide acétique (2,65 ml). Ensuite, le mélange de réaction a été versé dans de l'eau (150 ml) et les produits ont été extraits avec du chlorure de méthylène (4 x 50 ml). Les couches organiques réunies ont été évaporées jusqu'à siccité sous pression réduite et le résidu a été fractionné par chromatographie sur colonne de gel de silice (50g). Des fractions contenant du 3d pur ont été réunies et évaporées jusqu'à siccité. Le résidu a été cristallisé à partir d'éthylacétate (2,323 g, 77%), point de fusion 134-135 ° C.

Anal. Calc. pour $C_{39}H_{52}N_6O_6$: C, 66,83; H, 7.48; N, 11.99;obtenu C, 66.53; H,7.66; N, 11.70. $[\alpha]_D^{20}$ + 33 ° (c = 1, DMF). UV

$$\lambda_{max}^{EtOH}$$

(nm(ε)) 228(37,900), 277 (13,900), 257 (shoulder). $^1$H-NMR (DMSO-$d_6$) δ 0.85(t, 3H, $(CH_2)_{14}$-$CH_3$); 1.18-1.35 (m,24H,$(CH_2)_{12}$-$CH_3$); : 1.58 (m,2H'' C( = O) -$CH_2$-$CH_2$-); 2.41-2.48 (m,3H,$H_2$'' and C( = O)-$CH_2$-$(CH_2)_{13}$); 2.76 (m,1H,$H_2$'); 3.48 (m,2H,$H_5$' and $H_5$''); 4.21 (m,1H, $H_4$'); 4.35 (m,1H,$H_3$'); 4.86 (t,1H,$OH_5$'); 5.43 (d,1H, $OH_3$'); 6.38 (dd,1H,$H_1$',J = 2.36 and 7.67 Hz); 7.30-7.51 (m,10H,ArH); 8.66(s,1H,$H_8$); 10.65 (s,1H,C( = O)-NH-).

5) Préparation de N-palmitoyl-2 O-diphénylcarbamoyl-6 O-benzoyl-3' α désoxy-2' quanosine (5d) (schéma II)

Du chlorure de diméthoxy-4,4' trityl (0,39 g) 1,15 mmole), a été ajouté à une solution de 3d (0,7 g, 1 mmole) dans de la pyridine sèche (3,5 ml). Le mélange résultant a été agité pendant deux heures à température ambiante et du chlorure de benzoyle (0,152 ml, 1,3 mmole) a été ajouté. Le mélange a été agité pour une période additionnelle de 4,4 heures et 1 ml d'eau a été ajouté. Après cinq minutes d'agitation, le mélange de réaction a été versé dans de l'hydrogénocarbonate de sodium aqueux saturé (40 ml) et les produits ont été extraits avec du chlorure de méthylène (4 x 30 ml). Les couches organiques combinées ont été évaporées jusqu'à siccité sous pression réduite. Le résidu a été dissous dans du chlorure de méthylène-méthanol (7/3, v/v, 20 ml). A la solution refroidie (0 ° C) résultante, a été ajouté une solution refroidie d'acide benzènesulfonique 10 % (p/v) dans le même mélange (5ml). Après 14 mn d'agitation à 0 ° C, le mélange de réaction a été neutralisé avec de l'hydrogénocarbonate de sodium aqueux (20 ml) et partagé entre l'hydrogénocarbonate de sodium aqueux saturé (40 ml) et du chlorure de méthylène (4 x 30 ml). Les couches organiques combinées ont été évaporées jusqu'à siccité et le résidu a été fractionné par chromatographie sur colonne de gel de silice (30g). Des fractions contenant du produit 5d pur ont été combinées et évaporées. Le résidu a été cristallisé à partir de méthanol pour donner des cristaux incolores (0,65 g, 81 %), point de fusion 144-146 ° C. Anal. Calc. pour$C_{46}H_{56}N_6O_7$: C, 68.64; H, 7.01; N, 10.44; obtenu C, 68.37; H, 7.04; N, 10.35. $^1$H-NMR($CDCl_3$) δ 0.87 (t,3H,$(CH_2)_{14}$-$CH_3$); 1.18-1.35 (m,24H, $(CH_2)_{12}$-$CH_8$);1.66-1.74 (m,2H,C( = O) $CH_2$-$CH_2$-); 2.67(pseudo t,2H,C( = O)-$CH_2$-$(CH_2)_{13}$); 2.96-3.03 (m,3H,$H_2$',$H_2$'' et $OH_5$'); 3.87-3.90 (m,2H,$H_5$' et $H_5$''); 4.56 (m,1H,$H_4$'); 5.61 (m,1H,$H_3$'); 6.53 (pseu do t,1H,$H_1$',$J_{app}$ = 4.3 Hz); 7.21-7.68 (m,15H,ArH); 8.22 (s,1H,NH); 8.27(s,1H,$H_8$).

6) Méthode générale de préparation de O-diméthoxytrityl-5' α désoxyribonucléoside 3'-O-[(2-chloro-4-tritylphényl) (2-cyanoéthyl)]phosphates

Du chlorure de diméthoxy-4,4' trityl (1,091 g, 3,22 mmoles) a été ajouté à une solution de α-désoxyribonucléoside dont la base est protégée (2,8 mmoles) dans de la pyridine sèche (7 ml) et le mélange a été agité à température ambiante jusqu'à disparition complète du matériau nucléosidique de départ (2 à 2,5 h). Ensuite, du (chloro-2 trityl-4 phényl) (cyano-2 éthyl, phosphate de triéthylammonium (2,06 g, 3,5 mmoles), du chlorure de mésitylène-2-sulfonyle (1,531 g, 7 mmoles) et du N-méthylimidazole (0,56 ml, 7 mmoles) ont été successivement ajoutés. Le mélange de réaction a été agité à température ambiante pendant une heure et un mélange d'eau (2 ml) et de pyridine (5 ml) a été ajouté. Après une agitation supplémentaire de 15 mn, le mélange de réaction a été versé dans du dihydrogénophosphate de sodium aqueux 0, 2 M (150 ml) et extrait avec du chlorure de méthylène (3 x 60 ml). Les couches organiques réunies ont été lavées successivement avec du dihydrogénophosphate de sodium aqueux 0,2 M (75 ml) et de l'hydrogénocarbonate d'ammonium aqueux 0,6 M (1 % par rapport au chlorure de sodium, 75

ml). La couche organique a été évaporée jusqu'à siccité sous pression réduite et le résidu a été fractionné par chromatographie sur colonne de gel de silice (60g). Les fractions appropriées ont été combinées et évaporées jusqu'à siccité et lyophilisées à partir du benzène.

Pour le O-diméthoxytrityl-5' N-benzoyl-6 α désoxy-2' adénosine 3'-[(chloro-2 trityl-4 phényl) (cyano-2 éthyl)]phosphate (4c).

Le rendement en mélange diastéréoisomérique était de 92 %,Anal. Calc. pour $C_{66}H_{56}ClN_6O_9P$, 0.5 $H_2O$: C, 68.77; H, 4.98; N, 7.29; obtenu C,68.85; H, 4.99; N, 7.35. $^{31}$P-NMR ($CDCl_3$) δ -7.88 et -7.96. $^1$H-NMR ($CDCl_3$) δ 2.58-2.65 (m,2H,$CH_2$-CN): 2.95 (m,1H,$H_2$''); 3.08(m,1H,$H_2$'); 3.22-3.47 (m,2H,$H_5$' et $H_5$''); 3.76 et 3.77 (2s,6H,2 O-$CH_3$); 4.05-4.16 (m,2H,P-O-$CH_2$-); 4.71 et 4.83 (2 pseudo t,1H,$H_4$'); 5.33 (m,1H,$H_3$'); 6.71 (pseudo t,1H,$H_1$',$J_{app}$= 4.9 Hz); 6.8-8 (m,36H,ArH); 8.28 et 8.30 (2s,1H,$H_2$ or $H_8$);, 8.74 et 8.79-(2s,1H,$H_8$ or $H_2$); 8.97 et 9 (2s,1H,NH).

Pour le O-diméthoxytrityl-5' N-palmitoyl-2 O-diphénylcarbamoyl-6 α désoxy-2' guanosine 3'-[(chloro-2-trityl-4 phényl) (cyano-2 éthyl)]phosphate (4d).

Le rendement en mélange diastéréoisomérique était de 53 %, Anal. Calc. pour $C_{88}H_{91}ClN_7O_{11}P$ : C, 70.98; H, 6.16; M, 6. 58; obtenu C, 71.26; H,6.18; N, 6.47. $^{31}$P-NMR ($CDCl_3$) δ -8.15 et -8.52. $^1$H-NMR ($CDCl_3$) δ 0.87 (t,3H,$(CH_2)_{14}$-$CH_3$); 1.15-1.33 (m,24H, $(CH_2)_{12}$-$CH_3$);1.68-1.73 (m,2H,C(=O)-$CH_2$-$CH_2$-); 2.41-2.48 (m,2H,$CH_2$-CN); 2.65-2.74 (m,2H,C(=O)-$CH_2$-$CH_2$-); 3.03 (m,2H,$H_2$, et $H_2$''); 3.19-3.46(m,2H,$H_5$' et $H_5$''); 3.75 et 3.76 (2s,6H,2O-$CH_3$); 3.83-3.95(m,2H,P-O-$CH_2$-); 4.65 et 4.82 (2 pseudo t, 1H,$H_4$'); 5.31-(m,1H,$H_3$'); 6.56 (pseudo t,1H,$H_1$',$J_{app}$= 3.3 Hz); 6.80-7.43 (m,41H,ArH); 7.91 et 7.97 (2S,1H,NH); 8.17 et 8.23 (2s,1H,$H_8$).

7) Méthode générale d'assemblage d'oligodésoxyribonucléotides α

Les oligonucléotides α entièrement protégés ont été assemblés par répétition d'un cycle de réactions sélectives comprenant une détritylation, une décyanoéthylation et une condensation, ces étapes sont classiques.

8) Méthode générale de déprotection des oligodésoxyribonucléotides α contenant des résidus G protégés.

Une solution d'oligonucléotide α entièrement protégé (0,058 mmole) du 2-pyridinealdoxime (1,117 g, 9,15 mmoles) et du N[1],N[1],N[3],N[3]-tétraméthylguanidine (1,15 ml, 9,15 mmoles) dans un mélange dioxanne-eau (7:1, v/v, volume final ajusté à 9,15 ml) a été chauffée à 70 ° C pendant 24 heures. La solution a été évaporée et le résidu a été dissous dans de l'ammoniaque aqueux à 50 ° C pendant 5 heures. Ensuite, la solution a été évaporée et le résidu a été dissous dans de l'acide acétique 80 % (16 ml). Après un traitement acide de 15 mn, la solution a été lavée avec du chlorure de méthylène (5 x 20 ml). La solution aqueuse a été évaporée jusqu'à siccité et les produits ont été chromatographiés sur une colonne (2,25 cm) de DEAE-Séphadex A25 ($HCO_3^-$) utilisant un tampon d'hydrogénocarbonate de triéthylammonium (pH 7,5 gradient linéaire de 0,001 M à 1M sur 1000 ml) comme éluant. Des fractions de 9 ml ont été recueillies et les fractions appropriées ont été combinées et évaporées. Le résidu restant a été traité avec une Dowex 50 W (Na+) donnant l'oligonucléotide α recherché sous forme de sel de sodium. Les deux oligonucléotides α α-[d(CATGCG)] et α-[d(CGCATG)] ont été obtenus respectivement dans des rendements de 59 et 52 %. L'analyse HPLC sur colonne $C_{18}$ indiquait des puretés supérieures à 98 %.

EXEMPLE II : Synthèse de l'hexaribonucléotide α-CpUpCpCpCpU par la méthode au phosphotriester (voir schémas III et IV ci-après)

REFERENCES :

1. S.S JONES, B. RAYNER, C.B. REESE, A. UBASAWA and M. UBASAWA, Tetrahedron, 36, 3075 (1980).
2. R.A. SANCHEZ and L.E. ORGEL, J. Mol. Biol., 47, 531 (1970).
3. W.T. MARKIEWICZ, J. Chem. Res., Synop. 24 (1979) et J. Chem. Res. Miniprint, 181 (1979).
4. J.B. CHATTOPADHYAYA and C.B. REESE, Tet. Letters, 5059 (1979).

La synthèse de l'hexaribonucléotide α-CpUpCpCpCpU décrite dans ce qui suit a été effectuée selon la méthode au phosphotriester en solution. Elle est similaire à celle décrite par C.B. REESE et col. (1) pour la série naturelle β. Elle a nécessité la préparation du synthon nucléosidique 6a constituant l'extrémité 5' de l'oligonucléotide, des synthons 5a et 5b pour l'extension de la chaîne oligonucléotidique et du synthon 7b constituant l'extrémité 3' de l'oligonucléotide. Les deux triribonucléotides partiellement protégés 12 et 16

ont été préparés selon le schéma IV, puis assemblés pour produire l'hexaribonucléotide entièrement protégé 17. Après déprotection, l'hexaribonucléotide α-CpUpCpCpCpU a été purifié et caractérisé.

SCHEMA III

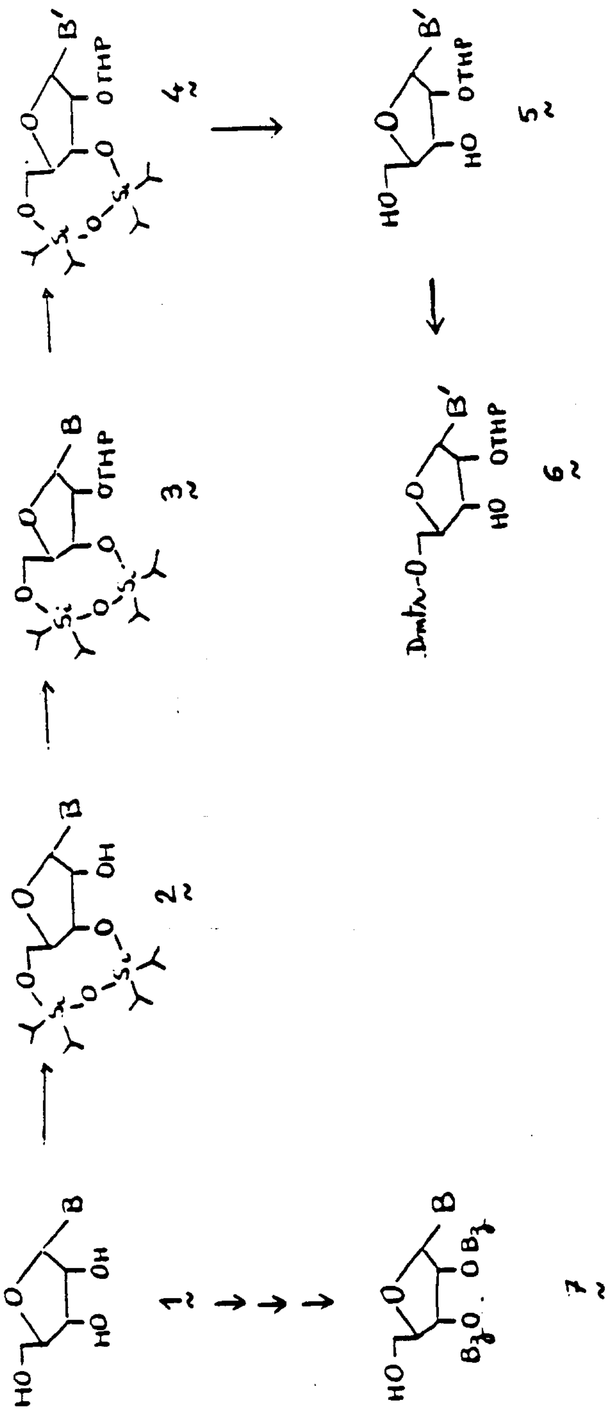


a B : CYTOSINE
b B : URACILE

a B' : N-BENZOYL-4 CYTOSINE ($C^{Bz}$)
b B' : URACILE (U)

THP : TETRAHYDROPYRANYL-2
Dmtr : DIMETHOXY-4,4' TRITYL
Ar : CHLORO-2 PHENYL
Bz : BENZOYL

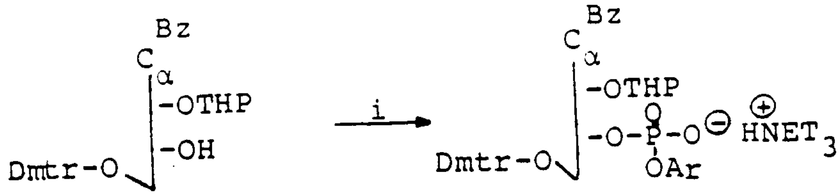


6a 8a

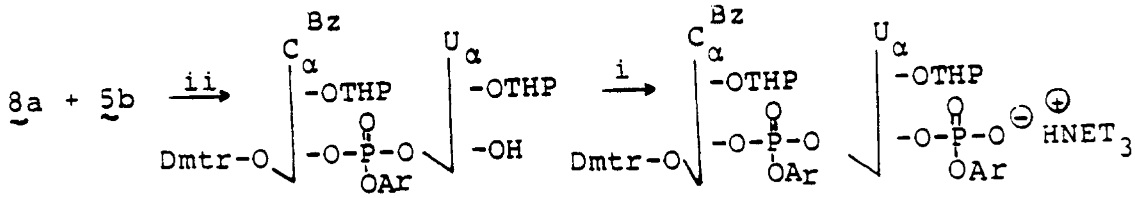


9 10

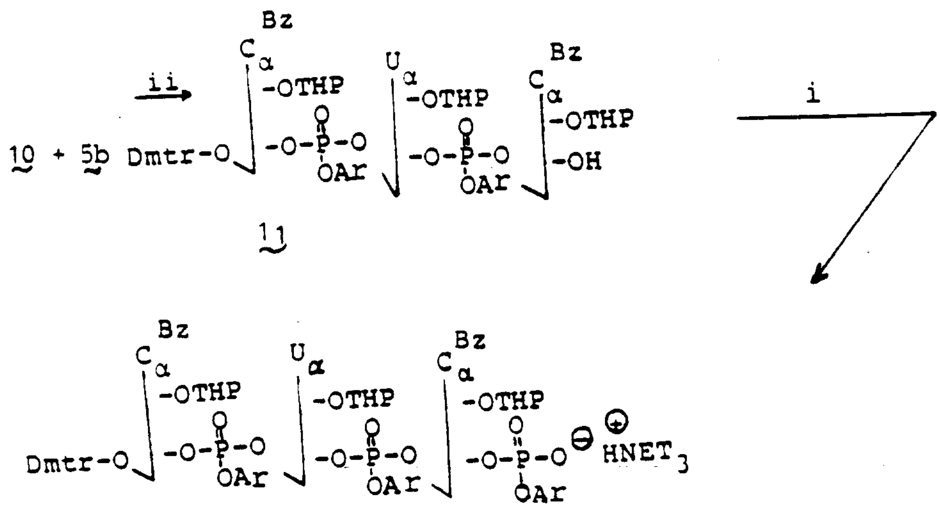


11

12

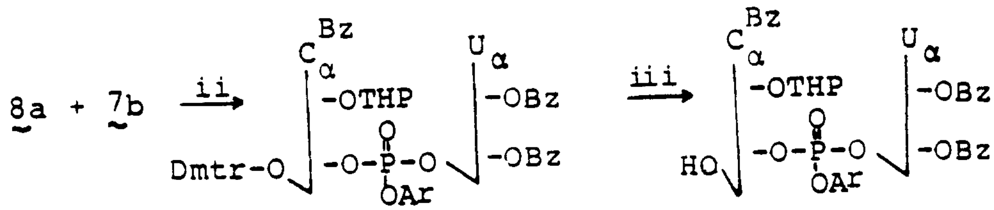


13 14

8a + 14

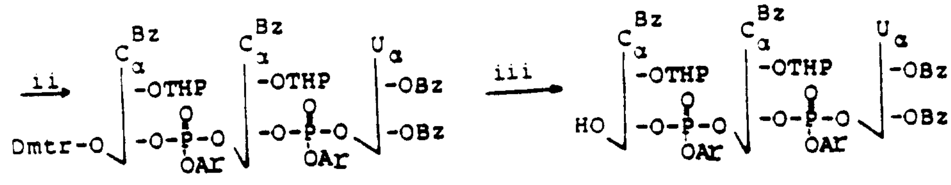


15 16

12 + 16

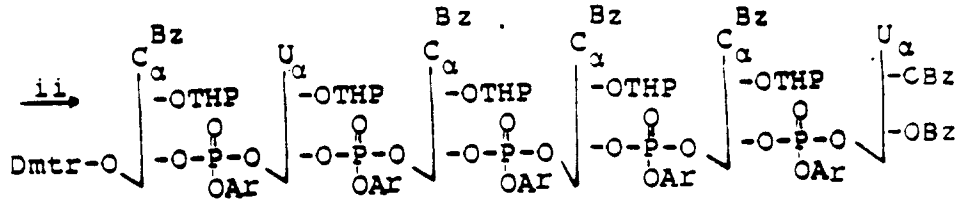


17

THP : TETRAHYDROPYRANNYL-2
Dmtr : DIMETHOXY-4,4' TRITYL
Bz : BENZOYL
Ar : CHLORO-2-PHENYL
Et : ETHYL

i) o-CHLOROPHENYL PHOSPHORODI-(TRIAZOL-1,2,4 IDE)/ACETONITRILE/PYRIDINE PUIS EAU/TRIETHYLAMINE/PYRIDINE.

ii) MESITYLENESULFONYL-1 NITRO-3 TRIAZOLE-1,2,4 (MSNT)/PYRIDINE.

iii) ACIDE TRIFLUOROACETIQUE 0,5 % / DICHLOROMETHANE A 0°C OU ACIDE BENZENESULFONIQUE 2 % / DICHLOROMETHANE METHANOL A 0°C.

SCHEMA IV

1) Préparation des synthons nucléosidiques 5a, 5b, 6a et 7b

Ils ont été préparés à partir des ribonucléosides α 1a et 1b obtenus selon des méthodes décrites dans la littérature (2). L'introduction sélective du groupement protecteur acido-labile tétrahydropyrannyl-2 (THP) a été effectuée selon la méthode suivante (3) (Voir schéma III). Après traitement du composé 1b par le tétraisopropyl-1,1,3,3 dichloro-1,3 disiloxane dans un mélange diméthylformamide-pyridine, le dérivé obtenu 2b est traité par le tétrahydropyranne en présence d'acide trifluoroacétique pour donner 3b. Aprés purification, ce dernier est soumis à un traitement par une solution 1M de fluorure de tétrabutylammonium dans le tétrahydrofuranne. La protection tétraisopropyl-1,1,3,3 disilyle est ainsi sélectivement ôtée et on obtient le composé 5b.

La cytidine α est traitée de façon analogue à celle précédemment décrite avec cependant la modification suivante qui permet d'introduire une protection benzoyle en position 4 de la base cytosine. Après introduction du groupement tétrahydropyrannyle, le composé 3a est traité par le chlorure de

benzoyle dans une solution de pyridine et le composé résultant 4a est traité par une solution 1M de fluorure de tétrabutylammonium dans le tétrahydrofuranne pour donner, après purification, le composé désiré 5a. Le dérivé nucléosidique 6a a été obtenu après tritylation de 5a par le chlorure de diméthoxy-4,4' trityle en milieu pyridine.

Enfin, le synthon nucléosidique 7b, constituant l'extrémité 3' de l'oligonucléotide, a été obtenu en trois étapes à partir de la cytosine α. Ces trois étapes sont : a) tritylation sélective de l'hydroxyle-5' par le chlorure de diméthoxy-4,4' trityle, puis b) benzoylation de positions 2' et 3' par le chlorure de benzoyle et enfin c) déprotection sélective de l'hydroxyle-5' par un traitement à l'acide benzènesulfonique à 2 % dans un mélange dichlorométhane-méthanol (7/3, v/v). Le rendement global de ces trois étapes est de 67 %.

2) Assemblage de l'hexaribonucléotide entièrement protégé α-CpUpCpCpCpU

Le triribonucléoside diphosphate 16 et le triribonucléoside triphosphate 12 ont été préparés selon le schéma III. Chaque cycle d'assemblage comprend une étape de phosphorylation permettant d'introduire un groupement phosphodiester en position 3' d'un dérivé nucléosidique ou nucléotidique partiellement protégé et une étape de couplage entre le composé phosphodiester-3' obtenu à l'étape précédente et un dérivé nucléosidique ou nucléotidique dont la fonction OH-5' est libre. Dans certains cas (voir schéma II), une étape supplémentaire de détritylation est nécessaire pour déprotéger cette fonction OH-5'.

Les étapes de phosphorylation en 3' ont été effectuées par traitement avec de l'O-chlorophénylphosphorodi-(triazol-1,2,4-ide) (4) d'un dérivé OH-3' en solution dans un mélange acétonitrile-pyridine anhydre, suivi d'une hydrolyse en présence de triéthylamine. Ainsi, les dérivés O-arylphosphate-3' 8a, 10 et 12 ont été obtenus avec des rendements supérieurs à 90 %.

Les étapes de couplage entre un dérivé O-arylphosphate-3' et un dérivé nucléosidique ou nucléotidique 5'-OH ont été effectuées en solution dans la pyridine anhydre en présence d'agent de couplage : le mésitylène-sulfonyl-1 nitro-3-triazole-1,2,4 (MSNT) (1).

Ainsi, à titre d'exemple, à une solution de triribonucléoside triphosphate 12 (0,334 mmole) et de triribonucléoside diphosphate 16 (6,278 mmoles) dans de la pyridine anhydre (2 ml), on ajoute du MSNT (1,67 mmole). Le mélange est agité pendant 55 minutes à température ambiante, puis est versé sur une solution d'hydrogénocarbonate de sodium (50 ml). Le mélange obtenu est extrait avec du dichlorométhane (3 x 30 ml). Les phases organiques sont rassemblées et évaporées sous pression réduite. Le résidu est purifié par chromatographie sur colonne de gel de silice (éluant : dichlorométhane-méthanol). Les fractions contenant le produit désiré 17 sont rassemblées, évaporées sous pression réduite et le résidu est précipité avec de l'éther de pétrole (rendement 58 %).

3) Déprotection, purification et caractérisation de l'hexaribonucléotide αCpUpCpCpCpU

L'hexaribonucléotide entièrement protégé 17 a été soumis aux traitements suivants :

. nitro-4 benzaldoximate de tétraméthylguanidinium 1M dans un mélange dioxanne-eau (1/1, v/v) pendant 18 heures.
. ammoniaque concentrée pendant 5 heures à 50 ° C.
. acide chlorhydrique (pH 2) pendant 4 jours à température ambiante.

Au terme de ces traitements, l'oligonucléotide déprotégé α-CpUpCpCpCpU a été purifié par chromatographie sur colonne échangeuse d'anions DEAE-Sephadex (forme $HCO_3^-$) en utilisant comme éluant une solution d'hydrogénocarbonate de triéthylammonium (gradient linéaire O,O1M → 1M sur 1000 ml).

Le rendement de la déprotection a été de 56 %.

Une fraction (environ 5 unités de $DO_{260}$ ) de l'oligoribonucléotide α-CpUpCpCpCpU a été soumise à dégradation enzymatique par la phosphodiestérase de venin de serpent et la phosphate alcaline. L'analyse par chromatographie liquide haute performance des produits de la digestion a indiqué une dégradation complète en α-uridine et α-cytidine dans un rapport 1/2,1.

## EXEMPLE III

### Synthèse supportée d'oligodésoxyribonucléotides α

La synthèse supportée d'α-oligodésoxyribonucléotides, qui est décrite ci-après, procède selon la méthode au phosphoroamidite. Elle est similaire à celle décrite pour la série naturelle β par Caruthers et col. (S.L. Beaucage et M.H. Caruthers, Tetrahedron Lett., 22, 1859-1862 (1981) et L.J. McBride and M.H. Caruthers, Tetrahedron Lett., 24, 245-248 (1983)). Elle a nécessité la préparation (1) des synthons α-

désoxynucléoside-phosphoramidites 2a-d correspondant aux quatre bases T, C, A et G selon le schéma suivant :

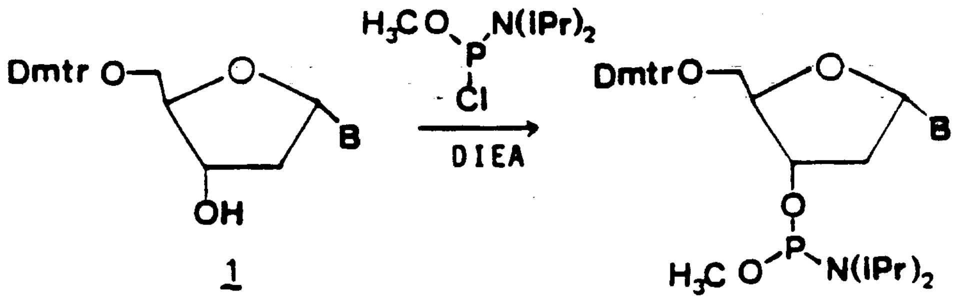


1

2

Les abreviations ont les significations suivantes :

a B = T = Thymine
b B = $C^{BZ}$ = N-benzoyl-4 cytosine
c B = $A^{BZ}$ = N-benzoyl-6 adénine
d B = $G^{Pal}$ = N-palmitoyl-2 guanine.

Dmtr : Diméthoxy-4,4'-trityl iPr : isopropyl $B_Z$ : benzoyl

Cette synthèse a également nécessité la fonctionnalisation d'un support solide (2) incorporant undérivé α-désoxyribonucléosidique.

Dans un deuxième temps, l'élongation de la chaîne oligonucléotidique (3) a été effectuée à l'aide d'un synthétiseur manuel ou automatique. Enfin, au terme du nombre requis de cycles de synthèse, l'oligonucléotide a été décroché du support, déprotégé et purifié.

(1) Préparation des α-désoxyribonucléoside-phosphoramidites 2a-d.

Les quatre α-désoxyribonucléoside-phosphoramidites N-protégés 2a-d ont été obtenus à partir des O-diméthoxytrityl-5' α-désoxyribonucléosides N-protégés correspondants 1a-d . Le-dérivé nucléosidique 1 a été traité par du chloro N,N-diisopropylamino phosphoramidite de méthyle (2,5 équivalents molaires) en présence de N,N,N-diisopropyléthylamine (DIEA) dans le dichlorométhane, sous atmosphère inerte. Le dérivé phosphoramidite obtenu 2 a été purifié par chromatographie sur gel de silice et isolé avec des rendements variant de 79 % à 95 %. L'analyse des quatre dérivés phosphoramidites 2a-d par $^{31}P$-RMN indique une pureté supérieure à 97 %.

La méthode générale de préparation des dérivés N,N-diisopropylamino phosphoramidite-3' de α-désoxy-2'-nucléosides 2a-d a été la suivante.

A une solution de O-diméthoxytrityl-5' N-protégé α-désoxynucléoside 1 (1 mmole) et de N,N,N-diisopropyléthylamine (4 mmoles) dans du dichlorométhane anhydre (3 ml), on ajoute, sous argon et par l'intermédiaire d'une seringue, le chloro N,N-diisopropylamino-phosphoramidite de méthyle (2,5 mmoles). Le mélange réactionnel est agité à température ambiante pendant 10 mn, puis est transféré dans une ampoule à décanter contenant de l'acétate d'éthyle (35 ml). Le mélange est extrait avec de la saumure (4 x 80 ml) et la phase organique est séchée sur sulfate de sodium anhydre et évaporée sous pression réduite. Le résidu d'évaporation est purifié par chromatographie sur gel de silice (10 g) en utilisant comme éluant un mélange hexane-dichlorométhane contenant 1 % de triéthylamine. Les fractions contenant le produit pur sont rassemblées et évaporées sous pression réduite. Le résidu est précipité dans du n-hexane (50 ml) froid (- 78°) à partir d'une solution dans le toluène (5 ml) ou lyophilisé à partir d'une solution dans le benzène (15 ml). Des composés 2a-d sont obtenus sous forme de poudres blanches et conservés sous argon à - 20°.

Les résultats d'analyses des quatre dérivés phosphoramidites 2a-d par $^{31}P$-RMN sont les suivants :

2a : rendement 88% . $^{31}P$-RMN δ 150,9

$^{1}H$-RMN δ 8,19 (large s, 1H, H-$N_3$) ; 7,60 et 7,57 (2d, 1H, $H_6$) ; 7,45-6,79 (m, 13H, ArH) ; 6,34 (pseudo d, 1H, $H_1'$ , $J_{app.}$ = 7,5 Hz) ; 4,63-4,45 (m, 2H, $H_3'$ et $H_4'$) ; 3,80 (s, 6H, $CH_3$-OAr) ; 3,68-3,42 (m, 2H , $H_5'$ et $H_5''$) ; 3,32 et 3,31 (2d, 3H, $CH_3$-O-P, J = 13,3 Hz) ; 3,25 (m, 2H, N[CH-$(CH_3)_2]_2$);2,86-2,65 (m, 1H, $H_2'$) ; 2,30-2,14 (m, 1H, $H_2''$) ; 1,92 (s, 3H, $(H_3C)_5$); 1,17-1,01 (m, 12H, $[(CH_3)_2$-$CH]_2N$).

2b : rendement 79% . $^{31}$P-RMN δ 150,42 et 151,7
$^{1}$H-RMN δ 8,62 (large s, 1H, H-$N_4$) ; 8,12 et 8,11 (2 d, 1H, $H_6$' $J_{5-6}$ = 7,2 Hz) ; 7,90 (d, 1H, $H_5$) ; 7,66-6,82 (m, 18H, ArH) ; 6,35 (pseudo t, 1H, $H_1$' , $J_{app.}$ = 5,5 Hz) ; 4,72 et 4,63 (2 pseudo t, 1H, $H_3$') ; 4,54-4,47 (m, 1H, $H_4$') ; 3,80 (s, 6H, $CH_3$-O Ar) ; 3,52-3,35 (m, 2H, $H_5$' et $H_5$") ; 3,29 et 3,23 (2d, 3H, $CH_3$-O-P, J = 13,3 Hz) ; 3,29-3,10 (m, 2H, N[CH-$(CH_3)_2$]$_2$);2,93-2,71 (m, 1H, $H_2$') ; 2,53-2,36 (m, 1H, $H_2$") ; 1,12-0,98 (m, 12H, [$(CH_3)_2$-CH]$_2$N).

2c : rendement 95% . $^{31}$P-RMN δ 150,81 et 150,91
$^{1}$H-RMN δ 8,97 (s, 1H, NH) ; 8,84 et 8,59 (2d, 2H, $H_2$ et $H_8$) ; 8,04-6,81 (m, 18H, ArH) ; 6,74-6,68 (m, 1H, $H_1$') ; 4,67 -4,56 (m, 2H, $H_3$' et $H_4$') ; 3,80 (s, 6H, $CH_3$-OAr) ; 3,61-3,43 (m, 2H, $H_5$' et $H_5$") ; 3,28 et 3,26 (2d, 3H, $CH_3$-O-P, J = 13 Hz) ; 3,40-3,16 (m, 2H, N[CH-$(CH_3)_2$]$_2$) ; 3,0-2,9 (m, 1H, $H_2$') ; 2,72-2,53 (m, 1H, $H_2$") ; 1,16-1,01 (m, 12H, [$(CH_3)_2$-CH]$_2$N).

2d : rendement 87% . $^{31}$P-RMN δ 150,76 et 150,97
$^{1}$H-RMN δ 11,88 (large s, 1H, NH) ; 8,28 et 8,24 (2 s, 1H, $H_8$) ; 7,46-6,82 (m, 14H,NH et ArH) ; 6,28 (pseudo t, 1H, $H_1$' , $J_{app.}$ = 7,8 Hz) ; 4,70-4,44 (m, 2H, $H_3$' et $H_4$') ; 3,80 (s, 6H, $CH_3$-O Ar) ; 3,76-3,41 (m, 2H, $H_5$' et $H_5$") ; 3,31 et 3,25 (2d, 3H, $CH_3$-O-P, J = 13,5 H) ; 3,36-3,12 (m, 2H, N [CH-$(CH_3)_2$]$_2$);2,94-2,80 (m, 1H, $H_2$') ; 2,61-2,36 (m, 3H, $H_2$" et $(CH_2)_{13}$-$CH_2$-C(=O)) ; 1,70 (m, 2H, $CH_2$-$CH_2$-C(=O)) ; 1,25 (m, 24H, $CH_3$-$(CH_2)_{12}$) ; 1,17-1,08 (m, 12H, [$(CH_3)_2$-CH]$_2$N) ; 0,88 (m, 3H, $CH_3$-$(CH_2)_{14}$).

(2) Fonctionnalisation du support solide

Elle a été effectuée par l'établissement d'un lien succinyle entre le groupement hydroxyle-3' du dérivé N-acyl-O-diméthoxytrityl-5' α-désoxynucléoside 1 et le groupe amino du support solide selon le schéma suivant :

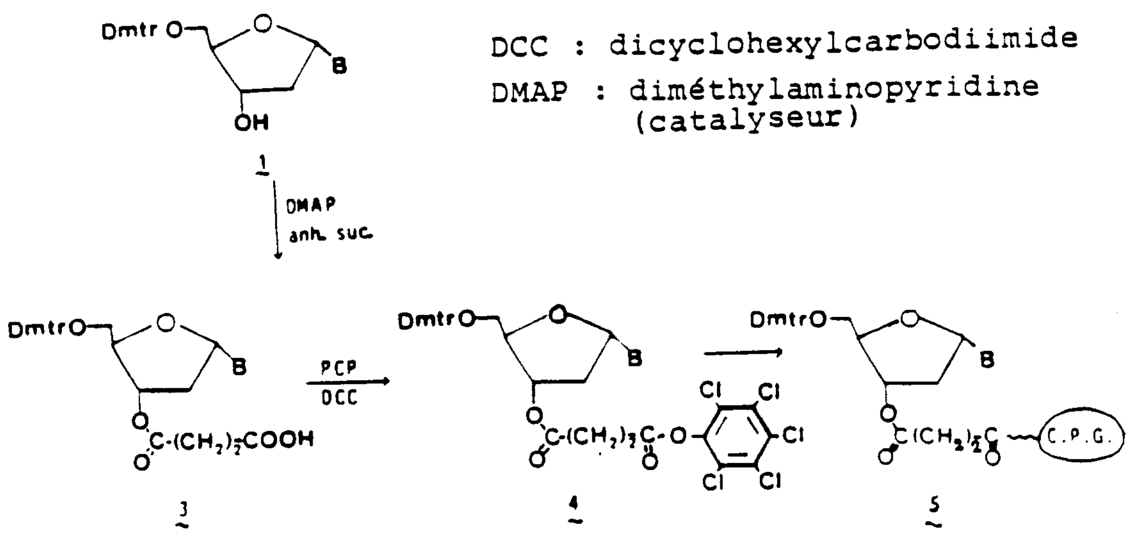


Le composé 1 a préalablement été transformé en dérivé pentachlorophényl succinate-3' 4 et ensuite mis en réaction avec le groupement amino de la longue chaîne alkylamine fixée sur billes de verre poreuses (LCA-CPG) (S.P. Adams, K.S. Kavka, E.J. Wykes, S.B. Holder and G.R. Galluppi, J. Am. Chem. Soc., 105, 661 (1983)).

La quantité de nucléoside fixé sur le support solide a été déterminée par dosage spectrophotométrique du cation diméthoxytrityle libéré par un traitement avec une solution à 10 % d'acide trichloroacétique dans le dichlorométhane. Le degré de fonctionnalisation varie de 25 à 38 μmoles par gramme suivant la nature de la base nucléosidique.

(3) Synthese de α-d(T A C G G C C A G T)

La décanucléotide α-d(TACGGCCAGT) a été synthétisé manuellement. La O-diméthoxytrityl-5'α-thymidine liée de façon covalente au support LCA-CPG (1 μmole environ) est introduite dans un petit réacteur. L'élongation de cet oligonucléotide a été effectuée par une succession de neuf cycles de synthèse (voir tableau III) ci-après).

Outre les lavages, chaque cycle comprend une détritylation (étape 4), une condensation (étape 12), une acétylation (étape 13) et une oxydation (étape 15). Au terme du nombre de cycles requis, le support a été traité par mélange thiophénol/triéthylamine/dioxane (1/2/3, v/v/v) pendant une heure pour enlever le groupement méthyle des phosphates. Puis le support a été traité avec de l'ammoniaque concentré à 60° pendant douze heures, afin d'enlever les groupements protecteurs des amines hétérocycliques et détacher les produits nucléotidiques du support solide. L'oligonucléotide désiré, portant encore le groupement diméthoxytrityl à son extrémité 5', a été purifié par chromatographie liquide haute performance sur colonne de phase greffée $C_{18}$ avec un rendement de 20 %. Un traitement par de l'acide acétique à 80 % a permis de déprotéger son extrémité 5'. Enfin, le décanucléotide purifié a été soumis à dégradation enzymatique par la phosphodiestérase de venin de serpent et la phosphatase alcaline. L'analyse par chromatographie liquide haute performance des produits de la digestion a indiqué une dégradation complète en α-thymidine, α-désoxy-2'-cytidine, α-désoxy-2'-adénosine et α-désoxy-2'-guanosine dans un rapport 2,1/3,2/1,8/3,3 (Rapport théorique : 2/3/2/3).

Ces résultats montrent que des oligodésoxyribonucléotides comprenant uniquement des unités nucléotidiques de configuration anomérique α peuvent être rapidement synthétisés par la méthode au phosphoramidite sur support solide.

Le protocole pour la synthèse manuelle d'α-oligonucléotides sur billes de verre poreux (CPG) a été le suivant.

Le réacteur était chargé avec 30 mg de support fonctionnalisé (25-38 µmoles/g).

Le volume correspondant à chaque étape est de 1 ml, sauf indication contraire.

TABLEAU III

| Etape | Réactif ou Solvants | Temps |
|---|---|---|
| 1, 2, 3 | $CH_2CL_2$ | 10 s par étape |
| 4 | DCA/$CH_2CL_2$ (1/50, v/v) | 3 mn |
| 5, 6 | $CH_2CL_2$ | 10 s par étape |
| 7, 8, 9, 10, 11 | $CH_3CN$ | 10 s par étape |
| 12 | O,1M phosphoramidite* (0,2 ml) at 0,4M tétrazole (0,2 ml) dans acétonitrile | 5 mn |
| 13 | DMAP/THF/lutidine (6/90/10, p/v/v), puis 0,1 ml d'anhydride acétique | 2 mn |
| 14 | THF/lutidine/$H_2O$ (2/2/1, v/v/v) | 1 mn |
| 15 | 0,1M $I_2$ dans THF/lutidine/$H_2O$ (2/2/$1^2$, v/v/v) | 30 s |
| 16, 17, 18, 19 | $CH_3CN$ | 10 s par étape |

* Dans le cas de G et pour des raisons de solubilité, une solution 0,05 M de phosphoramide est utilisée. Dans ce cas, l'étape 12 est effectuée une seconde fois avant de passer à l'étape 13.

EXEMPLE IV : Synthèse automatique d'oligodésoxyribonucléotides α

Un synthétiseur de gènes, modèle 380 d'Applied Biosystems, a été utilisé. En raison de la faible solubilité du dérivé phosphoramidite de G dans l'acétonitrile, une solution 0,05M de ce phosphoramidite, maintenue en permanence à 37°C, a été utilisée. Le programme standard de synthèse a été modifié de façon à répéter les étapes correspondant à l'incorporation de G et à assurer un haut rendement de couplage (supérieur à 98 %).

L'oligonucléotide α-d(TAAAAGGGTGGGAATC) a ainsi été obtenu (62 unités de $DO_{260}$). Sa pureté et sa taille ont été vérifiées par électrophorèse sur gel dans des conditions dénaturantes.

EXEMPLE V : Stabllité enzymatique des oligonucléotides α- et β-[d(CATGCG)] vis-à-vis de certaines nucléases.

Trois nucléases ont été utilisées : une endonucléase, la nucléase S1 qui est spécifique des ADN monobrins ; une 5'-exonucléase, la phosphodiesterase de rate de veau ; et une 3'-exonucléase, la phosphodiesterase de venin de serpent. L'hyperchromicité à 260 mm et l'HPLC ont été utilisés pour suivre l'hydrolyse enzymatique. Aucun changement significatif dans l'absorbance n'est apparue en 20 mn quand l'oligomère α a été incubé avec la nucléase S1, tandis que l'oligomère β donnait une augmentation d'absorbance avec la durée.

L'absorption UV ne permet pas une comparaison quantitative entre les oligomères β et α s'agissant de leur dégradation. Nous avons donc confirmé ces résultats par une analyse HPLC. La disparition du pic correspondant à l'hexanucléotide a été enregistrée pendant des incubations avec la nucléase S1 à 37° C. Il n'y avait plus d'hexamères 3 après 10 mn d'incubation, tandis que seulement 7 % de l'hexamère α étaient dégradés (Tableau IV).

La stabilité comparative des deux hexamères α et β par rapport à l'hydrolyse par la phosphodiesterase de ratede veau est illustrée au Tableau II ci-après. La différence entre les deux oligomères anomériques est frappante : aucun changement de l'oligomère α est apparu en 10 mn, tandis que 89 % de l'oligomère β était hydrolysé dans le même temps.

TABLEAU IV

| Hydrolyse de[d(CATGCT)] α- ou β- (166 mmoles chacun) en fonction du temps par la nucléase S1 (93 unités/ml) et la phosphodiesterase de rate de veau (0,013 unité/ml) à 37° C. Fraction d'hexamère restant après 1 ou 10 mn. | | | | | |
|---|---|---|---|---|---|
| | Nucléase S1 durée (min.) | | | Phosphodiesterase de rate de veau durée (min.) | |
| | 0 | 1 | 10 | 0 | 10 |
| β-[d(CATGCG)] | 100% | 1.3% | 0 | 100% | 11% |
| α-[d(CATGCG)] | 100% | 98% | 93% | 100% | 104% |

La stabilité des deux hexamères anomériques vis-à-vis de l'hydrolyse par la phosphodiesterase de venin de serpent a été étudiée en suivant l'absorbance UV à 260 mm. A de faibles concentrations en enzyme , l'oligonucléotide α a été hydrolysé lentement, tandis que la dégradation de l'oligonucléotide β correspondant était très nette.

1) Comparaison des substrats [d(CATGCG)] α- et β- par rapport à différentes nucléases.

Analyse HPLC : L'appareil HPLC était un Waters Associates et était équipé avec une colonne C 18 Bondapak. L'élution isocratique a été effectuée avec le système suivant : de l'acétonitrile 27 % dans de l'acétate ammonium 20 mM (pH 6,5) et la procédure de détection à 260 nm.

a) Nucléase S1.

Cette nucléase était de marque GIBCO BRL.

Une unité solubilise à 1 µg d'ADN dénaturé en 1 mn à 37° C.

Une solution d'oligonucléotide α- ou d'oligonucléotide β- (50 nmol.) dans de l'eau (30 µl) a été mélangée avec le tampon suivant (30 µl) : acétate de sodium 0,5 M (pH 4,7), chlorure de sodium 3 M, acétate de zinc 0,1 M. Ce volume a été complété avec 300 µl d'eau. La nucléase S1 (28 unités, 3 µl) a été ensuite ajoutée. Le mélange a été incubé à 37° C et les aliquots (20 µl) ont été prélevés en temps voulu et mélangés avec le tampon bloquant : phosphate de potassium 20 mM (pH 6,5, 180 µl),avant d'être chauffés à 100° C pendant 5 minutes. Un traitement similaire a été fait pour les expériences de contrôle. Des volumes de 50 µl étaient injectés.

b) Phosphodiesterase de rate de veau.

Une procédure similaire à celle décrite ci-dessus a été suivie, si ce n'est qu'on a additionné ladite phosphodiesterase ($4.10^{-3}$ unités, 1 µl) à la place de la nucléase S1 dans 0,1 M d'acétate d'ammonium (pH 6,5). La phosphodiesterase de rate de veau était fournie par BOEHRINGER (MANNHEIN). Une unité hydrolyse 1 µmol. de thymidine-3'-nitrophénylphosphate en une minute à 25° C. La réaction a été arrêtée dans de l'eau à la place d'une solution de phosphate de potassium.

Spectrophotométrie UV.

Un spectrophotomètre Uvikon à 810 équipé avec un système thermostaté opérant à 37° C a été utilisé. Le volume total de réaction était de 1 ml. Les oligonucléotideS (60 nmol.) ont été ajoutés et l'absorbance à

260 nm a été ajustée à zéro. La nucléase S1 (1 $\mu$l, 9,3 unités) a été ajoutée et on a enregistré l'absorbance à 260 nm en fonction du temps. La procédure analogue a été utilisée avec la phosphodiesterase de venin de serpent également fournie par BOEHRINGER (1 $\mu$l, $3.10^{-6}$ unités).

La figure 1 represente les courbes d'absorbance UV à 260 nm en fonction du temps pour respectivement les oligonucléotides [d(CATGCG)] 3- et $\alpha$- . Une solution tampon était constituée d'acétate de sodium 0,05 M (pH 4,7), de chlorure de sodium 0,3 M et d'acétate de zinc 0,01 M. At = 0 la nucléase S1 (0,3 unité/ml) a été ajoutée à 60 $\mu$M d'hexamère $\alpha$- ou $\beta$-.

La figure 2 représente l'absorbance UV à 260 nm en fonction du temps. La solution tampon a été constituée de tris-HCl 0,1 M (pH 9) et de chlorure de magnésium 0,01 M. Au temps t = 0 mn de la phosphodiesterase de vénin de serpent ($3.10^{-6}$ unités/ml) a été ajoutée à 66 $\mu$M d'hexamère $\alpha$- ou $\beta$- à 37 ° C.

<u>EXEMPLE VI</u> : <u>Appariement parallèle de deux brins $\alpha$ et $\beta$</u>

A partir des séquences $\alpha$ synthétisées, comprenant les quatre bases, les résultats ci-après prouvent que les séquences $\alpha$ et $\beta$ sont parallèlement appariées, alors que deux séquences $\alpha$ complémentaires s'apparient anti-parallèlement. D'autre part, l'appariement $\alpha$ $\beta$ est beaucoup plus stable que l'appariement $\beta$ $\beta$.

En effet, le spectre RMN des protons imino à 4 ° C de ces solutions dans $D_2O$-$H_2O$ révèle que

a) la séquence $\alpha$-[d(CATGCG)] présente deux pics bien définis à 12,57 et 14,17 ppm d'intensité ≃ 1 : 1) provenant de l'appariement anti-parallèle :

```
5'-C A T G C G
   | | | |
G C G T A C-5'
```

b) la séquence $\beta$-[d(GTACGC)] présente deux pics plus larges à 13,65 et 12,58 ppm. Là encore, l'intensité relative et des considérations de symétrie suggèrent que ceci est dû à l'appariement anti-parallèle :

```
5'-G T A C G C
   | | | |
C G C A T G-5'
```

c) le mélange des séquences des $\alpha$[d(CATGCG)] et $\beta$-[d(GTACGC)] (1:1) présente six pics imino à 14,22 , 13,83 , 13,23 , 13,02 , 12,73 and 12,57 ppm et un large pic à 13,66 ppm. Puisque les déplacements chimiques du premier ensemble de six signaux sont tous différents de ceux observés en (a) et (b) (excepté pour $\delta$ = 12,57 lequel est présent à la fois pour les séquences $\alpha$- et $\beta$-), il semble raisonnable d'associer ces six pics à ceux des protons imino provenant de l'appariement de $\alpha$-[d(CATGCG)]-$\beta$-[d-(GTACGC)].Le nombre des signaux de protons imino observés est plus en accord avec un appariement parallèle (A, qu'avec un appariement anti-parallèle (B) :

```
       5'-C A T G C G              5'-C A T G C G
(A)       | | | | | |       (B)       |   |   |
       5'-G T A C G C               C-G C A T G-5'
```

EXEMPLE VII : Association d'un tétramère de thymidine α avec un agent d'intercalation

Un tétramère de thymidine α, lié de façon covalente par l'intermédiaire d'une chaîne pentaméthyléne à un dérivé de 2-N-méthyl-9-hydroxyellipticinium acétate, a été synthétisé en trois étapes selon le schéma suivant

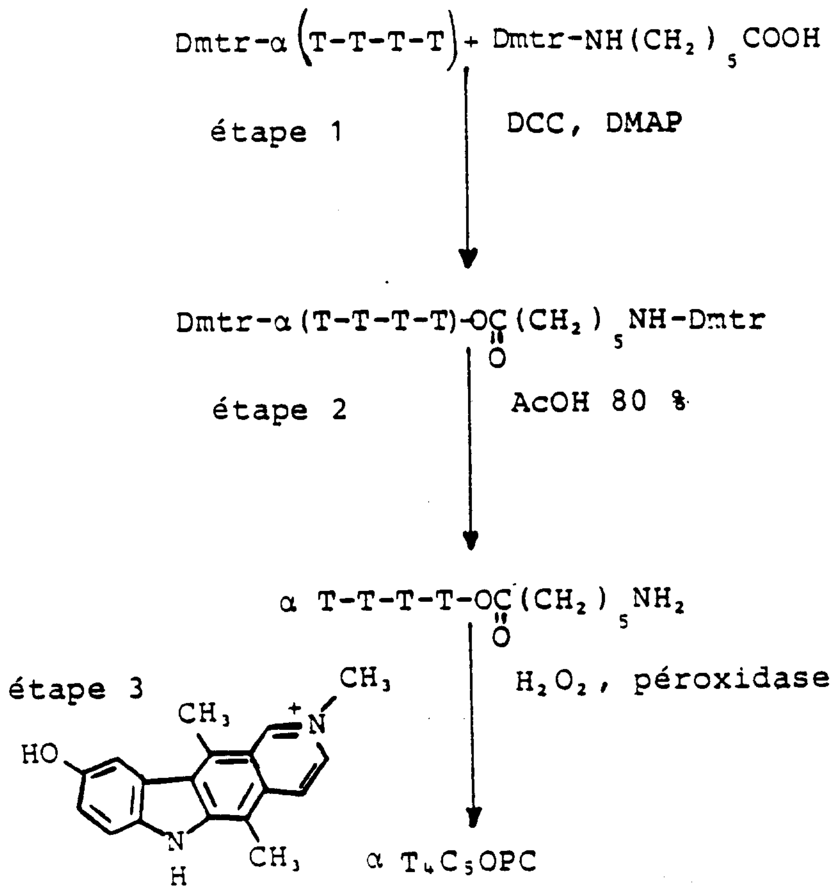


- Etape 1 : Liaison du bras pentaméthylène sur le tétramère de thymidine α.

La terminaison OH 3' du tétramère protégé en 5' avec un groupe diméthoxytrityle (Dmtr) est esterifiée avec un acide aminocaproïque, dont le résidu terminal est bloqué avec Dmtr. La réaction est exécutée à température ambiante dans de la pyridine avec du dicyclohexylcarbodiimide (DCC) comme agent de couplage et du diméthylaminopyridine (DMAP) comme catalyseur. La réaction est complète après trois heures.

- Etape 2 : Déprotection.

Les groupes Dmtr sont éliminés avec de l'acide acétique à 80 %. Le produit résultant :

$$\alpha\left[(Tp)_3T\right]-3'O-\underset{\underset{O}{\|}}{C}-(CH_2)_5-NH_2,$$

ainsi obtenu, est purifié à travers une colonne en phase inverse. A ce point, le rendement des réactions (1 + 2) est égal à 65 %.

- Etape 3 : Liaison du dérivé ellipticine avec le tétramère modifié.

Dans cette étape, nous avons utilisé un système enzymatique : péroxydase de raifort -$H_2O_2$. Le dérivé ellipticine N-méthyl-2 hydroxy-9 ellipticinium (NMHE) est oxydé par cette réaction enzymatique, conduisant à un dérivé O-quinone de NMHE. Cet intermédiaire électrophile ensuite subit l'attaque de la fonction amino portée par le tétramère modifié. Un noyau oxazole est ainsi créé, conduisant au composé fluorescent

$$\alpha\ [(Tp)_3T]-3'O-\underset{\underset{O}{\|}}{C}-(CH_2)_4-OPC \equiv \alpha-T_4-C_5-OPC$$

de formule suivante

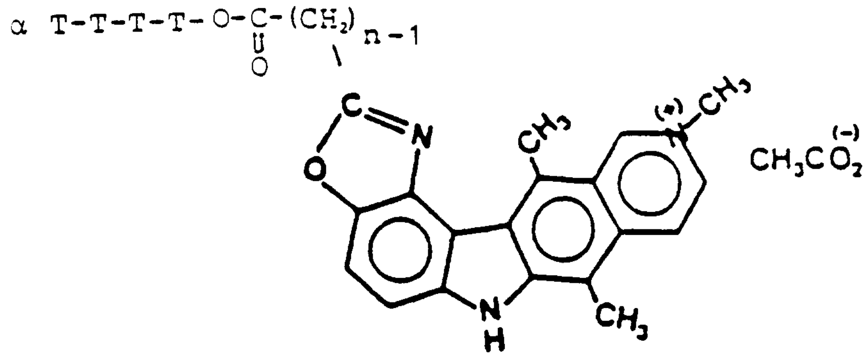

$\alpha\ T_4C_nOPC$

La réaction est effectuée dans un tampon phosphate pH 7 et le produit final est purifié sur une colonne C 18 en phase inverse et par HPLC.

1) Préparation de O-benzoyl-3' α-thymidine

389,7 mg (1,15 mmole) de 4-4'-diméthoxytrityl-chlorure ont été ajoutés a une solution de 2'-α-thymidine (1 mmole et 242,2 mg) dans de la pyridine anhydride (2,5 ml). Le mélange résultant a été agité pendant trois heures, à température ambiante, et on a ajouté du méthanol (1 ml). Après une agitation additionnelle de 15 mn, le mélange a été versé dans 40 ml d'hydrogénocarbonate de sodium aqueux saturé et extrait avec du chlorure de méthylène (3 x 20 ml). Les couches organiques mélangées ont été évaporées jusqu'à siccité et le résidu a été fractionné par chromatographie en utilisant comme éluant un mélange acétone-chlorure méthylène (0 à 40 %). Les fractions contenant du O-diméthoxytrityl-5' α-thymidine pur ont été rassemblées et évaporées jusqu'à siccité. Le résidu a été précipité à partir de l'éther (inférieur à 40 ° C) (514,6 mg 94,5 %).

42,2 mg de (0,30 mmole) de chlorure de benzoyl ont été ajoutés à une solution de 5'-O-diméthoxytrityl-α-thymidine (0,25 mmole 0,136 g) dans de la pyridine anhydre (1 ml). La solution a été agitée pendant une heure à température ambiante et on a ajouté 0,5 ml d'eau. Après 15 mn d'agitation, le mélange de réaction a été versé dans 20 ml d'hydrogénocarbonate de sodium aqueux saturé et extrait avec du chlorure de méthylène (3 x 10 ml). Les couches organiques ont été évaporées jusqu'à siccité. Le résidu a été dissous dans du chlorure de méthylène-méthanol (7-3 v/v ; 5 ml) et 10 % (v/v) d'acide benzènesulfonique dans du chlorure de méthylene ont été également ajoutés. Après 17 mn d'agitation à température ambiante, le mélange réactionnel a été neutralisé avec 2 ml d'hydrogénocarbonate de sodium aqueux saturé et réparti entre 20 ml d'hydrogénocarbonate de sodium aqueux saturé et (3 x 15 ml) de chlorure de méthylène.Les phases organiques rassemblées ont été évaporées jusqu'à siccité et le résidu a été fractionné par chromatographie (10 g). Les fractions contenant du dérivé 3'-O-benzoyl-α-thymidine ont été réunies et évaporées. Le résidu a été précipité à partir d'éther (inférieur à 40 ° C) pour donner une poudre amorphe incolore (0,075 g, 87 %).

2) Préparation de 5'-O-diméthoxytrityl-$\alpha$-[$(Tp)_3T$]

A 0,393 g (0,15 mmole) d'une solution de Dmtr-$\alpha$-$(TP)_3T$-Bz protégée dans de l'eau-dioxane (1/7, v/v, 5 ml) était ajouté 0,6106 g ( 5mmoles) de pyridine-2-aldoxime et 0,627 ml (5 mmoles) de $N^1,N^1,N^3,N^3$-tétraméthylguanidine. La solution résultante a été chauffée à 70 ° C pendant 9,5 h, puis refroidie et évaporée jusqu'à siccité. Le résidu a été dissous dans de l'hydrogénocarbonate de tétrylammonium (TEAB, 50 ml, 50 mmoles) et a été lavée avec du diéthyléther (2 x 25 ml). La solution aqueuse a été évaporée jusqu'à siccité et le résidu a été chromatographié sur une colonne de $C_{18}$ de silica gel en phase inverse (1,2 x 10 cm) en utilisant un gradient de méthanol (0 à 50 %) dans de l'eau (0,1 M par rapport à TEAB). Les fractions appropriées ont été réunies et évaporées jusqu'à siccité, La lyophilisation du résidu a donné le composé souhaité sous forme de poudre incolore (0,223 g) dans un rendement de 84 %.

3) Préparation de composé $\alpha$-$(Tp)_3T$ 3'-O-$\epsilon$-aminohexanoate

A une solution de 5'-O-diméthoxytrityl-$\alpha$-$(TP)_3T$ (0,223 mg , 0,13 mmole) et d'acide N-diméthoxytrityl-$\omega$-aminohexanoïque (0,281 g , 0,65 mmole), dans de la pyridine anhydre (3 ml) ont été ajoutés 0,2678 g (1,3 mmole) de dicyclohexylcarbodiimide et 0,0793 mg (0,65 mmole) de N,N-diméthylamino-4-pyridine. La solution résultante a été agitée pendant trois heures et demie à température ambiante. 1 ml d'eau a ensuite été ajouté et le mélange a été filtré. Le filtrat a été évaporé jusqu'à siccité et le résidu a été précipité à partir de diéthyléther (cette dernière opération a été répétée trois fois). Le précipité a été dissous dans de l'acide acétique à 80 % (5 ml) et la solution a été gardée à température ambiante pendant 20 mn. Le mélange a été extrait avec du diéthyléther, évaporé jusqu'à siccité. Au résidu on a ajouté du toluène et le mélange résultant a été évaporé jusqu'à siccité là encore. Le résidu a été purifié par chromatographie sur colonne $C_{18}$ silica gel en phase inverse (1,2 x 10 cm) en utilisant un gradient étagé de méthanol de (0 à 40 %) dans l'eau. Les fractions contenant le produit ont été évaporées jusqu'à siccité et le résidu a été lyophilisé . Le composé recherché a été obtenu sous forme de poudre incolore (0,130 g) pour un rendement de 65 %.

4) Préparation de $\alpha$-$T_4$-O-$\underset{\overset{\|}{O}}{C}$-$(CH_2)_4$-OPC

Les quantités de réactif mises en oeuvre étaient les suivantes.

Pour 130 mg (0,082 mmole de $\alpha$-$(Tp)_3T$-3'O-$\epsilon$-aminohexanoate et 11 mg (0,032 mmole) de 2-méthyl, 9-hydroxyellipticinium acétate ont été dissous dans 50 ml d'un tampon phosphate O,O2 M pH 7. La solution a été maintenue et sous agitation continue à température ambiante. 0,8 mg de péroxydase (raifort) ont été ajoutés au mélange. Après la dissolution de l'enzyme, 1,5 ml d'une solution de $H_2O_2$ 20 mM ont été ajoutés goutte à goutte. La solution a été filtrée et évaporée jusqu'à siccité. Le résidu a été ensuite prépurifié par chromatographie sur colonne Lighrosorbe RP 18 en phase inverse (0 à 60 %) dans l'eau. Les fractions appropriées ont été combinées et évaporées jusqu'à siccité. La purification du produit a été accomplie par HPLC. Le produit a été passé à travers une colonne Dowex 50 W ($NH_4$ + forme). Après lyophilisation, le composé final obtenu était une poudre jaune de (2,7 mg) en un rendement de 5 %.

EXEMPLE VIII : Appariement de $\alpha$-$T_4$-$C_5$-OPC avec un poly(rA)

L'interaction entre $\alpha$-$T_4$-$C_5$-OPC et poly(rA) a été étudiée en utilisant l'absorption UV et la fluorescence. Le spectre UV de $\alpha$-$T_4$-$C_5$-OPC présente deux maximums à 271 nm et 317 nm. En outre, ce composé est fluorescent avec excitation et émission maximum respectivement à 318 nm et 525 nm.

En présence de quantités croissantes de poly(rA), le spectre de $\alpha$-$T_4$-$C_5$-OPC (0,05 mM dans du cacodylate de sodium 10 mM, NaCl 100 mM, pH 7) est modifié. Une hypochromicité apparaît entre 260 nm et 320 nm et correspond à l'association de $\alpha$-$T_4$-$C_5$-OPC avec la séquence poly(rA). A 270 nm, on peut suivre la formation du duplex entre la chaîne tétra-$\alpha$-thymidilate et poly(rA), tandis qu'à 310 nm, on suit l'interaction du fragment oxazolopyridocarbazole. Par le rapport de l'absorbance de l'$\alpha$-$T_4$-$C_5$-OPC libre sur l'absorbance initiale de l' $\alpha$-$T_4$-$C_5$-OPC, en fonction du rapport de la concentration de poly(rA) exprimée en nucléotides, sur la concentration de dérivé oxazolopyridocarbazole, on peut déduire la stoechiométrie de l'interaction, laquelle correspond à une molécule de $\alpha$-$T_4$-$C_5$-OPC liée pour 4 résidus adénine. Dans les mêmes conditions, aucune association entre $\alpha$-$T_4$ et poly(rA) a été détectée.

On peut également étudier l'association de l'$\alpha$-$T_4$-$C_5$-OPC avec poly(rA) en fonction de la température. Une fois que le complexe a été formé à 8° C, le spectre UV de l'$\alpha$-$T_4$-$C_5$-OPC libre a été progressivement retrouvé en élevant la température. Deux points isobestiques apparaissent à 347 nm et 324 nm. Pour les températures supérieures à 26° C, l'absorbance à 317 nm est plus grande que celle correspondant au $\alpha$-$T_4$-$C_5$-OPC libre à 8° C. Ceci indique que un détachement de la molécule $\alpha$-$T_4$-$C_5$-OPC apparaît quand la température est élevée. La température correspondant à 50 % de dissociation du complexe est égale à 24,5° C à 270 nm et 26° C à 310 nm. Ce résultat pourrait être comparé à celui observé pour des complexes formés entre $\beta$-$T_4$-$C_5$-OPC et poly(rA). Dans ce dernier cas, la température de dissociation du complexe, dans les mêmes conditions, était de 16° C.

La fluorescence du fragment OPC de la molécule $\beta$-$T_4$-$C_5$-OPC n'est pas changée après formation du complexe avec poly(rA). Ceci suggère que ce fragment est probablement non intercalé entre les paires de base nouvellement formées. Ceci constitue une autre différence entre les $\alpha$- et $\beta$-$T_4$-$C_5$-OPC, dans la mesure où, dans le dernier cas, l'interaction entre la substance et le poly(rA) provoque une augmentation de fluorescence.

En outre, en variant la concentration de $\alpha$-$T_4$-$C_5$-OPC, et en mesurant la température Tm correspondante (Tm représente la température de dissociation à 50 % du complexe), les paramètres thermodynamiques de son interaction avec poly(rA) ont été déterminés. Ces paramètres se déduisent de l'équation suivante :

$$1/Tm = S/ H + 2{,}3R/ H \log Cm$$

équation dans laquelle Tm représente la température de demi-dissociation du complexe,
Cm représente la concentration de $\alpha$-$T_4$-$C_5$-OPC libre t = Tm et R est égal à 2 calories/mole/K.

Tm a été mesuré à 270 nm et 310 nm, et les valeurs suivantes ont été obtenues :

à 270 nm : $\Delta H$ = - 38,7 kcal/mole, $\Delta S$ = 105,3 cal/mole et $\Delta G$ à 298° K = - 7,2 kcal/mole.
à 310 nm : $\Delta H$ = - 36,8 kcal/mole, $\Delta S$ = 98,8 cal/mole et $\Delta G$ à 298° K = - 7,4 kcal/mole.

En conclusion, il a été possible de synthétiser une nouvelle molécule : $\alpha$-$T_4$-$C_5$-OPC, dans laquelle un agent d'intercalation potentiel ayant une structure oxazolopyridocarbazole est lié de façon covalente par l'intermédiaire d'un bras pentaméthylène à une tétrathymidilate non naturelle consistant exclusivement d'unités nucléotide $\alpha$-anomérique. L'absorption UV et la fluorescence indiquent que le fragment oligonucléotide fournit les éléments de reconnaissance nécessaires pour se lier sélectivement à la séquence complémentaire par appariement de base. En comparaison avec l'anomère $\beta$- on constate deux différences significatives :

a) la configuration anomérique $\alpha$- conduit à un effet de stabilisation supplémentaire dans la formation du complexe avec poly(rA) ;

b) le résidu oxazolopyridocarbazole ne semble pas s'intercaler dans le duplex formé par appariement de base entre l'oligonucléotide $\alpha$- et poly(rA).

EXEMPLE IX : Résistance des substrats $\alpha$-$T_4$-$C_5$-OPC vis-à-vis d'un nucléase S1.

La dégradation par l'endonucléase S1 a été étudiée comparativement sur les $\alpha$- et $\beta$-$T_4$-$C_5$-OPC par HPLC. L'intensité du pic a été suivie en fonction du temps de réaction avec nucléase S1 à 37° C. Après 20 mn de réaction, le composé a complètement disparu quand la configuration anomérique de la chaîne oligonucléotide est $\beta$, tandis qu'il est encore intact après 30 mn quand la configuration anomérique est $\alpha$.

L'analyse HPLC a été entreprise sur une colonne C18 $\mu$-Bondapak. L'élution isocratique a été exécutée avec le système suivant : acétonitrile 31 % dans de l'acétate d'ammonium 20 mM (pH 6,5 ; 5 mM par rapport au tétrabutylammonium chlorure). La détection UV a été effectuée à 270 nm.

Conditions d'analyse: une solution d'$\alpha$- ou $\beta$-$T_4$-$C_5$-OPC (33 nmol.) dans de l'eau (8 $\mu$l) a été mélangée avec le tampon suivant : acétate de sodium 0,5 M (pH 4,7), chlorure de sodium 3 M et acétate de zinc 0,1 M. Le volume du mélange réactionnel a été complété jusqu'à 300 ml avec de l'eau et ensuite la nucléase S1 (Gibco BRL, 28 unités, 3 $\mu$l) a été ajoutée. Le mélange a été incubé à 37° C et des aliquots (20 $\mu$l) ont été retirés à différents moments, mais immédiatement mélangés avec un tampon bloquant : phosphate de sodium 20 mM (pH 6,5, 180 $\mu$l) et chauffés à 100° C pendant 5 minutes. Des traitements similaires ont été exécutés pour chaque expérience de contrôle. Pour chaque analyse HPLC, un volume de 50 $\mu$l a été injecté.

Dans les exemples X à XXII ci-après, les abréviations suivantes sont utilisées :

Dans la description et les exemples, les abréviations suivantes sont utilisées :

| | |
|---|---|
| $\alpha$-dA : | $\alpha$-D-2'-désoxyadénosine |
| $\alpha$-dbzA : | N-benzoyl-$\alpha$-D-2'-désoxyadénosine |

| | |
|---|---|
| α-dC : | α-D-2'-désoxycytidine |
| α-dbzC : | N-benzoyl-α-D-2'-désoxycytidine |
| α-dG : | α-D-2'-désoxyguanosine |
| α-pal : | N-palmitoyl-α-D-2'-désoxyguanosine |
| α-T : | α-D-thymidine |
| α-Tbz : | α-D-thymidine 3'-benzoyle |
| α-[(sp)T] : | α-D-thymidine 5'-thiophosphate |
| α-T(ps) : | α-D-thymidine 3'-thiophosphate |
| α-T(ps)R : | |

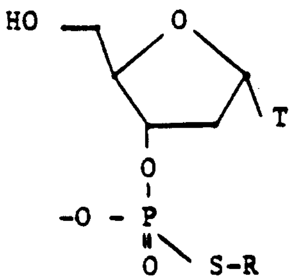

α-T(p(s))(Cnet)$_2$ :

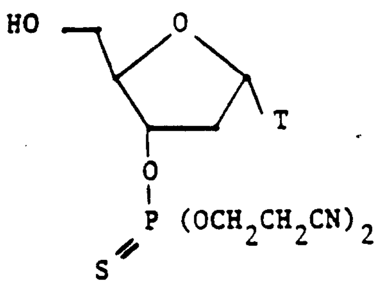

| | |
|---|---|
| ABH : | acide benzohydroxamique |
| Cnet : | β-cyanoéthyle |
| DBU : | diaza-1,8 bicyclo[5,4,0] undécène-7 |
| Et : | éthyle |
| Me : | méthyle |
| MST : | mésitylènesulfonyltétrazolide |
| p : | phosphoester |
| β : | p-chlorophénylphosphoester |
| Acr : | |

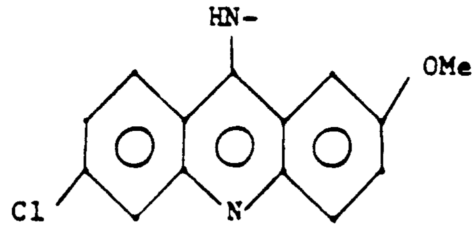

EDTA : R=H

EDTAMe$_3$ : R=$CH_3$

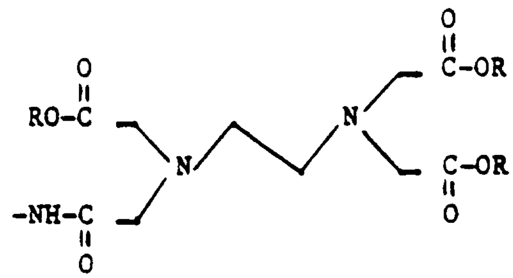

Phe :

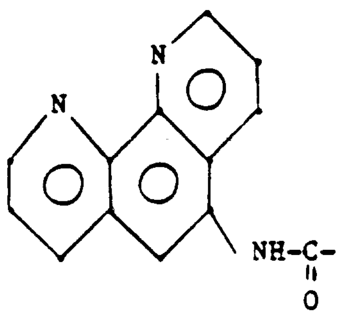

Prof :

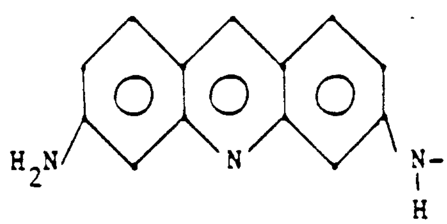

CCM : chromatographie sur couche mince sur gel de silice
Système A : $CH_2Cl_2$, MeOH (90-10, v/v)
Système B : $CH_2Cl_2$, MeOH (85-15, v/v)

EXEMPLE X :α-(TpTpTpTpTpTpTpTp)$(CH_2)_5$Acr

1) 5'-O-(diméthoxytrityl)α-thymidine

A une solution de α-thymidine (3,6 g) dans la pyridine anhydre (15 $cm^3$), on ajoute sous agitation 5,35 g de chlorure de diméthoxytrityle. Après 4 heures de réaction à la température ambiante on détruit l'excès de chlorure de diméthoxytrityle par addition de méthanol (3 $cm^3$). On évapore le solvant sous pression réduite ; le résidu est repris avec du chloroforme. La phase organique est lavée avec de l'eau puis séchée et

concentrée. Le produit est purifié par chromatographie sur gel de silice. Rendement ≃ 80 %.

2) 5'-O-(diméthoxytrityle)α-thymidine 3'-(p-chlorophénylphosphate) de pyridinium

On ajoute sous agitation, à -20 ° C, 3 équivalents de méthyl p-chlorophényl chlorophosphate à une solution de 9 équivalents de diméthyl-1,5-tétrazole dans la pyridine anhydre ; l'addition terminée, on continue l'agitation pendant une heure à la température ambiante, puis ajoute une solution de 1 équivalent de-5'-O-(diméthoxytrityle)α-thymidine dans la pyridine. Après quelques heures de réaction à la température ambiante, on ajoute alors de l'eau glacée et extrait le diester avec du chloroforme. La solution chloroformique est séchée et concentrée sous pression réduite. Le diester obtenu sous forme de solution dans la pyridine est pratiquement pur pour être utilisé dans les étapes suivantes. CCM (système B) : Rf = 0,18.

3) α-thymidine 3'-(p-chlorophényl β-cyanoéthylphosphate)

A une solution de (Dmtr)α-T(1 mmole) et de p-chlorophényl β-cyanoéthylphosphate de triméthylammonium (2 mmoles) dans la pyridine anhydre (10 $cm^3$), on ajoute à la température ambiante et sous agitation du chlorure de mésitylène sulfonyle (3 mmoles) et du tétrazole (6 mmoles). L'évolution de la réaction est suivie par CCM (système A). Lorsque la réaction est terminée, l'excès de chlorure de mésitylène sulfonyle est détruit par addition d'eau glacée. Le triester est extrait avec du chloroforme. La phase organique est lavée avec une solution saturée de bicarbonate de sodium puis avec de l'eau, séchée et concentrée. Le résidu obtenu est traité ensuite, pendant 15 minutes, à 0 ° C, avec 15 $cm^3$ d'acide benzène sulfonique [solution à 2 % dans $CHCl_3$-MeOH (7-7, v/v)] puis la solution est neutralisée avec une solution de bicarbonate de sodium. Le produit est extrait avec du chloroforme, la phase organique est lavée avec de l'eau glacée, séchée et évaporée. Le résidu obtenu est purifié sur une colonne de gel de silice (éluant : $CH_2Cl_2$-MeOH). CCM (système A) Rf≃0,27 ; rendement : 70 %.

4) (Dmtr)α-(Tp̂Tp̂)Cnet

On fait réagir sous agitation pendant 2 heures à la température ambiante une solution de 0,6 mmole de (Dmtr)α-Tp̂ (exemple I-2), de 0,5 mmole de α-Tp̂ Cnet (exemple X-3) et de 1,5 mmole de mésitylènesulfonyltétrazolide (MST) dans 4,5 $cm^3$ de pyridine anhydre ; on ajoute ensuite 0,5 $cm^3$ d'eau glacée et continue l'agitation pendant 30 minutes. On concentre la solution sous pression réduite. Le résidu est repris avec du chloroforme ; la phase organique est lavée avec une solution aqueuse à 5 % de $NaHCO_3$ puis séchée et évaporée sous pression réduite. Le produit est purifié sur une colonne de gel de silice (éluant : $CH_2Cl_2$-MeOH). CCM (système A) : Rf = 0,50 et 0,52 ; rendement ≃ 72 %.

5) (Dmtr)α-(Tp̂Tp̂)

On fait réagir pendant 2 heures à la température ambiante une solution de (Dmtr)α-(Tp̂Tp̂)Cnet (0,3 mmole) (exemple X-4) et de 1 $cm^3$ de triéthylamine dans la pyridine (2 $cm^3$). On chasse les produits volatils sous pression réduite puis précipite le diester par agitation dans l'éther. CCM (système B) : Rf = 0,19.

6) (Dmtr)α-$(T\hat{p})_4$Cnet

Le composé (Dmtr)α-$(T\hat{p})_2$Cnet (0,2 mmole) est traité avec l'acide benzène sulfonique à 0 ° C pendant 15 minutes. Le mélange est repris avec 40 $cm^3$ de chloroforme. La phase organique est lavée avec une solution aqueuse de $NaHCO_3$ puis séchée sur $Na_2SO_4$ et concentrée sous pression réduite. Au résidu obtenu on ajoute 0,25 mmole de (Dmtr)α-(Tp̂Tp̂). Après avoir séché le mélange par co-évaporation avec le pyridine anhydre, on ajoute 3 $cm^3$ de pyridine anhydre puis 0,6 mmole de MST et laisse le mélange réactionnel à la température ambiante pendant 2 heures. On détruit l'excès de réactif de couplage par addition d'eau glacée puis termine la préparation comme dans l'exemple X-4.

7) (Dmtr)α-$(T\hat{p})_8$Cnet

En remplaçant le dinucléotide (Dmtr)α-$(T\hat{p})_2$Cnet par le tétranucléotide (Dmtr)α-$(T\hat{p})_4$Cnet et en opérant comme dans X-5 et X-6 on obtient l'octanucléotide totalement protégé.

8) (Dmtr)α-(Tp̂)$_8$(CH$_2$)$_5$Acr

(Dmtr)α-(Tp̂)$_8$Cnet (0,01 mmole) est traité pendant 2 heures à la température ambiante avec 0,3 $cm^3$ d'une solution de pyridine-triéthylamine (2-1, v/v). On chaise le solvant sous pression réduite et lave le diester obtenu sous forme de solide avec de l'éther puis le sèche sous pression réduire. Le diester est ensuite couplé avec 0,02 mmole de méthoxy-2 chloro-6 (ω-hydroxypentylamino)-9 acridine en présence de MST (0,04 mmole) dans la pyridine anhydre ; on termine ensuite la préparation comme dans l'exemple X-4.

9) α-(Tp)$_8$(CH$_2$)$_5$Acr

L'octanucléotide (Dmtr)α-(Tp̂)$_8$(CH$_2$)$_5$Acr est traité pendant 24 heures à la température ambiante avec une solution molaire en acide benzohydroxamique (ABH) et en diaza-1,8 bicyclo (5,4,0) endécène-7 (DBU) dans la pyridine anhydre (en utilisant 10 équivalents de ABH-DBU par équivalent de phosphotriester arylé à déprotéger). Le milieu réactionnel est neutralisé avec du DOWEX 50 (forme pyridinium) et évaporé sous pression réduite. Le résidu est ensuite traité avec l'acide acétique à 80 % pendant quelques heures à la température ambiante puis l'acide acétique est éliminé par co-évaporation plusieurs fois avec de l'éthanol. On reprend le résidu avec de l'eau, lave la phase aqueuse avec de l'éther puis purifie le produit par HPLC. Le temps de rétention du produit purifié est donné dans le tableau V.

EXEMPLE XI : [Acr(CH$_2$)$_5$]α-(pT)$_8$

1) α-thymidine 3'-benzoyle,α-Tbz

A une solution de (Dmtr)α-T (1 mmole) dans la pyridine anhydre, on ajoute sous agitation à la température ambiante du chlorure de benzoyle (1,5 mmole) puis laisse le mélange réactionnel pendant 48 heures à la température ambiante. On détruit l'excès de chlorure de benzoyle par addition d'eau glacée puis termine la préparation comme dans l'exemple X-3. CCM (système A) : Rf≈0,36 ; rendement ≈ 75 %.

2) (Dmtr)α-(Tp̂T)bz

On opère comme dans l'exemple X-4 en remplaçant l'α-Tp̂-Cnet par le composé α-Tbz. On obtient le dinucléoside monophosphate protégé.

3) (Dmtr)α-[(Tp̂)$_3$T]bz

En remplaçant le dinucléotide (Dmtr)α-(Tp̂)$_2$Cnet par le composé (Dmtr)α-(Tp̂T)bz et en opérant selon l'exemple X-6, on obtient le tétranucléosidetriphosphate protégé.

4) (Dmtr)α-[(Tp̂)$_7$T]bz

Le composé (Dmtr)α-[(Tp̂)$_3$T]bz (1 équivalent) est détritylé par l'acide benzène sulfonique ; le mélange est repris avec du chloroforme et lavé avec une solution aqueuse de $NaHCO_3$ puis séché et concentré. A ce résidu on ajoute 1,2 équivalents de (Dmtr) α-(Tp̂)$_4$ (préparé par décyanoéthylation du composé (Dmtr)α-Tp̂)$_4$Cnet selon l-exemple X-5). Après avoir séché le mélange par co-évaporation avec la pyridine, on ajoute à la solution de pyridine 3 équivalents de MST et laisse le mélange réactionnel sous agitation pendant 2 heures à la température ambiante. On termine la préparation comme dans l'exemple X-6.

5) α-[(Tp̂)$_7$T]bz

Le composé de l'exemple XI -4 est traité pendant 15 minutes à 0 °C avec l'acide benzène sulfonique [2 % en solution dans le chloroforme-méthanol (7-3, v/v)]. Le mélange est repris avec du chloroforme. La solution chloroformique est lavée avec une solution aqueuse de $NaHCO_3$ puis séchée et évaporée sous pression réduite. Le résidu obtenu est utilisé dans l'étape suivante.

6) α-[p̂(Tp̂)$_7$T]bz

On ajoute sous agitation et à -20 °C, 6 équivalents de méthyl p-chlorophényl chlorophosphate dans la pyridine anhydre. L'addition terminée, on continue l'agitation pendant 1 heure à la température ambiante

puis ajoute une solution de 1 équivalent de α-[(Tp̂)$_7$T]bz. Après 2 heures de réaction à la température ambiante, on ajoute de l'eau glacée puis extrait le produit avec du chloroforme.

7) [Acr$(CH_2)_5$]α-(p̂(Tp̂)$_7$T]bz

On fait réagir pendant 2 heures à la température ambiante une solution de méthoxy-2 chloro-6 (ω-hydroxypentylamino)-9 acridine (2 équivalents), de α-[p̂(Tp̂)$_7$T]bz (1 équivalent) et de MST (3 équivalents) puis termine la préparation comme dans l'exemple X-4.

8) [Acr$(CH_2)_5$]α-(pT)$_8$

L'oligonucléotide protégé (exemple XI-7) est traité pendant 48 heures sous agitation à la température ambiante avec une solution molaire en ABH-DBU dans la pyridine anhydre (10 équivalents de ABH-DBU par équivalent de phosphoester arylé à déprotéger). A ce mélange, on ajoute 2 volumes d'une solution de soude 0,4 M puis continue l'agitation pendant 1 heure à la température ambiante. Après avoir neutralisé le milieu réactionnel avec l'acide chlorhydrique, on lave la phase aqueuse avec de l'éther puis purifie l'oligonucléotide déprotégé par HPLC (le temps de rétention est donné dans le tableau V).

<u>EXEMPLE XII</u> :

$$[Acr(CH_2)_3\overset{\overset{Et}{|}}{N}(CH_2)_2]\alpha\text{-}[(sp)(Tp)_7T]$$

1) Bis-(β-cyanoéthyl)-diisopropylamino-phosphite

A une solution de β-cyanoéthanol (15 $cm^3$) et de triméthylamine (20 g) dans le mélange d'éther (100 $cm^3$) et de benzène (50 $cm^3$), on ajoute goutte à goutte à -5 °C et sous agitation une solution de 20 g de dichloro diisopropylamino-phosphite dans 40 $cm^3$ de benzène. L'addition terminée on continue l'agitation pendant 1 heure à la température ambiante puis élimine le chlorohydrate de triméthylammonium par filtration. Ou chasse le solvant sous pression réduite et purifie le produit par évaporation sur couche mince (distillation moléculaire).

2) α-[(Cnet)$_2$((s)p)(Tp̂)$_7$T]bz

A une solution anhydre de α-[(Tp̂)$_7$T]bz (1 équivalent) et de tétrazole (10 équivalents) dans le mélange de solvant $CH_3CN$-DMF (50-50 , v/v) on ajoute sous atmosphère d'argon et sous agitation use solution de bis-(β-cyanoéthyl)-diisopropylamino-phosphite (5 équivalents) dans l'acétonitrile. Après 30 minutes à la température ambiante, on ajoute au mélange réactionnel une suspension de soufre (50 équivalents) dans la pyridine puis continue l'agitation pendant 90 minutes à 30 °C. On élimine l'excès de soufre par filtration, chasse les solvants sous pression réduite puis reprend le résidu avec du toluène ; la phase organique est lavée avec une solution aqueuse de $NaHCO_3$ puis séchée et concentrée. Le produit est purifié par chromatographie sur gel de silice.

3) α-[(sp)(Tp)$_7$T]

L'octanucléotide préparé selon l'exemple XII-2 est traité pendant 48 heures à la températuré ambiante avec une solution molaire en ABH et DBU (10 équivalents de ABH-DBU par équivalent de phosphotriester) puis avec 2 volumes d'une solution aqueuse de soude 0,6 M pendant 24 heures. On neutralise le milieu réactionnel avec l'acide chlorhydrique, lave la phase aqueuse avec l'éther et purifie le produit par HPLC. Le temps de rétention du produit est donné dans le tableau V.

4) $[Acr(CH_2)_3\overset{\overset{Et}{|}}{N}(CH_2)_2]\alpha\text{-}[(sp)(Tp)_7T]$

On fait réagir pendant 5 heures à la température ambiante uns solution de α-[(sp)(Tp)$_7$T] (1 équivalent) et de méthoxy-2 chloro-6[N-(β-chloroéthyl, éthylamino)-3 propylamino]-9 acridine (4 équivalents) dans un mélange DMSO-$H_2O$-$NaHCO_3$ à 5 % dans l'eau (2-2-1, v/v) puis purifie le produit formé par HPLC. Le tableau III donne le temps de rétention du produit préparé.

EXEMPLE XIII: [Prof($CH_2$)$_3$]α-[(sp)(Tp)$_7$T]

Une solution de α-[(sp)(Tp)$_7$T] (1 équivalant) et de N-(ω-bromopropyl)proflavine (5 équivalents) dans le mélange de DMSO-$H_2O$-$NaHCO_3$ à 5 % dans l'eau (2-2-1, v/v) est laissée sous agitation pendant 20 heures à la température ambiante ; le produit obtenu est purifié par HPLC. (Le temps de rétention du produit obtenu est donné dans le tableau V.)

EXEMPLE XIV: (Phe-$CH_2$)α-[(sp)(Tp)$_7$T]

On opère comme dans l'exemple XII-4 en remplaçant le dérivé de l'acridine par la (N-iodoacétylamino)-5 phénanthroline-1,10. Le temps de rétention du produit obtenu est donné dans le tableau V.

EXEMPLE XV : [Acr($CH_2$)$_5$]α-(pT)$_8$(ps)

1) α-thymidine 3'-(bis-cyanoéthylthiophosphate)

En partant de (Dmtr)α-T (1 équivalent), de tétrazole (4 équivalents), de bis-(β-cyanoéthyl)-diisopropylamino-phosphite (2 équivalents) et du soufre (30 équivalents) et en opérant selon l'exemple XII-2 on obtient le composé (Dmtr)α-T(p(s))(Cnet)$_2$ qui est ensuite détritylé par l'acide benzène sulfonique selon l'exemple XI-5. Le rendement est de 55 %.

2) (Dmtr)α-[Tp̂T](p(s))(Cnet)$_2$

On opère comme dans l'exemple X-4 en remplaçant le composé α-Tp̂Cnet par le composé α-T(p(s))-(Cnet)$_2$. On obtient ainsi le dinucléotide qui est purifié par chromatographie sur silice [éluant : $CH_2Cl_2$-$H_2O$-Acétone (31-1-68, v/v)]. Le rendement est voisin de 84 %.

3) (Dmtr)α-(Tp̂)$_6$Cnet

On opère comme dans l'exemple X -6 en remplaçant le dinucléotide (Dmtr)α-(Tp̂)$_2$Cnet par le tétranucléotide (Dmtr)-α-(Tp̂)$_4$ Cnet. On obtient ainsi l'héxanucléotide avec un rendement de 75 %. CCM (système A) : Rf = 0,31.

4) [Acr($CH_2$)$_5$]α-(p̂T)$_6$p̂

En partant du composé (Dmtr)α-(Tp̂)$_6$Cnet et en opérant selon les exemples XI -5, XI-6 et XI-7, on obtient le composé [Acr ($CH_2$)$_5$]α-(p̂T)$_6$p̂Cnet qui est ensuite traité avec la triéthylamine selon l'exemple X-5. CCM (système B) : RF = 0,44.

5) [Acr($CH_2$)$_5$]α-(p̂T)$_8$(p(s))(Cnet)$_2$

L'octanucléotide est préparé par couplage de l'héxanucléotide [Acr($CH_2$)$_5$]α-(p̂T)$_6$p̂ (1 équivalent) avec le dinucléotide α-(Tp̂T)(p(s))(Cnet)$_2$ (1 équivalent) (préparé par détritylation du composé de l'exemple XV -2) en présence de MST (3 équivalents) selon l'exemple XI-4. Le produit est purifié sur gel de silice [éluant : $CH_2Cl_2$-MeOH (de 96-4 à 96-14, v/v)].

6) [Acr($CH_2$)$_5$α-(pT)$_8$(ps)

En partant du composé préparé selon l'exemple XV-5 et en opérant selon l'exemple XII-3, on obtient l'octanucléotide déprotégé dont le temps de rétention est donné dans le tableau V.

EXEMPLE XVI :

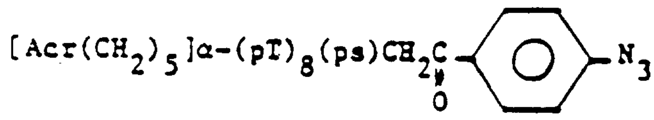

Une solution de $[Acr(CH_2)_5]\alpha$-$(pT)_8(ps)$ (1 équivalent) et de bromure de 4-azidophénacyle (4 équivalents) dans le mélange de DMSO-$H_2O$-$NaHCO_3$ à 5 % dans l'eau (2-2-1, v/v) est laissée sous agitation pendant une heure à la température ambiante. Le produit de couplage est ensuite purifié par HPLC. Le temps de rétention du produit obtenu est donné dans le tableau V.

EXEMPLE XVII : $[Acr(CH_2)_5]\alpha$-$(pT)_8(ps)CH_2CO_2^-$

On fait réagir pendant 3 heures à la température ambiante l'acide bromoacétique (6 équivalents) avec l'oligonucléotide $[Acr\ (CH_2)_5]\alpha$-$pT)_8(ps)$ (1 équivalent) selon l'exemple XVI puis purifie le produit formé par HPLC. Le temps de rétention du produit obtenu est donné dans le tableau V.

EXEMPLE XVIII:$\alpha$-$(T\hat{p})_2(CH_2)_6EDTAMe_3$

On fait réagir pendant une heure et à la température ambiante une solution de (Dmtr)$\alpha$-$(T\hat{p})_2$ (1 équivalent), de $Me_3EDTA$ ($-CH_2)_6$-OH (3 équivalents) et de MST (3 équivalents). On détruit l'excès de réactif de couplage par addition d'eau glacée puis termine la préparation comme dans l'exemple X -3. CCM (système A) : Rf≃0,4.

EXEMPLE XIX: $[Acr(CH_2)_5]\alpha$-d(pCpCpTpTpApTpApTpT)

1) (Dmtr)$\alpha$-d(bzA$\hat{p}$)

a) Un mélange de 3',5'-di-(p-nitrobenzoyl)$\beta$-D-thymidine (3,6 mmoles) et de N-benzoyladénine (12 mmoles) dans l'acétonitrile (22 $cm^3$) est traité avec le bis-triméthylsilylacétamide (15 mmoles) puis avec du triméthylsilyltrifluorométhanesulfonate (4,7 mmoles) selon T. Yamaguchi et M. Saneyoshi (Chem. Pharm. Bull. 1984, 32, 1441-1450). Le composé 3',5'-di-(p-nitrobenzoyl)$\alpha$-D-d-(N-benzoyladénosine) [CCM (système A) : $\overline{Rf}=0,55$] est séparé du dérivé 3',5'-di-(p-nitrobenzoyl)$\beta$-D-d-(N-benzoyladénosine) [CCM (système A) : Rf = 0,63] par chromatographie sur plaque préparative de gel de silice.

b) La 3',5'-di-(p-nitrobenzoyl)$\alpha$-D-d-(N-benzoyladénosine) (1 g) dans le mélange de solvant [dioxanne-MeOH-$CH_2Cl_2$-$H_2O$] (50 $cm^3$) est traité à 0 °C avec 5 $cm^3$ de soude molaire. Lorsque tout le produit de départ est transformé en $\alpha$-d-N-benzoyladénosine [CCM ($CH_2Cl_2$-MeOH)(80-20, v/v) : Rf = 0,38], le milieu réactionnel est neutralisé par addition de résine Amberlite IR 120 (forme pyridinium), puis la $\alpha$-d-bzA est purifiée par chromatographie sur gel de silice.

c) Une solution de $\alpha$-d-bzA (1 équivalent) dans la pyridine anhydre est traitée pendant 4 heures à la température ambiante avec le chlorure de diméthoxytrityle (1,1 équivalents) puis on termine la préparation comme l'exemple X-1 et X-2. CCM (système B) : Rf = 0,32.

2) (Dmtr)$\alpha$-d(bzA$\hat{p}$T$\hat{p}$)Cnet

On opère dans l'exemple X-4 en remplaçant la (Dmtr)$\alpha$-T$\hat{p}$ par la (Dmtr)$\alpha$-d(bzA$\hat{p}$), on obtient le dinucléotide totalement protégé.

3) (Dmtr)$\alpha$-d(bzA$\hat{p}$T$\hat{p}$)

Le diester est obtenu par décyanoéthylation du composé XIX-2 selon l'exemple X-5.

4) (Dmtr)α-d(bzAp̂Tp̂bzAp̂Tp̂)Cnet

On opère comme dans l'exemple X-6 en remplaçant les composés (Dmtr)α(Tp̂Tp̂)Cnet et (Dmtr)α-(Tp̂Tp̂) respectivement par les composés des exemples XIX-2 et XIX-3. On obtient le tétranucléotide protégé.

5) (Dmtr)α-d(bzAp̂Tp̂bzAp̂Tp̂T)bz

Le diester (Dmtr)α-d(bzAp̂Tp̂bzAp̂Tp̂) (1 équivalent) (préparé par décyanoéthylation du composé XIX-4 selon l'exemple X-5) est couplé avec l'α-thymidine 3'-benzoyle (1 équivalent) selon l'exemple X-4 pour donner le pentanucléosidetétraphosphate protégé.

6) (Dmtr)α-d(bzCp̂)

En partant de la 3',5'-diacétyl β-D-d(N-benzoylcytidine) (Chem. Pharm. Bull. 1984, 32, 1441-1450) et en opérant comme dans XIX-1-b et XIX-1-c , on obtient le diester. CCM (système B) ; Rf = 0,32.

7) (Dmtr)α-d(bzCp̂bzCp̂Tp̂Tp̂)Cnet

Le dinucléotide α −(Tp̂)$_2$Cnet (1 équivalent) (préparé bar détritylation de (Dmtr)α-(Tp̂)$_2$Cnet selon l'exemple XI-5) est couplé avec le mononucléotide (Dmtr)α-dbzCp̂ (1 équivalent) en présence de MST (3 équivalents) selon l'exemple X-4 pour donner le trinucléotide protégé (Dmtr)α-d(bzCp̂Tp̂Tp̂)Cnet qui à son tour est détritylé puis couplé avec le diester (Dmtr)α-dbzCp̂ comme précédemment.

8) (Dmtr)α-d(bzCp̂bzCp̂Tp̂Tp̂bzAp̂Tp̂bzAp̂Tp̂T)bz

Le tétranucléotide 3'-phosphodiester (Dmtr)α-d(bzCp̂bzCp̂Tp̂Tp̂) (1,2 équivalents) (préparé par décyanoéthylation du composé XIX-7 selon l'exemple XI-5) est couplé avec le pentanucléotide α-d-(bzAp̂Tp̂bzAp̂Tp̂T)bz (1 équivalent) (obtenu par détritylation du composé XIX-5selon l'exemple XI-5 en présence de MST (3 équivalents) selon l'exemple X-4 pour donner le nonanucléotide protégé. CCM (système A) : Rf = 0,55.

9) [Acr($CH_2$)$_5$]α-d(pCpCpTpTpApTpApTpT)

En partant du composé XIX-8 et en opérant comme dans les exemples XI-5, XI-6, XI-7 et XII-3 , on obtient le produit déprotégé dont le temps de rétention est donné dans le tableau V.

TABLEAU V

| COMPOSES | | SYSTEME DE SOLVANT (A-B, gradient linéaire) | TEMPS DE RETENTION |
|---|---|---|---|
| X | α-$(Tp)_8(CH_2)_5$Acr | de 0 à 50 % en B en 25 mn | 9 minutes |
| XI | [Acr$(CH_2)_5$]α-$(pT)_8$ | de 0 à 50 % en B en 25 mn | 9 minutes |
| XII | [Acr$(CH_2)_3$N(Et)$(CH_2)_2$]α-[(sp)$(Tp)_7$T] | de 0 à 60 % en B en 15 mn | 5 mn 48 sec |
| XIII | [Prof$(CH_2)_3$]α-[(sp)$(Tp)_7$T] | de 0 à 60 % en B en 15 mn | 6 mn 54 sec |
| XIV | [Phe$CH_2$]α-[(sp)$(Tp)_7$T] | de 0 à 60 % en B en 15 mn | 8 mn 6 sec |
| XV | [Acr$(CH_2)_5$]α-$(pT)_8$(ps) | de 0 à 60 % en B en 15 mn | 8 mn 48 sec |
| XVI | [Acr$(CH_2)_5$]α-$(pT)_8$(ps)$CH_2$ 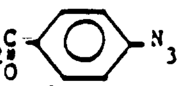 | de 0 à 60 % en B en 15 mn | 7 mn 48 sec |
| XVII | [Acr$(CH_2)_5$]α-$(pT)_8$(ps)$CH_2CO_2^-$ | de 0 à 60 % en B en 15 mn | 9 mn 12 sec |
| XVIII | [Acr$(CH_2)_5$]α-d(pCpCpTpTpApTpApTpT) | de 0 à 60 % en B en 15 mn | 8 mn 54 sec |
| | α-$(Tp)_7$T | de 0 à 50 % en B en 25 mn | 9 mn |
| | α-[(sp)$(Tp)_7$T] | de 0 à 60 % en B en 15 mn | 10 mn 54sec |

HPLC - Colonne polyanion HR5/5 (Pharmacia), débit 1 $cm^3$/mn

A : $10^{-3}$M phosphate de potassium (pH = 6)

B : M phosphate de potassium (pH = 6)

EXEMPLE XX : Stabilité des α-D-oligonucléotides couplés au dérivé de l'acridine vis-à-vis des nucléases.

Le tableau VI ci-après donne les vitesses d'hydrolyse par quatre nucléases différentes de deux α-D-oligonucléotides substitués par l'acridine en 3' ou en 5' comparées à celles des β-D-oligonucléotides correspondants. Aussi bien les deux endonucléases (P1 et S1) que les deux exonucléases (5' exo : phosphodiestérase de rate de veau ; 3' exo : phosphodiestérase de venin de serpent) sont 300 à 500 moins actives sur les α-D-oligonucléotides que sur les β-D- oligonucléotides. Les valeurs données dans le tableau sont les rapports des vitesses d'hydrolyse pour l'α-D-oligonucléotide comparé au β-D-oligonucléotide de même séquence.

TABLEAU VI

| | Nucléases | | | |
|---|---|---|---|---|
| | P1 | S1 | Exo 3' | Exo 5' |
| $(Tp)_8m_5Acr$ | $3.5\times 10^{-3}$ | $2\times 10^{-3}$ | - | $3.5\times 10^{-3}$ |
| $Acr\ m_5(pT)_8$ | $3\times 10^{-3}$ | $2.5\times 10^{-3}$ | $2\times 10^{-3}$ | |

EXEMPLE XXI

Coupure spécifique d'un ADN par un α-D-oligonucléotide couplé à la phénanthroline.

L'α-D-oligonucléotide décrit à l'exemple XIV (5μM) est dissous dans un tampon à pH 7 contenant 10 mM cacodylate de sodium et 0,1 M NaCl. Il est ajouté à un fragment d'ADN simple brin (10 nM) de séquence d($^{5'}$T G A G T G A G T A A A A A A A A T G A G T G C C A A$^{3'}$) marqué en position 5' par un phosphore radioactif. Du sulfate cuivrique (1μM) et de l'acide β-mercaptopropionique (1 mM) sont ajoutés à 0°C et le mélange est incubé à 0°C pendant 3 heures. De la bathocuproine est ajoutée pour arrêter la réaction. Le mélange est disposé sur un gel de polyacrylamide à 18 % et soumis à l'électrophorèse. Après autoradiographie les sites de coupures sont révélés par l'apparition de bandes dont la localisation indique que l'α-D-oligonucléotide est orienté parallèlement à la séquence $A_8$.

Le même oligonucléotide incubé en présence d'un fragment d'ADN double brin

5'T G A G T G A G T A A A A A A A A T G A G T G C C A A 3'
3'A C T C A C T C A T T T T T T T T A C T C A C G G T T 5'

induit des coupures sur les deux brins de la double hélice en présence de 1 mM spermine. La position des sites de coupure sur le double brin indique que l'α-D-oligothymidylate XIV est orienté parallèlement au brin contenant la séquence $A_8$.

EXEMPLE XXII : Pontage covalent et coupure par un dérivé d'α-D-oligonucléotide photoactivable.

La molécule décrite à l'exemple XVI est incubée à 0°C en présence du fragment d'ADN simple brin et du fragment d'ADN double brin marqués en position 5' radioactivement dans les conditions décrites à l'exemple XXI.

Après irradiation du mélange à 0°C à des longueurs d'ondes supérieures à 300 nm puis traitement du mélange irradié par la pipéridine 1 M à 90°C pendant 20 minutes les échantillons sont déposés sur un gel de polyacrylamide 18 % et soumis à l'électrophorèse. Après autoradiographie, des bandes sont révélées qui correspondent à des coupures de l'ADN cible en des positions bien précises indiquant que l'α-D-oligothymidylateXVI est fixé de façon parallèle à la séquence $A_8$ aussi bien sur l'ADN simple brin que l'ADN double brin. Dans ce dernier cas des coupures sont observées sur les deux brins.

Si l'électrophorèse est réalisée avant traitement des échantillons par la pipéridine, aucune coupure n'est révélée. Des molécules migrant moins vite que le fragment d'ADN de départ sons observées. Elles correspondent aux produits du pontage covalent de l'α-D-oligothymidylate XVI sur les bases d' l'ADN utilisé comme cible.

## Revendications

1. Composés chimiques constitués par un oligonucléotide ou un oligodéoxynucléotide, caractérisés en ce qu'ils comportent un enchaînement de nucléotides ou déoxynucléotides à configuration anomérique

non naturelle α, enchaînement lié de façon covalente à un agent effecteur, notamment un radical correspondant à un agent d'intercalation ou un radical chimique ou photoactivable, tel que un radical porteur d'une fonction réagissant directement ou indirectement avec les chaînes de nucléotides ou un radical dont la présence permet une détection facile et sensible.

2. Composés chimiques constitués par un oligonucléotide ou un oligodéoxynucléotide, caractérisés en ce qu'ils comportent un enchaînement de nucléotides ou déoxynucléotides à configuration anomérique non naturelle α non lié à un radical effectuer, lesdits nucléotides ou déoxynycléotides ou déoxynucléotides ne comportant pas uniquement les bases thymine et cytosine.

3. Composés selon la revendication 1 ou 2, caractérisés en ce qu'ils ont pour formule :

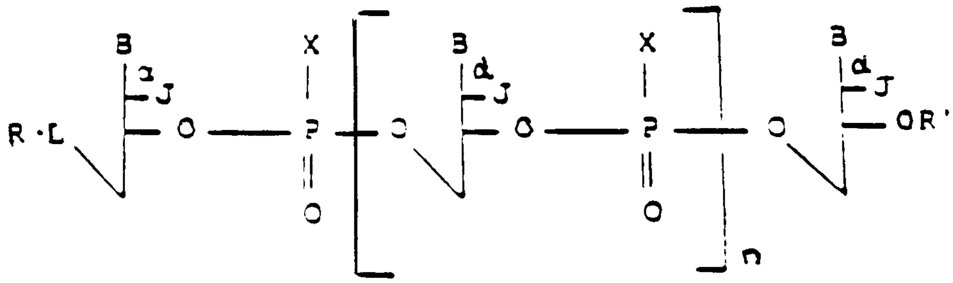

(I)

dans laquelle :

les radicaux B peuvent être identiques ou différents et représentent chacun une base d'un acide nucléique éventuellement modifiée, activable et/ou comportant un groupement intercalant et rattachée au cycle glycosidique selon une configuration anomérique α non naturelle ;

les radicaux X peuvent être identiques ou différents et représentent chacun un oxoanion $O^{\ominus}$, un thioanion $S^{\ominus}$, un groupe alkyle, un groupe alcoxy, aryloxy, un groupe aminoalkyle, un groupe aminoalcoxy, un groupe thioalkyle, un radical alkyle ou alcoxy substitué par un hétérocycle azoté ou un groupement -Y-Z ;

R et R', qui peuvent être identiques ou différents, représentent chacun un atome d'hydrogène ou un groupement -Y-Z ou Y'-Z' ;

Y et Y' identiques ou différents représentent chacun un radical alcoylène droit ou ramifié -alk- ou un radical choisi parmi

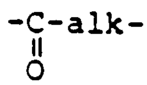 , 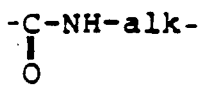 , 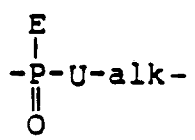 , 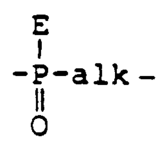 , alk-O-alk

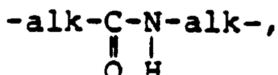 , 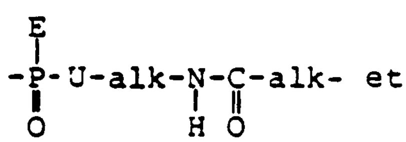 et 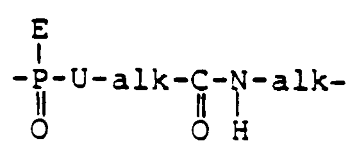 ,

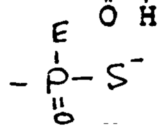 , 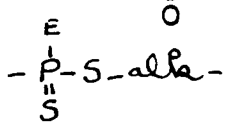

avec U = O, S ou N

E peut avoir les mêmes significations que X excepté Y-Z,

J représente un atome d'hydrogène ou un groupe hydroxy ;

Z et Z', identiques ou différents, représentent chacun un radical correspondant à un agent effecteur, notamment un radical correspondant à un agent d'intercalation ou un radical porteur d'une fonction qui réagit directement ou indirectement avec les chaînes de nucléotides ou un radical dont la présence permet une détection facile et sensible.

n est un nombre entier y compris O ;
L représente un atome d'oxygène, un atome de soufre ou un groupe -NH-.

**4.** Composés selon la revendication 3, caractérisés en ce que Y et Y' représentent un radical choisi parmi

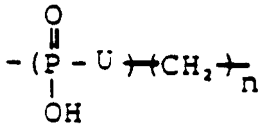 , 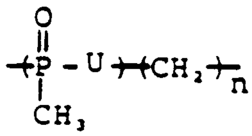 , 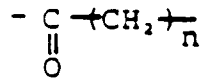 , 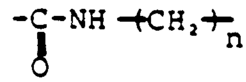

avec U = O, S ou N

**5.** Composés selon la revendication 3, caractérisés en ce que dans la formule générale I

- lorsque L représente un atome de soufre ou un groupement -NH-, les radicaux R et R', identiques ou différents, représentent chacun un atome d'hydrogène ou un groupement -Y-Z ou Y'-Z' dans lequel :
  . Y et Y', identiques ou différents, représentent chacun un radical alcoylène droit ou ramifié (-alk-) ou un radical

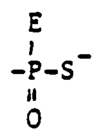

ou un radical

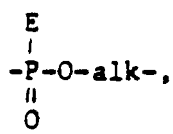 , 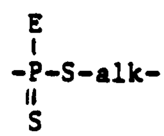

ou

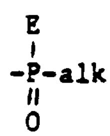

ou un radical -alk-O-alk- ou un radical -alk-CONH-alk- ou un radical

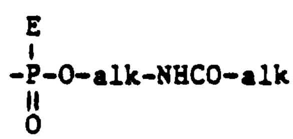

ou un radical

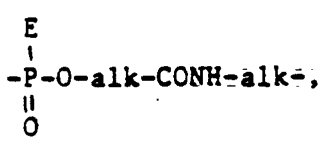

et

. Z et Z', identiques ou différents, représentent chacun un radical correspondant à un agent d'intercalation ou un radical porteur d'une fonction qui réagit directement ou indirectement avec les chaînes de nucléotides ou un radical donc la présence permet une détection facile et sensible,

- lorsque L représente un atome d'oxygène, les radicaux R et R', identiques ou différents, représentent chacun un atome d'hydrogène ou un groupement -Y-Z- ou Y'-Z' dis lequel Y, Y', Z et Z' sont définis comme précédemment, étant entendu que l'un au moins des radicaux R et R' contient un radical intercalant ou un radical chimique réactif,
- lorsque L représente un atome d'oxygène, R et R' représentent chacun un atome d'hydrogène et B représente une base nucléique modifiée activable et/ou comportant un groupement intercalant.

**6.** Nouveau dérivé selon l'une des revendications 3 à 5, caractérisé en ce que l'agent d'intercalation Z ou Z' est choisi parmi les composés polycycliques ayant une configuration plane.

**7.** Nouveau dérivé selon la revendication 6 caractérisé en ce que l'agent d'intercalation Z ou Z' est choisi parmi l'acridine et ses dérivés, la furocoumarine et ses dérivés, la daunomycine et les autres dérivés de l'anthracycline, la 1,10-phénanthroline, la phénanthridine et ses dérivés, la proflavine, les porphyrines, les dérivés du dipyrido [1,2-a:3',2'-d] imidazole, l'ellipticine ou l'ellipticinium et leurs dérivés et le diazapyrène et ses dérivés.

**8.** Nouveau dérivé selon l'une des revendications 3 à 5, caractérisé en ce que les groupes chimiques réactifs Z ou Z' sont choisis parmi l'acide éthylène-diamine-tétraacétique, l'acide diéthylène-triamine-pentaacétique, les porphyrines, la 1,10-phénanthroline, l'azido-4 acétophénone, l'éthylène-imine, la $\beta$-chloroéthylamine, le psoralène et leurs dérivés.

**9.** Composés selon l'une des revendications 3 à 7, caractérisés en ce qu'ils consistent en un oligonucléotide $\alpha$, lié de façon covalente, notamment par l'intermédiaire d'une chaîne pentaméthylène à un dérivé 2-N-méthyl-9-hydroxy ellipticinium acétate.

**10.** Nouveau dérivé selon l'une des revendications 3 à 9, caractérisé en ce que le radical B est un radical correspondant à la thymine, l'adénine, la 2-aminoadénine et ses dérivés substitués, la cytosine, la guanine et ses dérivés substitués, l'uracile, le bromo-5 uracile, l'azido-8 adénine, la 7-déazaadénine, la 7-déazaguanine, la 4-azido-cytosine, la 4-azido-thymine et les dérivés de ces bases comportant un groupement intercalant et/ou un groupement chimiquement ou photochimiquement activable.

**11.** Nouveau dérivé selon l'une des revendications 3 à 10, caractérisé en ce qu'il est choisi parmi : $\alpha$–[(Tp)$_n$T](Y$_1$)Z$_1$ ; Z$_2$(Y$_2$)$\alpha$–[(Tp)$_n$T] ; Z$_2$(Y$_2$)$\alpha$-[(Tp)$_n$T](Y$_1$)Z$_1$ ; Z$_2$(Y$_2$)$\alpha$-d(CpCpTpTpApTpApTpT), A représentant un radical $\alpha$-D-désoxyadénosine, C représentant un radical $\alpha$-D-désoxycytidine, T représentant un radical $\alpha$-D-thymidine, n étant un nombre entier compris entre 1 et 25 ; $Y_1$ et $Y_2$ représentant un radical alcoylène contenant 1 à 10 atomes de carbone et $Z_1$ et $Z_2$ représentant chacun un dérivé de l'acridine ou un dérivé de la proflavine ou un dérivé de la 1,10-phénanthroline ou un dérivé de l'azido-4 acétophénone ou un dérivé de l'EDTA ou un dérivé de la biotine.

**12.** Composés selon l'une des revendications 1 à 11, caractérisés en ce qu'il s'agit de composés $\alpha$-D-oligonucléotide.

**13.** Procédé de préparation d'oligonucléotides $\alpha$, selon l'une des revendications 3 à 12, caractérisé en ce qu'on applique des synthèses au phosphodiester, phosphotriester, phosphoramidite ou hydrogénophosphonate en partant de nucléosides $\alpha$ et en ce que l'on prépare les dérivés totalement protégés et que l'on élimine les groupements protecteurs.

**14.** Procédé selon la revendication 13 caractérisé en ce que l'on couple un $\alpha$-D-oligonucléotide 3'-phosphodiester protégé avec l'hydroxyle de l'agent intercalant ou du groupe chimique réactif ou avec l'hydroxyle-5' d'un $\alpha$-D-oligonucléotide protégé possédant déjà l'agent d'intercalation ou le groupe chimique réactif.

**15.** Procédé selon la revendication 13 caractérisé en ce que l'on couple soit un α-nucléotide 5'-phosphodiester protégé avec l'hydroxyle de l'agent intercalant ou du groupe chimique réactif soit un diester portant l'agent intercalant ou le groupe chimique réactif avec l'hydroxyle-5' d'un α-D-oligonucléotide protégé.

**16.** Procédé selon la revendication 13 caractérisé en ce que l'on couple l'hydroxyle de l'agent intercalant ou du groupe chimique réactif avec Un α-D-oligonucléotide-3'-5'-bis-(phosphodiester) protégé.

**17.** Procédé selon la revendication 13 caractérisé en ce que l'on couple un diester porteur de l'agent intercalant ou du groupe chimique réactif avec l'hydroxyle-5' d'un α-D-oligonucléotide protégé possédant déjà un agent intercalant ou un groupe chimique réactif.

**18.** Procédé selon la revendication 13 caractérisé en ce que l'on réalise la déprotection des groupes phosphoesters arylés en milieu anhydre à l'aide d'acide benzohydroxamique et en présence de base tertiaire non nucléophile.

**19.** Procédé de préparation d'un nouveau dérivé selon l'une des revendications 3 à 12, caractérisé en ce que l'on prépare les dérivés α-D-oligonucléotides non protégés comportant un groupe thiophosphate à l'une des extrémités 3' ou 5' ou aux deux extrémités 3' et 5' de la chaîne nucléotidique.

**20.** Procédé selon la revendication 19 caractérisé en ce que l'on couple un α-D-oligonucléotide 3'-thiophosphate non protégé avec un halogènoalkyle porteur de l'agent intercalant ou d'un groupe chimique.

**21.** Procédé selon la revendication 19 caractérisé en ce que l'on couple un α-D-oligonucléotide 5'-thiophosphate non protégé avec un halogènoalkyle porteur de l'agent intercalant ou d'un groupe chimique.

**22.** Procédé selon la revendication 19 caractérisé en ce que l'on couple un α-D-oligonucléotide 3',5'-bisthiophosphate non protégé avec un halogènoalkyle porteur de l'agent intercalant ou un groupe chimique.

**23.** Procédé de préparation d'oligonucléotide α, selon l'une des revendications 3 à 12, caractérisé en ce qu'on applique la synthèse au phosphotriester classique pour des anomères β, mais dans laquelle les dérivés α-nucléotides de guanine sont protégés en position $O^6$ par un groupe supplémentaire, de préférence le N,N-diphénylcarbamoyl.

**24.** Procédé de synthèse des conposés sans agent effecteur selon l'une des revendications 2 et 3, caractérisé en ce qu'on réalise une synthèse supportée selon la méthode au phosphoroamidite comportant

- une protection en 3' et 5' des nucléotides ou oligonucléotides de départ avec par exemple du diméthoxytrityl en 5' et du diisopropylaminophosphoramidite de méthyle en 3',
- la fonctionnalisation d'un support solide incorporant un dérivé α nucléosidique par un lien par exemple succinyle entre le groupement hydroxyle-3' du dérivé α nucléosidique et un groupement amino du support solide,
- l'élongation de la chaîne oligonucléotidique dans un réacteur synthétiseur,
- enfin le décrochage, la déprotection et la purification de l'oligonucléotide prolongé.

**25.** Procédé de synthese de composes incorporant un agent effecteur selon l'une des revendications 3 à 12 ,caractérisé en ce que l'on part d'un oligonucléotide sans agent effecteur protégé en 5', dont on fait réagir la terminaison OH 3' avec une fonction non protégée d'un bras difonctionnel dont la deuxième fonction est protégée et après déprotection du produit résultant, on lie ledit produit à l'agent effecteur par la deuxième fonction libre du bras difonctionnel.

**26.** Procédé selon la revendication 25, caractérisé en ce que la terminaison OH 3' de l'oligomère protégé en 5' est estérifiée avec un acide aminohexanoïque, dont la fonction amine est protégée.

**27.** Application des composés selon l'une des revendications 3 à 12, caractérisée en ce qu'ils s'apparient parallèlement à une séquence d'oligonucléotide $\beta$ complémentaire.

**28.** Application des composés selon l'une des revendications 3 à 12, caractérisée en ce que lesdits composés sont utilisés à titre de sondes d'hybridation ou comme composants de purification pour des séquences déterminées d'ADN ou d'ARN en simple brin ou en double hélice dans un but diagnostique ou pronostique.

**29.** Application des composés selon l'une des revendications 3 à 12, caractérisée en ce que les composés sont utilisés pour détecter des mutations au niveau d'un ADN ou d'un ARN.

**30.** Application des composés selon l'une des revendications 3 à 12, caractérisée en ce que ces composés sont utilisés pour réaliser le blocage spécifique de gènes cellulaires ou d'agents pathogènes préalablement choisis, tels que des virus, bactéries ou des parasites.

**31.** Application d'un dérivé selon l'une des revendications 3 à 12 pour bloquer sélectivement l'expression d'un gène soit par hybridation, soit par coupure sélective, soit par modification chimique du gène ou de son ARN messager d'origine cellulaire, virale, bactérienne ou parasitaire.

**32.** Application d'un dérivé selon l'une des revendications 3 à 12 pour bloquer sélectivement l'expression d'un ARN viral soit par hybridation, soit par coupure, soit par modification chimique de l'ARN viral ou de son ARN messager.

**33.** Application des composés selon l'une des revendications 3 à 12, caractérisée en ce qu'ils sont utilisables à titre de nucléases artificielles spécifiques de séquences.

**34.** A titre de médicaments, des composés selon l'une des revendications 3 à 12, utilisables comme antiviraux, antitumoraux, antibiotiques ou antiparasitaires ou dans toute pathologie où un gène est anormalement exprimé soit par mutation, soit par dérégulation.

**35.** Application d'un dérivé $\alpha$-D-oligonucléotide de formule générale :

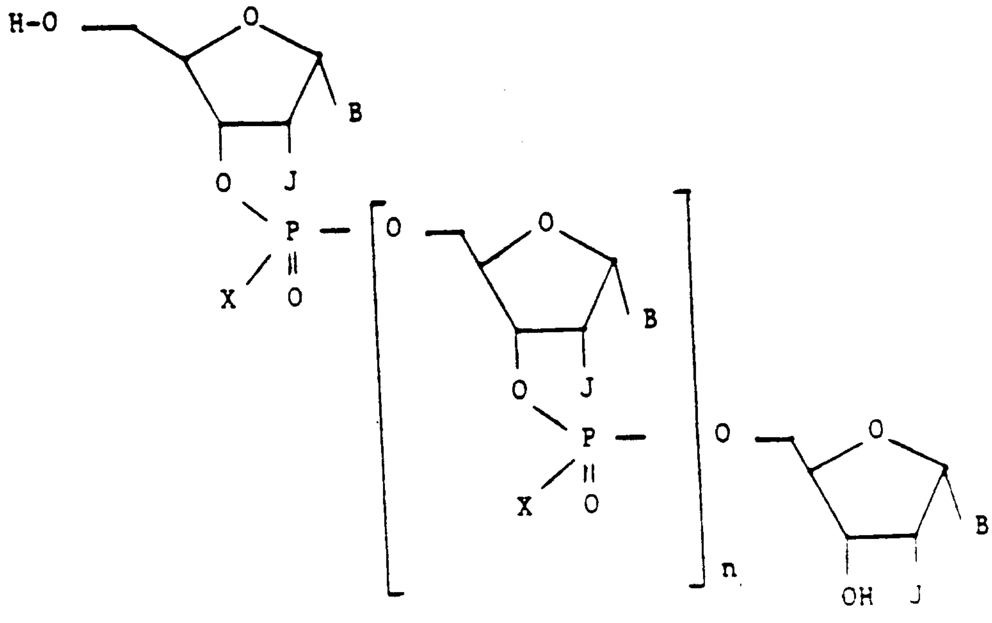

dans laquelle J et X sont définis comme dans la revendication 3 et B représente une base nucléique naturelle à titre de sonde pour la détection d'une séquence déterminée d'ADN ou d'ARN en simple brin ou en double hélice dans un but diagnostique ou pronostique.

**36.** Application d'un dérivé tel que défini dans la revendication 35 à la purification d'une séquence déterminée d'ADN ou d'ARN en simple brin ou en hélice.

**37.** Application d'un dérivé tel que défini dans la revendication 35 pour bloquer sélectivement l'expression d'un gène par hybridation sélective du gène ou de son ARN messager d'origine cellulaire, virale, bactérienne ou parasitaire.

**38.** Application d'un dérivé tel que défini dans la revendication 35 pour bloquer sélectivement l'expression d'un ARN viral ou de son ARN messager.

**39.** A titre de médicament nouveau un dérivé tel que défini dans la revendication 35 utilisable comme antiviral, antitumoral, antibiotique ou antiparasitaire ou dans toute pathologie où un gène est anormalement exprimé soit par mutation, soit par dérégulation.

## Claims

**1.** Chemical compounds consisting of an oligonucleotide or oligodeoxynucleotide, which compounds contain an arrangement of nucleotides or deoxynucleotides having an unnatural α anomeric configuration, it being possible for this arrangement linked to an effector agent, in particular a radical corresponding to an intercalating agent or a chemical or photoactivable radical, such as a radical bearing a group that reacts directly or indirectly with nucleotide chains or a radical whose presence permits ready and sensitive detection.

**2.** The chemical compounds, consisting of an oligonucleotide or oligodeoxynucleotide, which compounds contain an arrangement of nucleotides or deoxynucleotides having an unnatural anomeric configuration, the arrangement not being linked to an effector radical, and the nucleotides or desoxynucleotides not being all thymin or cytosin.

**3.** The compounds as claimed in claim 1 or 2, which are of the formula:

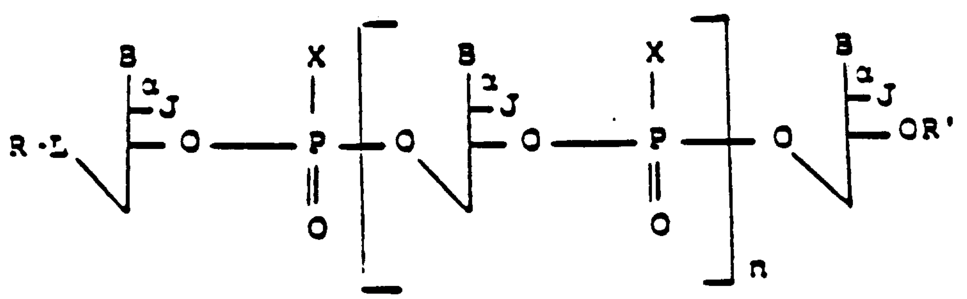
(I)

in which:

the radicals B may be identical or different and each denote an optionally modified nucleic acid base, activable and/or containing an intercalating group and attached to the glycoside ring according to an unnatural α anomeric configuration;

the radicals X may be identical or different and each denote an oxo (sic) anion $O^-$, a thio (sic) anion $S^-$, an alkyl group, an alkoxy group, an aryloxy group, an aminoalkyl group, an aminoalkoxy group, a thioalkyl group, an alkyl or alkoxy radical substituted with a nitrogen-containing heterocycle or a group -Y-Z;

R and R', which may be identical or different, each denote a hydrogen atom or a group -Y-Z or Y'-Z';

Y and Y', which may be identical or different, each denote a linear or branched alkylene radical -alk-or a radical chosen from

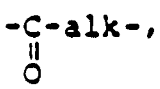, 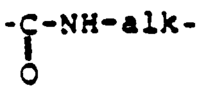, 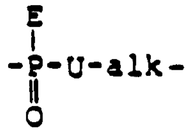, 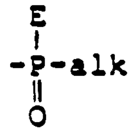, alk-O-alk

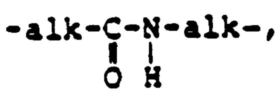, 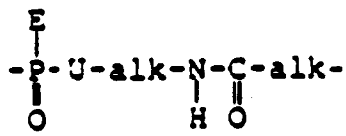 and 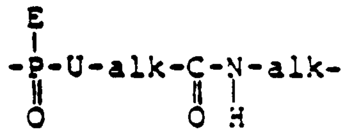

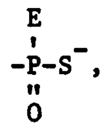, 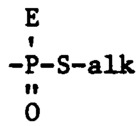

with U = O, S or N,

E can have the same meaning as X except for Y-Z,

J denotes a hydrogen atom or a hydroxy group;

Z and Z', which may be identical or different, each denote a radical corresponding to an effector agent, in particular a radical corresponding to an intercalating agent or a radical hearing a group which reacts directly or indirectly with nucleotide chains, or a radical whose presence permits ready and sensitive detection. (sic)

n is an integer including O;

L denotes an oxygen atom, a sulfur atom or an -NH-group.

**4.** The compounds as claimed in claim 3, in which Y and Y' denote a radical chosen from

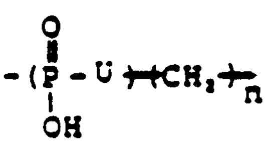, 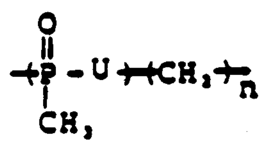, 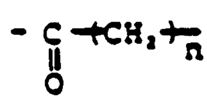, 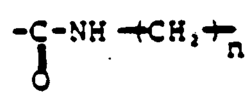

with U = O, S or N.

**5.** The compounds as claimed in claim 3, in which, in the general formula I,

- when L denotes a sulfur atom or an -NH- group, the radicals R and R', which may be identical or different, each denote a hydrogen atom or a group -Y-Z or Y'-Z' in which:
  . Y and Y', which may be identical or different, each denote a linear or branched alkylene radical (-alk-) or a radical

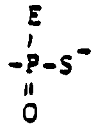

or a radical

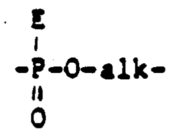,
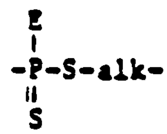

or

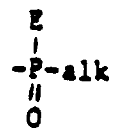

or a radical -Alk-O-alk or a radical -alk-CONH-alk- or a radical

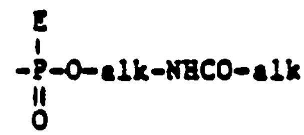

or a radical

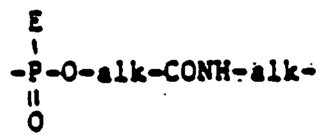,

and

. Z and Z', which may be identical or different, each denote a radical corresponding to an intercalating agent or a radical bearing a group which reacts directly or indirectly with nucleotide chains or a radical whose presence permits ready and sensitive detection,

- when L denotes an oxygen atom, the radicals R and R', which may be identical or different, each denote a hydrogen atom or a group -Y-Z- or Y'-Z' in which Y, Y', Z and Z' are defined as above, with the proviso that at least one of the radicals R and R' contains an intercalating radical or a reactive chemical radical,
- when L denotes an oxygen atom, R and R' each denote a hydrogen atom and B denotes a modified nucleic acid base which is activable and/or contains an intercalating group.

**6.** A new derivative as claimed in one of claims 3 to 5, in which the intercalating agent Z or Z' is chosen from polycyclic compounds having a flat configuration.

**7.** The new derivative as claimed in claim 6, in which the intercalating agent Z or Z' is chosen from acridine and its derivatives, furocoumarin and its derivatives, daunomycin and other anthracycline derivatives, 1,10-phenanthroline, phenanthridine and its derivatives, proflavine, porphyrins, dipyrido[1,2-a:3',2'-d]imidazole derivatives, ellipticine or ellipticinium and their derivatives, and diazapyrene and its derivatives.

**8.** A new derivative as claimed in one of claims 3 to 5, in which the reactive chemical groups Z or Z' are chosen from ethylenediaminetetraacetic acid, diethylenetriaminepentaacetic acid, porphyrins, 1,10-phenanthroline, 4-azidoacetophenone, ethylenimine, $\beta$-chloroethylamine, psoralen and their derivatives.

**9.** The compounds as claimed in one of claims 3 to 7, which consist of an α-oligonucleotide linked covalently, in particular via a pentamethylene chain, to an $N^2$-methyl-9-hydroxyellipticinium acetate derivative.

**10.** A new derivative as claimed in one of claims 3 to 9, in which the radical B is a radical corresponding to thymine, adenine, 2-aminoadenine and its substituted derivatives, cytosine, guanine and its substituted derivatives, uracil, 5-bromouracil, 8-azidoadenine, 7-deazaadenine, 7-deazaguanine, 4-azidocytosine, 4-azidothymine and derivatives of these bases containing an intercalating group and/or a chemically or photochemically activable group.

**11.** A new derivative as claimed in one of claims 3 to 10 which is chosen from: α-[$(Tp)_n$T]($Y_1$)$Z_1$; $Z_2$($Y_2$)α-$(Tp)_n$T]; $Z_2$($Y_2$)α-[$(Tp)_n$T]($Y_1$)$Z_1$; $Z_2$($Y_2$)α-d(CpCpTpTpApTpApTpT), A denoting an α-D-deoxyadenosine radical, C denoting an α-D-deoxycytidine radical, T denoting an α-D-thymidine radical, n being an integer between 1 and 25; $Y_1$ and $Y_2$ denoting an alkylene radical containing 1 to 10 carbon atoms and $Z_1$ and $Z_2$ each denoting an acridine derivative or a proflavine derivative or a 1,10-phenanthroline derivative or a 4-azidoacetophenone derivative or an EDTA derivative or a biotin derivative.

**12.** The compounds as claimed in one of claims 1 to 11, which are α-D-oligonucleotide compounds.

**13.** A process for preparing or (sic) an α-oligonucleotide as claimed in one of claims 3 to 12, wherein phosphodiester, phosphotriester, phosphoramidite or hydrogenophosphonate syntheses are applied, starting with α-nucleosides, and wherein the fully protected derivatives are prepared and wherein the protected groups are removed.

**14.** The process as claimed in claim 13, wherein a protected α-D-oligonucleotide 3'-phosphodiester is coupled with the hydroxyl of the intercalating agent or of the reactive chemical group, or with the 5'-hydroxyl of a protected α-D-oligonucleotide already possessing the intercalating agent or the reactive chemical group.

**15.** The process as claimed in claim 13, wherein either a protected α-nucleotide 5'-phosphodiester is coupled with the hydroxyl of the intercalating agent or of the reactive chemical group, or a diester bearing the intercalating agent or the reactive chemical group is coupled with the 5'-hydroxyl of a protected α-D-oligonucleotide.

**16.** The process as claimed in claim 13, wherein the hydroxyl of the intercalating agent or of the reactive chemical group is coupled with a protected α-D-oligonucleotide 3',5'-bis(phosphodiester).

**17.** The process as claimed in claim 13, wherein a diester bearing the intercalating agent or bearing the reactive chemical group is coupled with the 5'-hydroxyl of a protected α-D-oligonucleotide already possessing an intercalating agent or a reactive chemical group.

**18.** The process as claimed in claim 13, wherein the deprotection of the aryl phosphoester groups is carried out in an anhydrous medium using benzohydroxamic acid and in the presence of a non-nucleophilic tertiary base.

**19.** A process for preparing a new derivative as claimed in one of claims 3 to 12, wherein the unprotected α-D-oligonucleotide derivatives containing a thiophosphate group at one of the 3' or 5' ends or at both 3' and 5' ends of the nucleotide chain are prepared.

**20.** The process as claimed in claim 19, wherein an unprotected α-D-oligonucleotide 3'-thiophosphate is coupled with a haloalkyl bearing the intercalating agent or bearing a chemical group.

**21.** The process as claimed in claim 19, wherein an unprotected α-D-oligonucleotide 5'-thiophosphate is coupled with a haloalkyl bearing the intercalating agent or bearing a chemical group.

**22.** The process as claimed in claim 19, wherein an unprotected α-D-oligonucleotide 3',5'-bis-(thiophosphate) is coupled with a haloalkyl bearing the intercalating agent or bearing a chemical group.

**23.** A process for preparing an α-oligonucleotide as claimed in one of claims 3 to 12, wherein the phosphotriester synthesis which is classical for β anomers is applied, but in which the α-nucleotide derivatives of guanine are protected at the $O^6$ position by an additional group, preferably N,N-diphenylcarbamoyl.

**24.** A process for synthesizing compounds without an effector agent as claimed in one of claims 2 and 3, wherein a supported synthesis is carried out according to the phosphoramidite method, comprising

- a protection of the starting nucleotides or oligonucleotides at the 3'- and 5'-positions with, for example, dimethoxytrityl at the 5'-position and methyl diisopropylaminophosphoramidite at the 3'-position,
- the functionalization of a solid support incorporating an α-nucleoside derivative, by a link, for example succinyl, between the 3'-hydroxyl group of the α-nucleoside derivative and an amino group of the solid support,
- the elongation of the oligonucleotide chain in a synthesizing reactor, and
- finally, the detachment, deprotection and purification of the extended oligonucleotide.

**25.** Process for synthesizing compounds incorporating an effector agent as claimed in one of claims 3 to 12, wherein the starting substance is an oligonucleotide without an effector agent protected at the 5'-position, the 3'-OH end of which is reacted with an unprotected functional group of a difunctional arm whose second functional group is protected, and, after deprotection of the resulting product, the said product is linked to the effector agent via the second free functional group of the difunctional arm.

**26.** The process as claimed in claim 25, wherein the 3'-OH end of the oligomer protected at the 5'-position is esterified with an aminohexanoic acid in which the amine group is protected.

**27.** An application of the compounds as claimed in one of claims 3 to 12, wherein they pair in parallel fashion with a complementary β-oligonucleotide sequence.

**28.** An application of the compounds as claimed in one of claims 3 to 12, wherein the said compounds are used by way of hybridization probes or as purification components for specified single-stranded or double-helical DNA or RNA sequences, for diagnostic or predictive purposes.

**29.** An application of the compounds as claimed in one of claims 3 to 12, wherein the compounds are used for detecting mutations at the level of a DNA or an RNA.

**30.** An application of the compounds as claimed in one of claims 3 to 12, wherein these compounds are used for carrying out the specific blocking of cellular genes or pathogenic agents selected beforehand, such as viruses, bacteria or parasites.

**31.** The application of a derivative according to one of claims 3 to 12, for selectively blocking the expression of a gene, either by hybridization, or by selective cleavage, or by chemical modification of the gene or of its messenger RNA of cellular, viral, bacterial or parasitic origin.

**32.** The application of a derivative as claimed in one of claims 3 to 12 for selectively blocking the expression of a viral RNA, either by hybridization, or by cleavage, or by chemical modification of the viral RNA or of its messenger RNA.

**33.** An application of the compounds as claimed in one of claims 3 to 12, wherein the compounds are usable by way of sequence-specific artificial nucleases.

**34.** By way of medicinal products, compounds as claimed in one of claims 3 to 12 which are usable as antiviral, antitumor, antibiotic or antiparasitic agents, or in any pathological condition in which a gene is abnormally expressed, either by mutation or by deregulation.

**35.** The application of an α-D-oligonucleotide derivative of general formula:

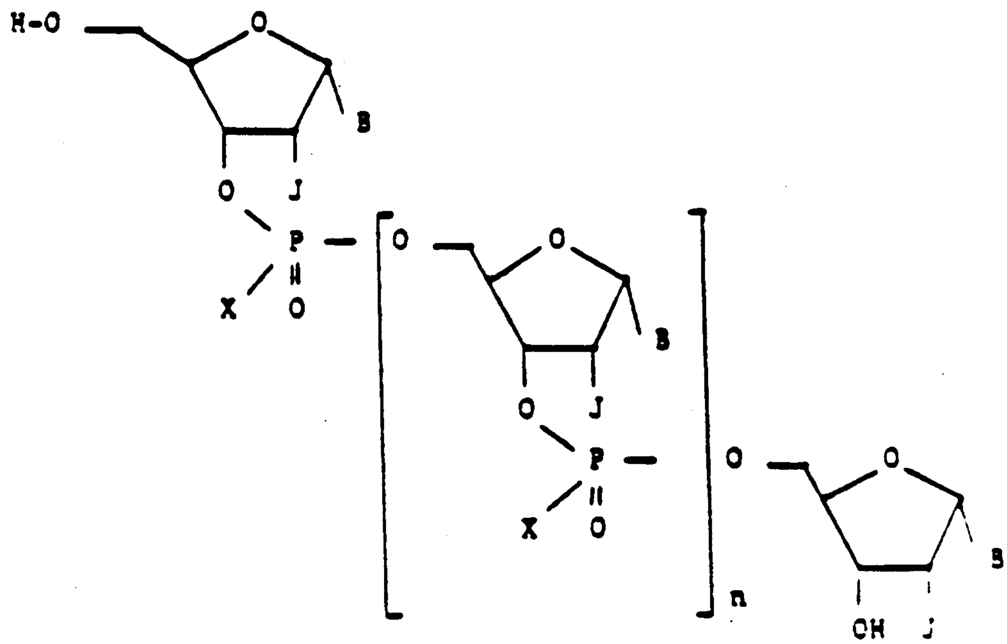

in which J and X are defined as in claim 3 and B denotes a natural nucleic acid base, by way of a probe for the detection of a specified single-stranded or double-helical DNA or RNA sequence, for diagnostic or predictive purposes.

**36.** The application of a derivative as defined in claim 35 to the purification of a specified single-stranded or helical DNA or RNA sequence.

**37.** The application of a derivative as defined in claim 35 for selectively blocking the expression of a gene by the selective hybridization of the gene or of its messenger RNA of cellular, viral, bacterial or parasitic origin.

**38.** The application of a derivative as defined in claim 35 for selectively blocking the expression of a viral RNA or its messenger RNA.

**39.** By way of a new medicinal product, a derivative as defined in claim 35 which is usable as an antiviral, antitumor, antibiotic or antiparasitic agent or in any pathological condition in which a gene is abnormally expressed, either by mutation or by deregulation.

**Patentansprüche**

**1.** Chemische Verbindungen, bestehend aus einem Oligonukleotid oder einem Oligodesoxynukleotid, dadurch gekennzeichnet, daß sie eine Verkettung von Nukleotiden oder Desoxynukleotiden mit anomerischer nicht natürlicher α-Konfiguration umfassen, wobei die Verkettung in kovalenter Weise mit einem Effektor-Mittel verbunden ist, insbesondere mit einem Radikal, das einem Intercalations-Mittel oder einem chemischen oder photoaktivierbaren Radikal entspricht, wie einem Radikal, das eine Funktion trägt, die direkt oder indirekt mit den Nukleotid-Ketten reagiert, oder einem Radikal, dessen Anwesenheit einen einfachen und empfindlichen Nachweis ermöglicht.

**2.** Chemische Verbindungen, bestehend aus einem Oligonukleotid oder einem Oligodesoxynukleotid, dadurch gekennzeichnet, daß sie eine Verkettung von Nukleotiden oder Desoxynukleotiden mit anomerischer, nicht natürlicher α-Konfiguration umfassen, die nicht mit einem Effektor-Radikal verbunden sind, wobei die genannten Nukleotide oder Desoxynukleotide nicht nur Thymin- oder Cytosin-Basen umfassen.

3. Chemische Verbindungen nach Anspruch 1 oder 2, dadurch gekennzeichnet, daß sie die allgemeine Formel I

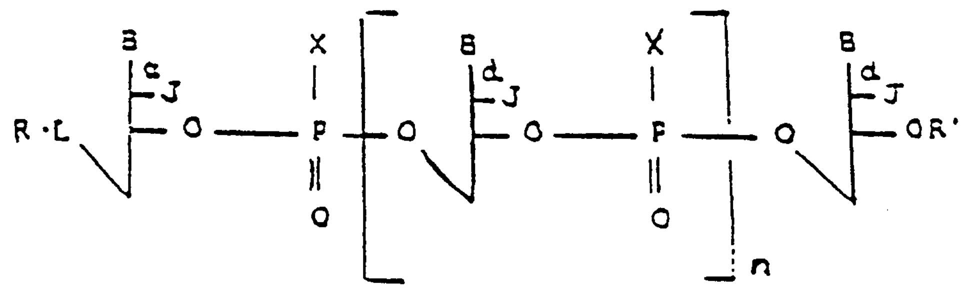

(I)

aufweisen, in der
die Radikale B gleich oder verschieden sein können und jeweils die Basis einer gegebenenfalls modifizierten Nukleinsäure darstellen, aktivierbar und/oder umfassend eine Intercalations-Gruppe und verbunden mit einem Glycosid-Ring gemäß einer anomerischen, nicht natürlichen $\alpha$-Konfiguration;
die Radikale X gleich oder verschieden sein können und jeweils ein Oxoanion $O^-$, ein Thioanion $S^-$, eine Alkylgruppe, eine Alkoxygruppe, eine Aryloxygruppe, eine Aminoalkylgruppe, eine Aminoalkoxygruppe, eine Thioalkylgruppe, ein Alkylradikal oder ein Alkoxyradikal darstellen, substituiert durch einen Stickstoff-Heterozyklusoder eine Gruppe -Y-Z;
R und R' gleich oder verschieden sein können und jeweils ein Wasserstoffatom oder eine Gruppe -Y-Z oder Y'-Z' darstellen;
Y und Y' gleich oder verschieden sein können und jeweils ein lineares oder verzweigtes Alkoylen-Radikal -alk- oder ein unter den folgenden Radikalen ausgewähltes Radikal darstellen:

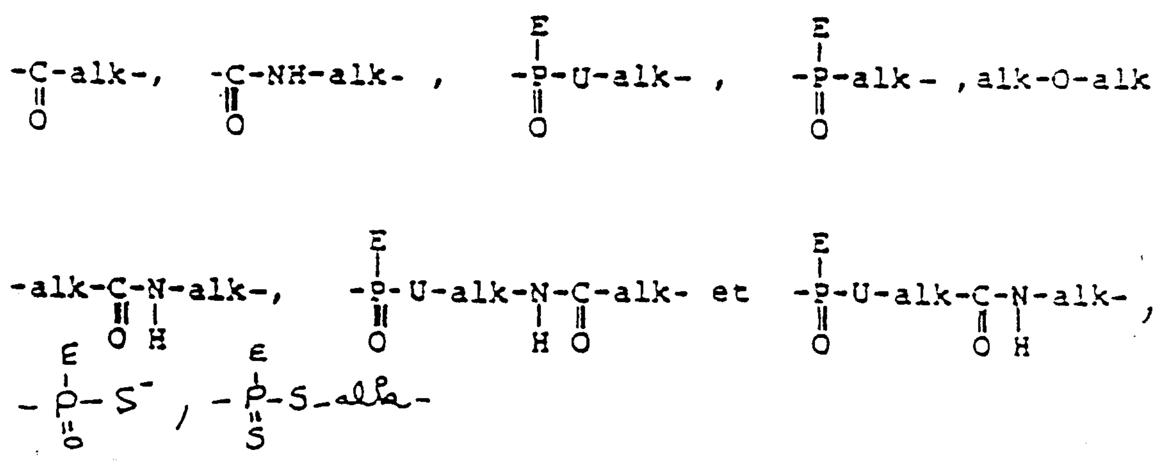

mit U = O, S oder N
E die gleichen Bedeutungen wie X besitzen kann, mit Ausnahme von Y-Z,
J ein Wasserstoffatom oder eine Hvdroxygruppe bedeutet,
Z und Z' gleich oder verschieden sein können und jeweils ein Radikal darstellen, das einem Effektor-Mittel entspricht, insbesondere einem Radikal, das einem Intercalations-Mittel oder einem Radikal entspricht, das eine Funktion trägt, die ihrerseits direkt oder indirekt mit den Nukleotid-Ketten oder einem Radikal reagiert, dessen Anwesenheit einen einfachen und empfindlichen Nachweis ermöglicht;
n eine ganze Zahl ist, die auch O umfaßt;
L ein Sauerstoffatom, ein Schwefelatom oder eine Gruppe -NH- bedeutet.

4. Verbindungen nach Anspruch 3, dadurch gekennzeichnet, daß Y und Y' ein Radikal darstellen, ausgewählt unter

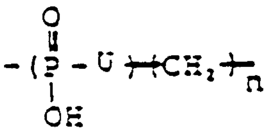, 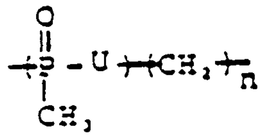, 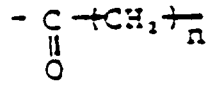, 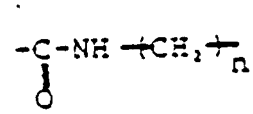

mit U = O, S oder N

**5.** Verbindungen nach Anspruch 3, dadurch gekennzeichnet, daß in der allgemeinen Formel I

- wenn L ein Schwefelatom oder eine Gruppe -NH- darstellt, die Radikale R und R', gleich oder verschieden, jeweils ein Wasserstoffatom oder eine Gruppe -Y-Z oder -Y'-Z' bedeuten, in der
  . Y und Y', gleich oder verschieden, jeweils ein gerades oder verzweigtes Alkoylen-Radikal (-alk-) darstellen oder ein Radikal

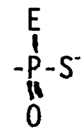

oder ein Radikal

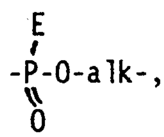, 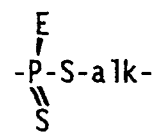

oder

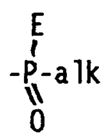

oder ein Radikal -alk-O-alk- oder ein Radikal -alk-CONH-alk- oder ein Radikal

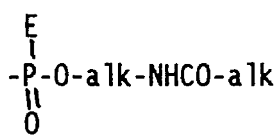

oder ein Radikal

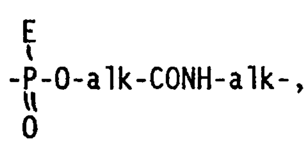,

und

. Z und Z', gleich oder verschieden, jeweils ein Radikal darstellen, das einem Intercalations-Mittel oder einem Radikal entspricht, das eine Funktion trägt, die ihrerseits direkt oder indirekt

mit den Nukleotid-Ketten oder einem Radikal reagiert, dessen Anwesenheit einen einfachen und empfindlichen Nachweis ermöglicht;

- wenn L ein Sauerstoffatom darstellt, die Radikale R und R', gleich oder verschieden, jeweils ein Wasserstoffatom oder eine Gruppe -Y-Z- oder Y'-Z' bedeuten, worin Y, Y', Z und Z' wie vorstehend definiert sind, mit der Maßgabe, daß mindestens eines der Radikale R und R' ein Intercalations-Radikal oder ein chemisch reaktives Radikal ist,
- wenn L ein Sauerstoffatom darstellt, die Radikale R und R' jeweils ein Wasserstoffatom bedeuten und B eine Nuklein-Basis ist, aktivierbar und/oder eine Intercalations-Gruppe enthaltend.

**6.** Neue Derivate nach einem der Ansprüche 3 bis 5, dadurch gekennzeichnet, daß das Intercalations-Mittel Z oder Z' unter den polyzyklischen Verbindungen mit einer ebenen Konfiguration ausgewählt wird.

**7.** Neue Derivate nach Anspruch 6, dadurch gekennzeichnet, daß das Intercalations-Mittel Z oder Z' ausgewählt wird unter Acridin und seinen Derivaten, Furocumarin und seinen Derivaten, Daunomycin und den anderen Derivaten von Anthracyclin, 1,10-Phenanthrolin, Phenanthridin und seinen Derivaten, Proflavin, den Porphyrinen, den Derivaten von Dipyrido[1,2-a:3',2'-d]-imidazol, Ellipticin oder Ellipticinium und seinen Derivaten und Diazapyren und seinen Derivaten.

**8.** Neue Derivate nach einem der Ansprüche 3 bis 5, dadurch gekennzeichnet, daß die reaktiven, chemischen Gruppen Z oder Z' ausgewählt werden unter Ethylendiamin-tetra-essigsäure, Diethylentriamin-pentaessigsäure, den Porphyrinen, 1,10-Phenanthrolin, 4-Azido-acetophenon, Ethylenimin, $\beta$-Chlorethylamin und Psoralen und seinen Derivaten.

**9.** Verbindungen nach einem der Ansprüche 3 bis 7, dadurch gekennzeichnet, daß sie aus einem $\alpha$-Oligonukleotid bestehen, verbunden in kovalenter Weise, insbesondere unter Zwischenschaltung einer Pentamethylen-Kette, mit einem 2-N-Methyl-9-hydroxy-ellipticiniumacetat-Derivat.

**10.** Neue Derivate nach einem der Ansprüche 3 bis 9, dadurch gekennzeichnet, daß das Radikal B ein Radikal ist, das entspricht: Thymin, Adenin, 2-Aminoadenin und seinen substituierten Derivaten, Cytosin, Guanin und seinen substituierten Derivaten, Uracil, 5-Brom-uracil, Azido-8-adenin, 7-Deazaadenin, 7-Deazaguanin, 4-Azido-cytosin, 4-Azido-thymin und den Derivaten dieser Basen, die eine Intercalations-Gruppe und/oder eine chemische oder photochemisch aktivierbare Gruppe umfassen.

**11.** Neue Derivate nach einem der Ansprüche 3 bis 10, dadurch gekennzeichnet, daß sie ausgewählt werden unter: $\alpha$-$[(Tp)_nT](Y_1)Z_1$; $Z_2(Y_2)\alpha$-$[(Tp)_nT]$;
$Z_2(Y_2)\alpha$-$[(Tp)_nT](Y_1)Z_1$; $Z_2(Y_2)\alpha$-d(CpCpTpTpApTpApTpT), worin A ein $\alpha$-D-Desoxyadenosin-Radikal, C ein $\alpha$-D-Desoxycytidin-Radikal und T ein $\alpha$-D-Thymidin-Radikal darstellen, n eine ganze Zahl zwischen 1 und 25 ist; $Y_1$ und $Y_2$ ein Alkoylen-Radikal mit 1 bis 10 Kohlenstoffatomen bedeuten und $Z_1$ und $Z_2$ jeweils ein Acridin-Derivat, ein Proflavin-Derivat, ein 1,10-Phenanthrolin-Derivat, ein 4-Azido-Acetophenon-Derivat, ein EDTA-Derivat oder ein Biotin-Derivat darstellen.

**12.** Verbindungen nach einem der Ansprüche 1 bis 11, dadurch gekennzeichnet, daß es sich um $\alpha$-D-Oligonukleotid-Verbindungen handelt.

**13.** Verfahren zur Herstellung von $\alpha$-Oligonukleotiden nach einem der Ansprüche 3 bis 12, dadurch gekennzeichnet, daß man Synthesen für Phosphodiester, Phosphotriester, Phosphoramidit oder Hydrogenphosphonat ausgehend von $\alpha$-Nukleosiden anwendet, daß man die vollständig geschützten Derivate herstellt und daß man die Schutzgruppen wieder entfernt.

**14.** Verfahren nach Anspruch 13, dadurch gekennzeichnet, daß man einen geschützten $\alpha$-D-Oligonukleotid-3'-phosphodiester mit dem Hydroxyl des Intercalations-Mittels oder der chemisch reaktiven Gruppe kuppelt, oder mit dem 5'-Hydroxyl eines geschützten $\alpha$-D-Oligonukleotides, das bereits das Intercalations-Mittel oder die chemisch reaktive Gruppe besitzt.

**15.** Verfahren nach Anspruch 13, dadurch gekennzeichnet, daß man entweder einen geschützten $\alpha$-Nukleotid-5'-phosphodiester mit dem Hydroxyl des Intercalations-Mittels oder der chemisch reaktiven Gruppe kuppelt, oder einen Diester, der das Intercalations-Mittel oder die chemisch reaktive Gruppe

trägt, mit dem 5'-Hydroxyl eines geschützten α-D-Oligonukleotides.

**16.** Verfahren nach Anspruch 13, dadurch gekennzeichnet, daß man das Hydroxyl des Intercalations-Mittels oder der chemisch reaktiven Gruppe mit einem geschützten α-D-Oligonukleotid-3'-5'-bis-(phosphodiester) kuppelt.

**17.** Verfahren nach Anspruch 13, dadurch gekennzeichnet, daß man einen Diester, der das Intercalations-Mittel oder die chemisch reaktive Gruppe trägt, mit dem 5'-Hydroxyl eines geschützten α-D-Oligonukleotides, das bereits das Intercalations-Mittel oder die chemisch reaktive Gruppe besitzt, kuppelt.

**18.** Verfahren nach Anspruch 13, dadurch gekennzeichnet, daß man die Abspaltung der arylierten Phosphoester-Gruppen im wasserfreien Medium mit Hilfe von Benzohydroxamin-Säure und in Anwesenheit einer nicht nukleophilen tertiären Base durchführt.

**19.** Verfahren zur Herstellung eines neuen Derivates nach einem der Ansprüche 3 bis 12, dadurch gekennzeichnet, daß man die nicht geschützten α-D-Oligonukleotide herstellt, die eine Thiophosphat-Gruppe an einem der Enden 3' oder 5' oder an zwei Enden 3' und 5' der Nukleotid-Kette umfassen.

**20.** Verfahren nach Anspruch 19, dadurch gekennzeichnet, daß man ein nicht geschütztes α-D-Oligonukleotid-3'-thiophosphat mit einem Halogenalkyl kuppelt, das das Intercalations-Mittel oder eine chemisch reaktive Gruppe trägt.

**21.** Verfahren nach Anspruch 19, dadurch gekennzeichnet, daß man ein nicht geschütztes α-D-Oligonukleotid-5'-thiophosphat mit einem Halogenalkyl kuppelt, das das Intercalations-Mittel oder eine chemisch reaktive Gruppe trägt.

**22.** Verfahren nach Anspruch 19, dadurch gekennzeichnet, daß man ein nicht geschütztes α-D-Oligonukleotid-3',5'-bisthiophosphat mit einem Halogenalkyl kuppelt, das das Intercalations-Mittel oder eine chemisch reaktive Gruppe trägt.

**23.** Verfahren zur Herstellung von α-Oligonukleotid nach einem der Ansprüche 3 bis 12, dadurch gekennzeichnet, daß man die klassische Phosphotriester-Synthese für die β-Anomeren anwendet, wobei in ihnen jedoch die α-Nukleotid-Derivate des Guanins in Position $O^6$ durch eine zusätzliche Gruppe, vorzugsweise N,N-Diphenylcarbamoyl, geschützt sind.

**24.** Verfahren zur Synthese der Verbindungen ohne Effektor-Mittel nach einem der Ansprüche 2 und 3, dadurch gekennzeichnet, daS man eine zusätzliche Synthese nach der Phosphoramidit-Methode durchführt, die umfaßt

- einen Schutz in 3' und 5' des Ausgangs-Nukleotides oder -Oligonukleotides mit beispielsweise Dimethoxytrityl in 5' und Methyl-Diisopropylaminphosphoramidit in 3',
- die Funktionalisierung eines festen Trägers, der ein α-Nukleosid-Derivat durch beispielsweise Succinyl-Bindung zwischen der 3'-Hydroxyl-Gruppe des α-Nukleosid-Derivates und einer Amino-Gruppe des festen Trägers einführt,
- die Verlängerung der Oligonukleotid-Kette in einem Synthese-Reaktor,
- schließlich die Abkupplung, Abspaltung der Schutzgruppen und die Reinigung der verlängerten Oligonukleotide.

**25.** Verfahren zur Synthese von Verbindungen, die ein Effektor-Mittel einführen, nach einem der Ansprüche 3 bis 12, dadurch gekennzeichnet, daß man von einem in 5' geschützten Oligonukleotid ohne Effektor-Mittel ausgeht, bei dem man das OH-Ende in 3' mit einer nicht geschützten Funktion eines difunktionellen Zweiges, dessen zweite Funktion geschützt ist, zur Reaktion bringt, und man nach der Abspaltung der Schutzgruppe von dem resultierenden Produkt dieses durch die zweite, freie Funktion des difunktionellen Zweiges mit dem Effektor-Mittel verbindet.

**26.** Verfahren nach Anspruch 25, dadurch gekennzeichnet, daS das OH-Ende 3' des geschützten Oligomeren in 5' mit einer Aminohexansäure verestert wird, deren Aminofunktion geschützt ist.

**27.** Anwendung der Verbindungen nach einem der Ansprüche 3 bis 12, dadurch gekennzeichnet, daß sie sich paarweise parallel zu einer zusätzlichen β-Oligosequenz zusammenlegen.

**28.** Anwendung der Verbindungen nach einem der Ansprüche 3 bis 12, dadurch gekennzeichnet, daß die genannten Verbindungen als Hybridations-Sonden oder als Reinigungsbestandteile für Bestimmungs-Sequenzen von DNA oder RNA in einfachem Strang oder in Doppel-Helix mit diagnostischem oder prognostischem Ziel verwendet werden.

**29.** Anwendung der Verbindungen nach einem der Ansprüche 3 bis 12, dadurch gekennzeichnet, daß die Verbindungen zum Nachweis von Mutationen im Bereich einer DNA oder RNA verwendet werden.

**30.** Anwendung der Verbindungen nach einem der Ansprüche 3 bis 12, dadurch gekennzeichnet, daß diese Verbindungen zur Realisierung der spezifischen Blockierung von Zell-Genen oder von zuvor ausgewählten pathogenen Mitteln, wie Viren, Bakterien oder Parasiten, verwendet werden.

**31.** Anwendung eines Derivates nach einem der Ansprüche 3 bis 12 zur selektiven Blockierung der Expression eines Gens, entweder durch Hybridation, durch selektive Abtrennung oder durch chemische Modifizierung des Gens oder seiner Messenger-RNA zellulären, viralen, bakteriellen oder parasitären Ursprungs.

**32.** Anwendung eines Derivates nach einem der Ansprüche 3 bis 12 zur selektiven Blockierung der Expression einer viralen RNA, entweder durch Hybridation, durch Abtrennung oder durch chemische Modifizierung der viralen RNA oder seiner Messenger-RNA.

**33.** Anwendung der Verbindungen nach einem der Ansprüche 3 bis 12, dadurch gekennzeichnet, daß sie als spezifische künstliche Nukleasen von Sequenzen verwendet werden.

**34.** Als Medikamente, Verbindungen nach einem der Ansprüche 3 bis 12, verwendbar als antivirale Mittel, antikanzerogene Mittel, Antibiotika oder antiparasitäre Mittel, oder bei jeder Erkrankung, wo ein Gen entweder durch Mutation oder durch Verunordnung anormal exprimiert wurde.

**35.** Anwendung eines α-D-Oligonukleotides der allgemeinen Formel:

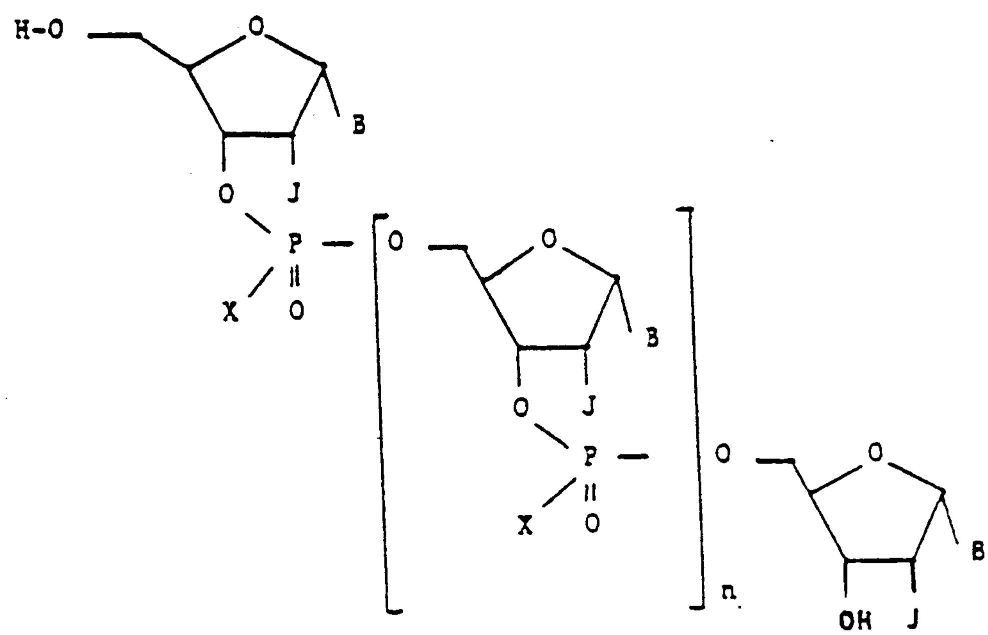

in der J und X wie in Anspruch 3 definiert sind und B eine natürliche Nuklein-Basis darstellt, als Sonde für den Nachweis einer Bestimmungs-Sequenz von DNA oder RNA in einfachem Strang oder in Doppel-Helix mit diagnostischem oder prognostischem Ziel.

**36.** Verwendung eines wie in Anspruch 35 definierten Derivates, zur Reinigung einer Bestimmungs-Sequenz von DNA oder RNA in einfachem Strang oder in Doppel-Helix.

**37.** Verwendung eines wie in Anspruch 35 definierten Derivates, zur selektiven Blockierung der Expression eines Gens durch selektive Hybridation des Gens oder seiner Messenger-RNA zellulären, viralen, bakteriellen oder parasitären Ursprungs.

**38.** Verwendung eines wie in Anspruch 35 definierten Derivates, zur selektiven Blockierung der Expression einer viralen RNA oder seiner Messenger-RNA.

**39.** Als neues Medikament, ein wie in Anspruch 35 definiertes Derivat, verwendbar als antivirales Mittel, antikanzerogenes Mittel, Antibiotikum oder antiparasitäres Mittel, oder bei jeder Erkrankung, wo ein Gen entweder durch Mutation oder durch Verunordnung anormal exprimiert wurde.

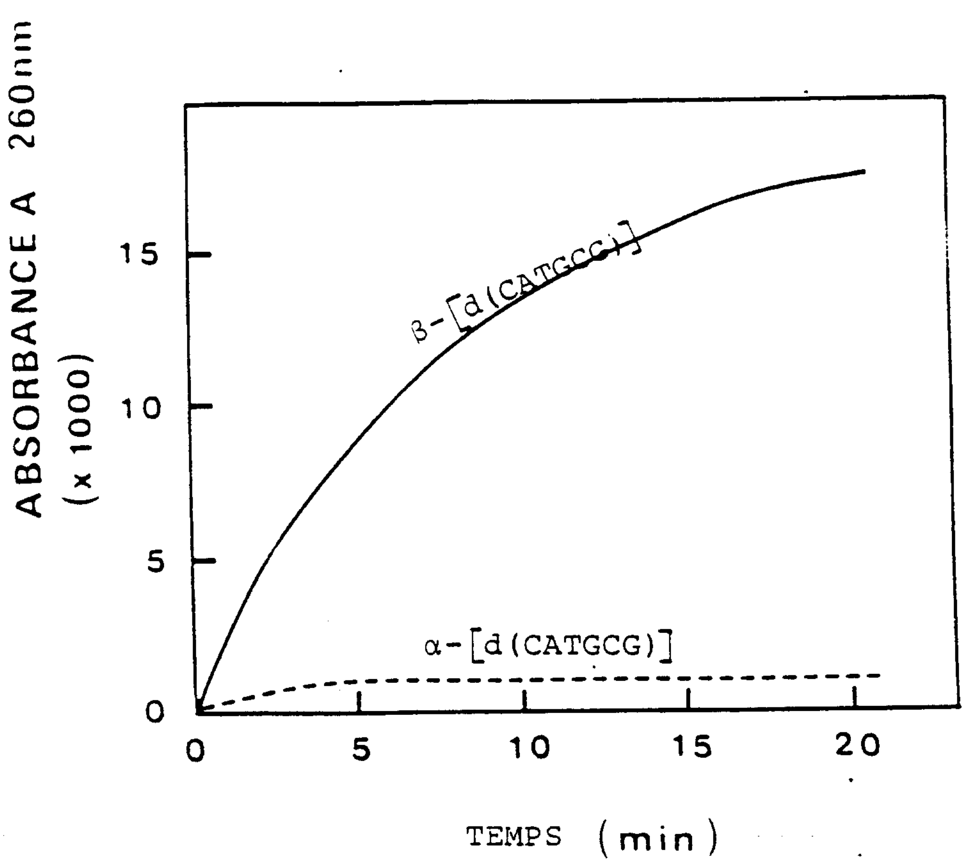
ABSORBANCE A 260nm
(x 1000)
15
10
5
0
β-[d(CATGCG)]
α-[d(CATGCG)]
0
5
10
15
20
TEMPS (min)

FIGURE 1.

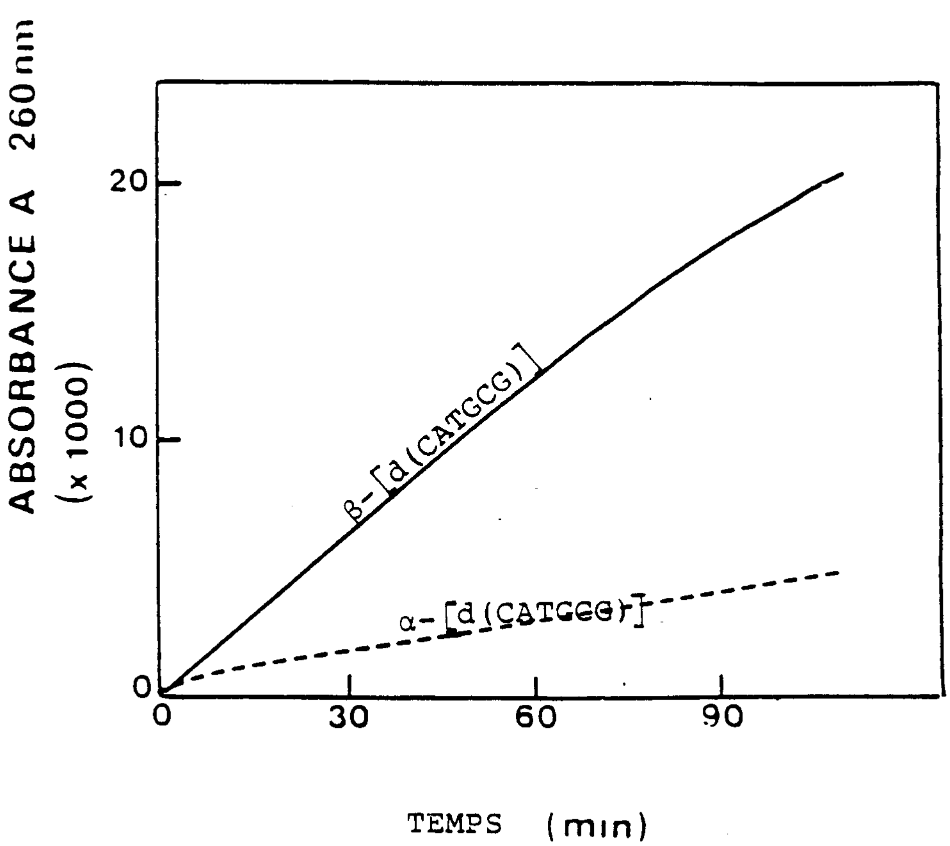
ABSORBANCE A 260nm
(x 1000)
20
10
0
0
30
60
90
β-[d(CATGCG)]
α-[d(CATGCG)]
TEMPS (min)

FIGURE 2